(19) Europäisches Patentamt
European Patent Office
Office européen des brevets

(11) Publication number: 0 528 172 A1

# (12) EUROPEAN PATENT APPLICATION

(21) Application number: **92111986.3**

(22) Date of filing: **14.07.92**

(51) Int. Cl.5: **C07D 213/57**, C07D 213/55, C07D 213/53, C07D 213/38, C07D 401/12, C07D 405/12, C07D 413/12, A61K 31/44, //(C07D401/12,213:00,209:00), (C07D405/12,307:00,213:00), (C07D413/12,263:00,213:00), (C07D401/12,241:00,213:00)

(30) Priority: **08.08.91 ES 9101855**

(43) Date of publication of application: **24.02.93 Bulletin 93/08**

(84) Designated Contracting States: **AT BE CH DE DK ES FR GB GR IT LI LU MC NL PT SE**

(71) Applicant: **J. URIACH & CIA. S.A.**
**Degà Bahi, 59-67**
**E-08026 Barcelona(ES)**

(72) Inventor: **Carceller, Elena**
**Arag n 117**
**ES-08015 Barcelona(ES)**
Inventor: **Jimenez, Pere J.**
**Jaume I 4**
**ES-43750 Flix, Tarragona(ES)**
Inventor: **Recasens, Nuria**
**Olesa 75-77**
**ES-08027-Barcelona(ES)**
Inventor: **Almansa, Carmen**
**Rossell 498**
**ES-08025 Barcelona(ES)**
Inventor: **Bartroli, Javier**
**Vives i Tut 55**
**ES-08034 Barcelona(ES)**

(74) Representative: **Zumstein, Fritz, Dr. et al**
**Zumstein & Klingseisen Patentanwälte**
**Bräuhausstrasse 4**
**W-8000 München 2 (DE)**

## (54) (2-Alkyl-3-pyridyl)methylpiperazine derivatives as PAF antagonists.

(57) The present invention relates to new (2-alkyl-3-pyridyl)methylpiperazine derivatives of general formula **I**:

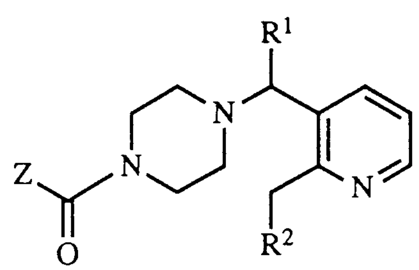

I

wherein $R^1$, $R^2$ and Z are as defined in Claim 1. The invention also relates to processes for their preparation and to pharmaceutical compositions containing them. These compounds are potent, orally active PAF antagonists and, consequently, they are useful in the treatment of the diseases in which this substance is involved.

EP 0 528 172 A1

Field of the invention.

The present invention relates to new (2-alkyl-3-pyridyl)methylpiperazine derivatives having a potent antagonist activity of the platelet activating factor (PAF). The invention also relates to a process for their preparation, to pharmaceutical compositions containing them and to their use in the treatment of the diseases in which PAF is involved, such as bronchial and allergic asthma, platelet aggregation disorders, septic shock, hypertension, inflammation, etc.

Description of the prior art.

The platelet activating factor (PAF) or (1-*O*-alkyl-2-acetyl-*sn*-glyceryl-3-phosphorylcholine), also called acetyl glyceryl ether phosphorylcholine (AGEPC) or PAF-acether, is a natural phospholipid synthesized by different cells (basophiles, macrophages, neutrophiles, platelets) and tissues (heart, lung and kidney) of the organism.

PAF was described for the first time as a potent platelet aggregating agent. Later on it was demonstrated to have other biological activities *in vivo*, such as peripheral vasodilatation, increase of the vascular permeability, induction of bronchoconstriction and hyperreactivity of the respiratory tract. PAF also produces immediate hypotension followed by pulmonary and renal hypertension in rats, guinea pigs , rabbits and dogs, and it has been rated as the most potent ulcerogenic agent described until now. Consequently, PAF is a mediator that is implicated in a large set of pathological processes such as asthma, septic shock, transplant rejection, thrombosis, ulceration, inflammation and renal diseases.

Even though its mechanism of action is still not known with precision, several studies show that the biological activities of PAF involve the existence of a specific receptor. Recently, the isolation of one of these receptors from human platelets has been achieved and it has been identified as a protein with a molecular weight of 160,000 daltons. On the other hand, the capacity to inhibit the binding of $^{3}$H-PAF to their receptors is well correlated with the amount of PAF needed to provoke the *in vitro* and *in vivo* observed effects. These facts indicate that the compounds that act as specific antagonists of PAF could result of interest for the treatment of all those pathological processes related directly or indirectly with PAF.

Until now, several PAF analogues with a potent PAF antagonist activity have been investigated. However, the ionic nature of these compounds usually is associated with a deficient and erratic oral absorption. On the other hand, although several non ionic compounds have been desccribed in the literature and they show a potent PAF antagonist activity when are administered orally (cfr. for example EP 0284359, EP 194416), the provision of new and more potent PAF antagonists suitable to be used in therapy has still a great interest.

Patent application EP 441226 describes (cyanomethyl)pyridines of general formula **I**:

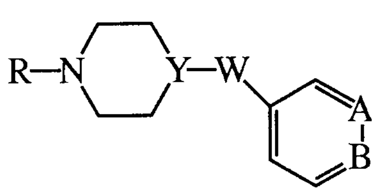

**I**

wherein **A** is nitrogen and **B** is -CH-, or **B** is nitrogen and **A** is -CH-;
the group **Y-W-** represents a group of formula

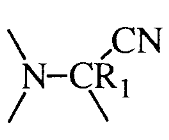 , 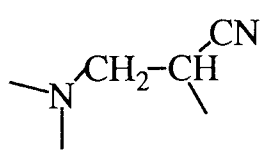 , 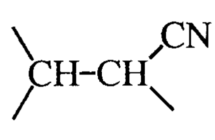 or 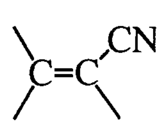

wherein $R_1$ is hydrogen or a $C_1$-$C_6$ alkyl group;
**R** represents a group of formula

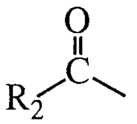 , 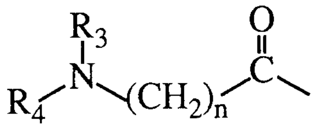 or 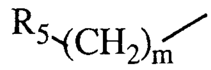

wherein $R_2$ represents $C_1$-$C_{15}$ alkyl, aryl, aryl-($C_1$-$C_6$)-alkyl, aryl-($C_1$-$C_6$)-alkenyl, diaryl-($C_1$-$C_6$)-alkyl, diaryl-($C_1$-$C_6$)-alkenyl, arylhydroxy-($C_1$-$C_6$)-alkyl, diarylhydroxy-($C_1$-$C_6$)-alkyl, aryl-($C_1$-$C_6$)-alkoxy, diaryl-($C_1$-$C_6$)-alkoxy, heteroaryl-($C_1$-$C_6$)-alkyl group or a group of formula

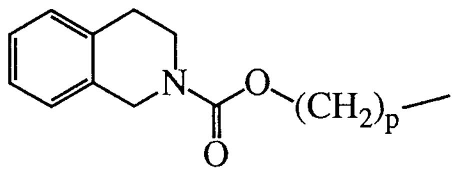

where p = 2 or p = 3; n is 0, 1, 2 or 3;

$R_3$ is hydrogen, $C_1$-$C_6$ alkyl, aryl or aryl-($C_1$-$C_6$)-alkyl group;

$R_4$ is $C_3$-$C_6$ cycloalkyl, aryl, aryl-($C_1$-$C_6$)-alkyl, diaryl-($C_1$-$C_6$)-alkyl, aryl-($C_1$-$C_6$)-alkylcarbonyl or diaryl-($C_1$-$C_6$)-alkylcarbonyl group, or $R_3R_4N$- is a group of formula

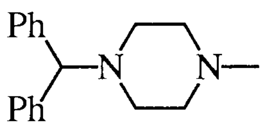 or 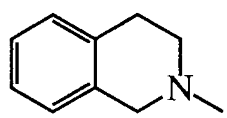

m is 0, 1 or 2;

$R_5$ is aryl-($C_1$-$C_6$)-alkyl, diaryl-($C_1$-$C_6$)-alkyl, diaryl-($C_1$-$C_6$)-alkylcarbonylamino or diaryl-($C_1$-$C_6$)-alkylaminocarbonyl group; being understood that the term "aryl" (or its symbol Ar) means phenyl or a radical from a fused or unfused benzenoid hydrocarbon (e.g. naphtyl, biphenyl, fluorenyl), and can be optionally substituted by one, two or three $C_1$-$C_6$ alkyl, $C_1$-$C_6$ haloalkyl, $C_1$-$C_6$ alkoxy, halogen, nitro, cyano, hydroxy, $C_1$-$C_6$ alkylcarbonyl, $C_1$-$C_6$ alkylcarbonyloxy, $C_1$-$C_6$ alkyloxycarbonyl, $C_1$-$C_6$ alkylsulfonyl, $C_1$-$C_6$ alkylsulfinyl and $C_1$-$C_6$ alkylthio group; and that the term "heteroaryl" means any radical from a 5 or 6 membered heterocycle, isolated or condensed with one benzene ring, with one ring heteroatom chosen from N, O or S, or with two ring heteroatoms chosen from the pairs N/N, N/O or N/S.

Patent application ES 91 00153 describes piperazinylcarboxamides of general formula:

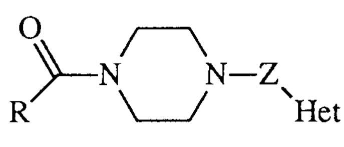

wherein Het is a 3-pyridyl or 4-pyridyl radical both optionally substituted by an halogen atom or a $C_1$-$C_4$ alkylthio group in any of the free positions except in the nitrogen, and in any case the pyridinic nitrogen can be substituted by a $C_1$-$C_4$ alkyl group or can be a N-oxide; Z is a carbonyl, thiocarbonyl, sulfonyl or -CHY- group wherein Y is hydrogen, $C_1$-$C_4$ alkyl, carbamoyl or phenylthio; R is a 3,4,5-trimethoxyphenyl group, $(Ph)_2CH-(CH_2)_q$-, $(Ph)_2C(OH)-(CH_2)_q$- wherein q is 0, 1 or 2, or $(Ph)_2CH-NR_1-(CH_2)_p$-wherein p is 1 or 2 and $R_1$ is hydrogen or $C_1$-$C_4$ alkyl.

The present invention describes novel compounds structurally related to those described therein, where the introduction of an alkyl group in the 2 position of the pyridine ring leads to new compounds which are potent PAF antagonists and show a better pharmacokinetic profile.

Description of the invention.

The present invention relates to new (2-alkyl-3-pyridyl)methylpiperazine derivatives of general formula **I**:

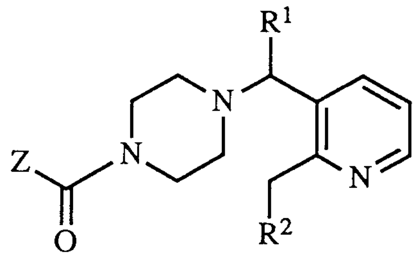

**I**

wherein:

**$R^1$** is -CN, $-CO_2R^3$, $-CON(R^3)_2$, $-COR^3$, $-C\equiv CR^3$ or $-(R^3)C=N-OR^3$, wherein $R^3$ is hydrogen or a $(C_1-C_6)$ alkyl group;

**$R^2$** is hydrogen or a $(C_1-C_3)$ alkyl group;

**Z** represents a group of formula:

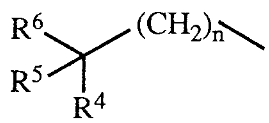

wherein

n is 0, 1 or 2;

$R^4$ represents hydrogen, $(C_1-C_6)$ alkyl, $Ar^1$ or Het; $Ar^1$ is a phenyl group optionally substituted by one or more groups chosen from halogen, trifluoromethyl, $(C_1-C_6)$ alkyl, $(C_1-C_6)$ alkoxy or hydroxy and Het is any radical from a 5- or 6-membered aromatic heterocycle with one ring heteroatom chosen from N, O or S, or with two ring heteroatoms chosen from the pairs N/N, N/O or N/S and which may be optionally substituted by a group chosen from halogen, $(C_1-C_6)$ alkyl, $(C_1-C_6)$ alkoxy or hydroxy;

$R^5$ represents hydrogen, $(C_1-C_6)$ alkyl, $Ar^1$, $Ar^2$, trifluoromethyl or hydroxy-$(C_1-C_6)$ alkyl such that

a) when $R^5$ represents hydrogen, $(C_1-C_6)$ alkyl or $Ar^1$, $R^6$ represents hydrogen, $(C_1-C_6)$ alkyl, $OR^7$, $Ar^3S(=O)_m-$, halogen, $R^3OC(=O)-$ or a group $NR^8R^9$, where:

$R^7$ is hydrogen, $(C_1-C_6)$ alkyl, $Ar^3$ or a group $(Ar^1)_2C=N-$;

m is 0, 1 or 2;

$R^8$ represents hydrogen, $(C_1-C_6)$ alkyl, $Ar^1$, $Ar^2$, $Ar^3$ or hydroxy; $R^9$ represents hydrogen, $(C_1-C_6)$ alkyl, $(C_1-C_6)$ alkenyl, $(C_1-C_6)$ alkynyl, $Ar^1$, $Ar^2$, $(R^3)_2NC(=O)-$, $HO(R^3)NC(=O)-$, $R^{10}OC(=O)-$, $Ar^1OC(=O)-$, $Ar^2OC(=O)-$, $Ar^1C(=O)-$, Het-$(C_1-C_6)$ alkyl, HetC(=O)-, $R^3C(=O)-$, fluorenyl, $R^{10}SO_2-$, $Ar^1SO_2-$ or $Ar^2SO_2-$, or the group $NR^8R^9$ forms a pyrrol, imidazol, or a saturated 5- or 6-membered heterocycle which may contain a second ring heteroatom selected from O, S, N and may be optionally substituted by one or two oxo groups and/or by a group $R^{10}$;

$Ar^2$ is a $(C_1-C_6)$ alkyl group substituted by one $Ar^1$ group;

$Ar^3$ is a $(C_1-C_6)$ alkyl group substituted by two $Ar^1$ that can be equal or different;

$R^{10}$ is a $(C_1-C_6)$ alkyl group;

b) when $R^5$ represents trifluoromethyl, $R^6$ represents hydrogen or $OR^3$, where $R^3$ has the above mentioned meaning;

c) when $R^5$ represents hydroxy-$(C_1-C_6)$ alkyl or $Ar^2$, $R^6$ represents a group $NR^8R^9$, where $R^8$ and $R^9$ have the above mentioned meaning; or **Z** represents a group of formula

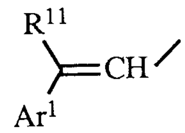

wherein $R^{11}$ represents hydrogen, $Ar^1$ or trifluoromethyl;
and the salts thereof.

The invention also provides the use of at least one compound of formula **I** or a pharmaceutically acceptable salt thereof for the manufacture of a medicament for the treatment or prophylaxys of PAF-mediated illnesses.

The invention further provides a pharmaceutical composition comprising an effective amount of at least one compound of formula **I** or a pharmaceutically acceptable salt thereof in admixture with a pharmaceutically acceptable excipient.

The invention still further provides processes for preparing the compounds of formula **I**, which in general terms comprise treating a compound of formula **II**

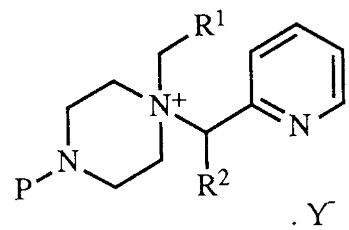

**II**

(wherein $R^1$ and $R^2$ have the previously defined meaning, P is an amine protecting group or ZCO, being Z as described above, and Y is an anion, such as chloride, bromide, iodide, alkylsulfonate or arylsulfonate) with a base, such as 1,8-diazabicyclo[5.4.0]undec-7-ene in acetonitrile, to give a compound of formula **III**

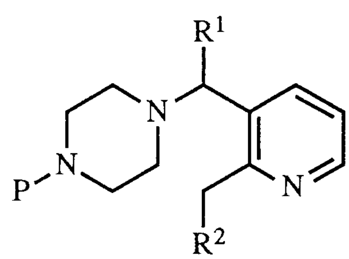

**III**

wherein $R^1$ and $R^2$ have the previously defined meaning, and P is ZCO (in which case **III** is **I**) or alternatively P is an amine protecting group, in which case it is deprotected to give the corresponding amine of formula **IV**

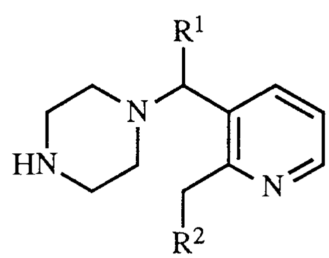

**IV**

(wherein $R^1$ and $R^2$ have the previously defined meaning), and then reacting **IV** with an acid **ZCOOH** or an equivalent acylating reagent, to give a compound of formula **I**

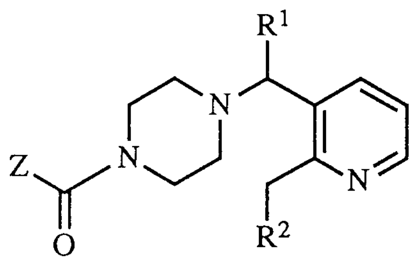

**I**

(wherein Z, $R^1$ and $R^2$ have the previously defined meaning);
and optionally, when in a compound of formula **I**, Z represents a group of formula

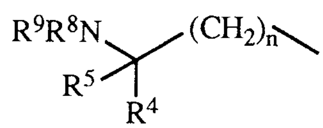

wherein $R^4$, $R^5$, $R^8$, $R^9$ and n have the above mentioned meaning, transforming the groups $R^8$ and/or $R^9$ in a compound of formula **I** into other groups $R^8$ and/or $R^9$.

Compounds of formula **I** have one or more asymmetric centers, which can give rise to different stereoisomers. The present invention covers both the individual stereoisomers as well as their mixtures.

Also an object of the present invention are the salts derived from compounds of formula **I** with pharmaceutically acceptable acids.

In the above definitions, the term "$C_1$-$C_n$ alkyl" means, unless otherwise specified, a linear or branched alkyl group that contains from one to n carbon atoms. Therefore, in the case in which n is 3 this term includes methyl, ethyl, propyl and isopropyl. When n is 6, it includes, among others, methyl, ethyl, propyl, isopropyl, butyl, isobutyl, sec-butyl, tert-butyl, pentyl, isopentyl, neopentyl, hexyl, isohexyl, 1-methylpentyl, 2-methylpentyl, 3-methylpentyl.

A "($C_1$-$C_6$)-alkoxy" group means a group derived from the union of a ($C_1$-$C_6$) alkyl group like the above mentioned to an oxygen atom of an ether functional group. Examples include methoxy, ethoxy, propoxy, isopropoxy, butoxy, isobutoxy, sec-butoxy, tert-butoxy, pentyloxy, isopentyloxy, neopentyloxy, hexyloxy, isohexyloxy, 1-methylpentyloxy, 2-methylpentyloxy, 3-methylpentyloxy.

The term "$Ar^1$" represents a phenyl group which may be optionally substituted by one or more groups chosen from halogen, ($C_1$-$C_6$) alkyl, hydroxy, ($C_1$-$C_6$) alkoxy or trifluoromethyl. In case there is more than one substituent, they can be the same or different. Examples include phenyl, 2-chlorophenyl, 3-chlorophenyl, 4-chlorophenyl, 2,4-dichlorophenyl, 4-fluorophenyl, 4-bromophenyl, 2-methylphenyl, 4-methylphenyl, 2-methoxyphenyl, 3-methoxyphenyl, 4-methoxyphenyl, 2,4-dimethoxyphenyl, 3,4,5-trimethoxyphenyl, 4-trifluoromethylphenyl, 4-hydroxyphenyl, 3,5-ditertbutyl-4-hydroxyphenyl.

The term "$Ar^2$" represents a group resulting from the substitution of one hydrogen atom of a ($C_1$-$C_6$)-alkyl group like the above mentioned by an $Ar^1$ group and includes, among others, benzyl, 1-phenylethyl, 2-phenylethyl, 3-phenylpropyl, 2-phenylpropyl, 1-methyl-2-phenylethyl, 4-phenylbutyl, 3-phenylbutyl, 2-phenylbutyl, 1-phenylbutyl, 5-phenylpentyl, 4-phenylpentyl, 3-phenylpentyl, 2-phenylpentyl, 6-phenylhexyl, 5-phenylhexyl, 4-phenylhexyl, of which benzyl and 2-phenylethyl are preferred and benzyl is more preferred.

The term "$Ar^3$" represents a group resulting from the substitution of two hydrogen atoms of a ($C_1$-$C_6$)-alkyl group like the above mentioned by two $Ar^1$ groups which can be the same or different. Examples include, among others, diphenylmethyl, 2,2-diphenylethyl, 1,1-diphenylethyl, 1,2-diphenylethyl, 3,3-diphenylpropyl, 2,2-diphenylpropyl, 1,1-diphenylpropyl, 3,2-diphenylpropyl, 1,3-diphenylpropyl, 1,2-diphenylpropyl, of which diphenylmethyl and 2,2-diphenylethyl are preferred and diphenylmethyl is more preferred.

The term "Het" represents any radical from a 5- or 6-membered aromatic heterocycle with one ring heteroatom chosen from N, O or S or with two ring heteroatoms chosen from the pairs N/N, N/O or N/S.

Examples of such heterocycles include pyrrole, imidazole, pyridine, pyrimidine, pyrazine, pyridazine, furane, thiophene, thiazole, oxazole, isoxazole. Said heterocycles may be optionally substituted by a group chosen from halogen, ($C_1$-$C_6$) alkyl, ($C_1$-$C_6$) alkoxy or hydroxy.

A "hydroxy-($C_1$-$C_6$)-alkyl" group means a group resulting from the substitution of one hydrogen atom of the above mentioned "$C_1$-$C_6$ alkyl" group by an hydroxyl group. Examples include, among others, hydroxymethyl, 2-hydroxyethyl, 1-hydroxyethyl, 3-hydroxypropyl, 2-hydroxypropyl, 1-methyl-2-hydroxyethyl, 4-hydroxybutyl, 3-hydroxybutyl, 2-hydroxybutyl, 1-hydroxybutyl, 2-methyl-3-hydroxypropyl, 1-methyl-3-hydroxypropyl, 1,1-dimethyl-2-hydroxyethyl, 5-hydroxypentyl, 4-hydroxypentyl, 3-hydroxypentyl, 2-hydroxypentyl, 1-methyl-4-hydroxybutyl, 2-methyl-4-hydroxybutyl, 3-methyl-4-hydroxybutyl, 6-hydroxy-hexyl, 5-hydroxyhexyl, 4-hydroxyhexyl, 1-methyl-5-hydroxypentyl, 2-methyl-5-hydroxypentyl, 3-methyl-5-hydroxypentyl, 4-methyl-5-hydroxypentyl.

The term "($C_1$-$C_6$) alkenyl" means a linear or branched alkyl chain containing from 2 to 6 carbon atoms and which further contains one or more double bonds. Examples include ethenyl, 1-propenyl, 2-propenyl, isopropenyl, 1-butenyl, 2-butenyl, 3-butenyl, 1,3-butadienyl, 1-pentenyl, 2-pentenyl, 3-pentenyl, 4-pentenyl, 1,3-pentendienyl, 2,4-pentendienyl, 1-hexenyl, 2-hexenyl, 3-hexenyl, 4-hexenyl and 5-hexenyl.

The term "($C_1$-$C_6$) alkynyl" means a linear or branched alkyl chain containing from 2 to 6 carbon atoms and which further contains one or more triple bonds. Examples include ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl and 5-hexynyl.

A "Het-($C_1$-$C_6$)-alkyl" group represents a group resulting from the substitution of one hydrogen atom of a ($C_1$-$C_6$)-alkyl group like the above mentioned by an Het group as above defined. Examples include among others, 2-pyridylmethyl, 3-pyridylmethyl, 4-pyridylmethyl, 2-pyrazinylmethyl, 2-furylmethyl, 3-furylmethyl, 2-pyrimidinylmethyl, 4-pyrimidinylmethyl, 3-pyridazinylmethyl, 4-pyridazinylmethyl, 2-pyrrolylmethyl, 3-pyrrolylmethyl, 1-(3-pyridyl)ethyl, 2-(3-pyridyl)ethyl, 3-(3-pyridyl)propyl.

In a compound of formula **I**, $NR^8R^9$ can be a saturated 5- or 6-membered heterocycle which may contain a second ring heteroatom selected from O, S or N. Examples include among others, pyrrolidine, piperidine, imidazoline, piperazine, oxazolidine, morpholine and thiazolidine. Said heterocycle may be optionally substituted by one or two oxo groups and/or by a group ($C_1$-$C_6$) alkyl.

Preferred embodiments of the present invention are those compounds of formula **I** where:

**$R^1$** is -CN;

**$R^2$** is hydrogen; and

Z has the previously defined meaning.

More preferred embodiments of the present invention are the following groups of compounds:

I) Those compounds of formula **I** where:

**$R^1$** is CN;

**$R^2$** is hydrogen; and

**Z** represents a group of formula

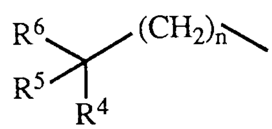

wherein

n is 1;

$R^4$ represents $Ar^1$ or Het, wherein $Ar^1$ and Het have the previously defined meaning;

$R^5$ represents ($C_1$-$C_6$) alkyl or $Ar^1$; and

$R^6$ represents hydrogen, ($C_1$-$C_6$) alkyl, $OR^3$ or $NH_2$, where $R^3$ has the previously defined meaning.

II) Those compounds of formula **I** where:

**$R^1$** is CN;

**$R^2$** is hydrogen; and

**Z** represents a group of formula

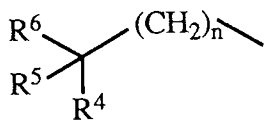

wherein

n is 1;

$R^4$ represents $Ar^1$ or Het, wherein $Ar^1$ and Het have the previously defined meaning;

$R^5$ represents trifluoromethyl; and

$R^6$ represents hydrogen or $OR^3$, where $R^3$ has the previously defined meaning.

III) Those compounds of formula **I** where:

**$R^1$** is CN;

**$R^2$** is hydrogen; and

**Z** represents a group of formula

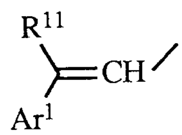

wherein $R^{11}$ represents $Ar^1$ or trifluoromethyl, where $Ar^1$ has the previously defined meaning.

IV) Those compounds of formula **I** where:

**$R^1$** is CN;

**$R^2$** is hydrogen;

**Z** represents a group of formula

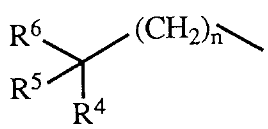

wherein

n is 0 or 1;

$R^4$ and $R^5$ represent each one independently hydrogen or $(C_1\text{-}C_6)$ alkyl; and

$R^6$ represents a group $NR^8R^9$, where:

$R^8$ represents $Ar^3$, being $Ar^3$ as above defined;

$R^9$ represents hydrogen, $(C_1\text{-}C_6)$ alkyl, $(R^3)_2NC(=O)$-, $R^{10}OC(=O)$-, $R^3C(=O)$- or $R^{10}SO_2$-, being $R^3$ and $R^{10}$ as above defined.

V) Those compounds of formula **I** where:

**$R^1$** is CN;

**$R^2$** is hydrogen;

**Z** represents a group of formula

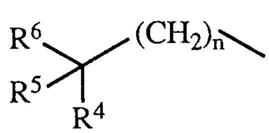

wherein

n is 1;

$R^4$ represents $Ar^1$ or Het, wherein $Ar^1$ and Het have the previously defined meaning;

$R^5$ represents hydrogen or $(C_1\text{-}C_6)$ alkyl; and

$R^6$ represents a group $NR^8R^9$, where $R^8$ represents hydrogen, $(C_1\text{-}C_6)$ alkyl, $Ar^1$ or hydroxy; $R^9$

represents hydrogen, $(C_1\text{-}C_6)$ alkyl, $(C_1\text{-}C_6)$ alkenyl, $(C_1\text{-}C_6)$alkynyl, $Ar^2$, $(R^3)_2NC(=O)$-, $HO(R^3)NC(=O)$-, $R^{10}OC(=O)$-, $Ar^1OC(=O)$-, $Ar^2OC(=O)$-, $Ar^1C(=O)$-, Het-$(C_1\text{-}C_6)$ alkyl, $HetC(=O)$-, $R^3C(=O)$-, $R^{10}SO_2$-, $Ar^1SO_2$- or $Ar^2SO_2$-, or the group $NR^8R^9$ forms an heterocycle of the formula (i), (ii), (iii), (iv), (v), (vi) or (vii):

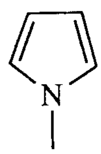
(i)

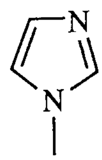
(ii)

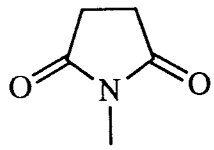
(iii)

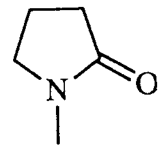
(iv)

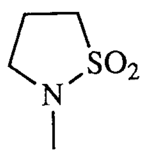
(v)

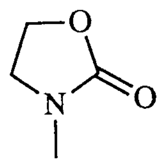
(vi)

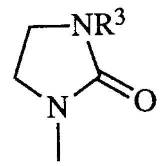
(vii)

wherein $R^3$, $R^{10}$, $Ar^1$, $Ar^2$ and Het have the previously defined meaning.

The formulae of some specific compounds are represented below, together with the number corresponding to the example in which their preparation is described:

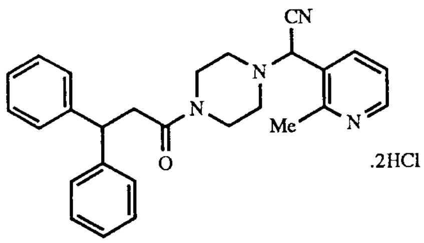

1

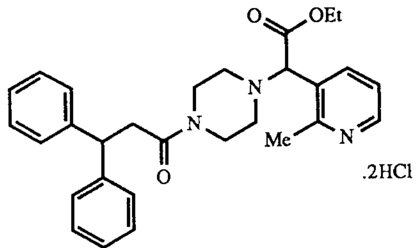

2

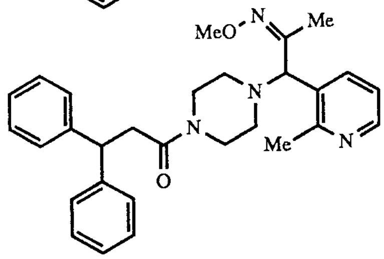

3

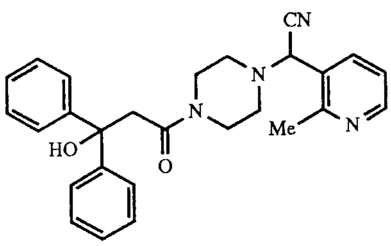

4

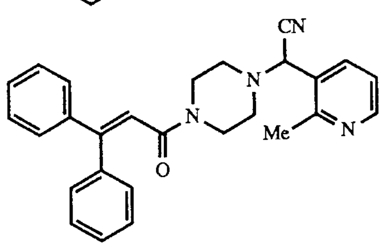

5

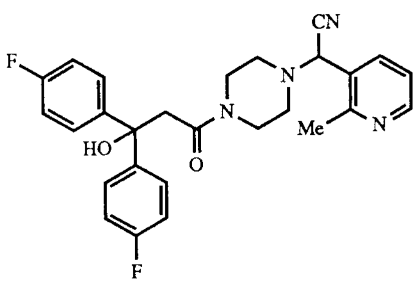

6

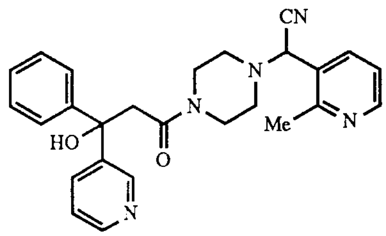

7

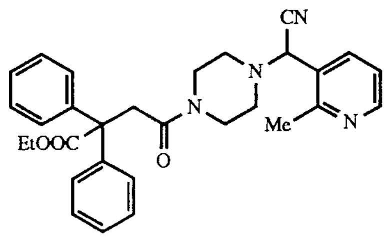

8

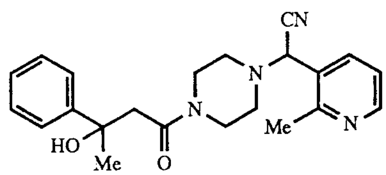

9

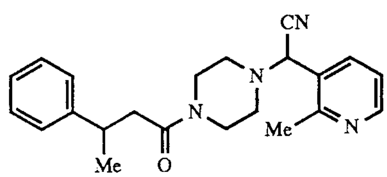

10

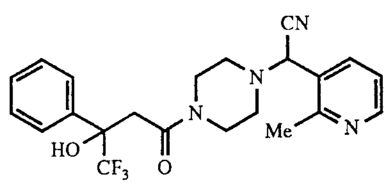

11

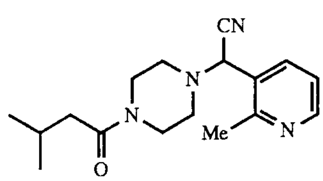

12

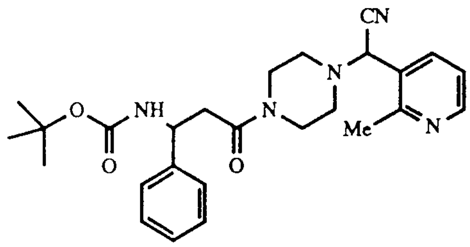

13

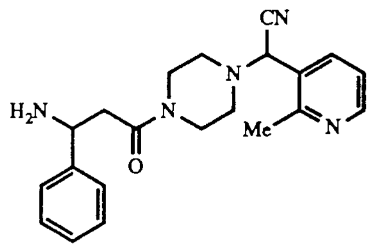

14

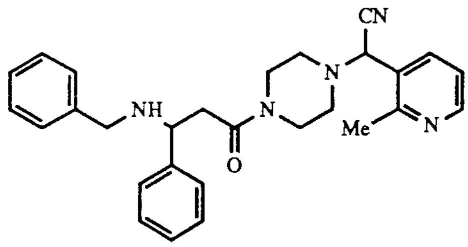

15

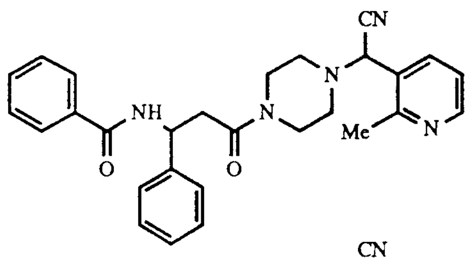

16

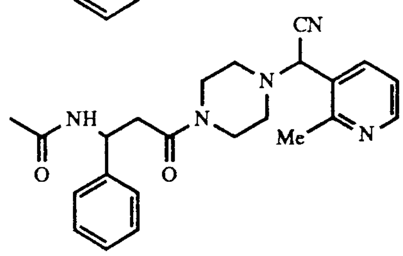

17

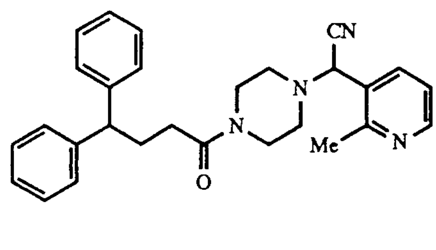

18

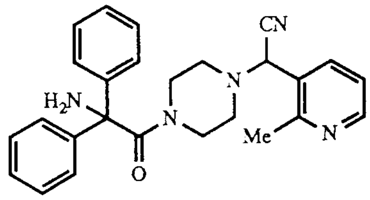

**19**

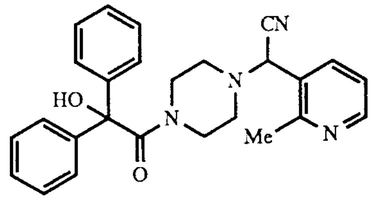

**20**

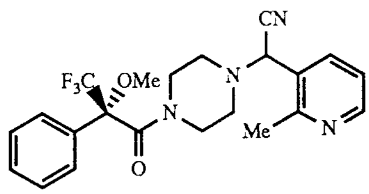

**21**

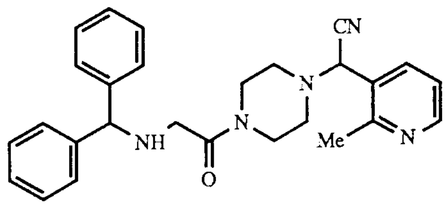

**22**

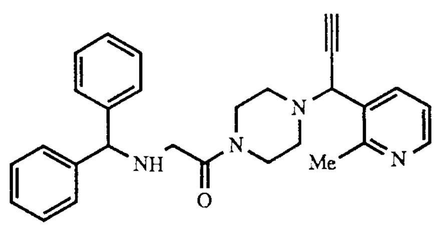

**23**

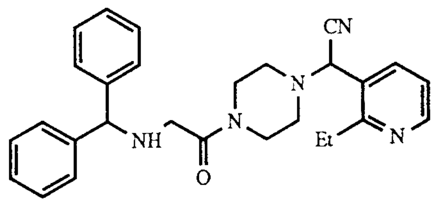

**24**

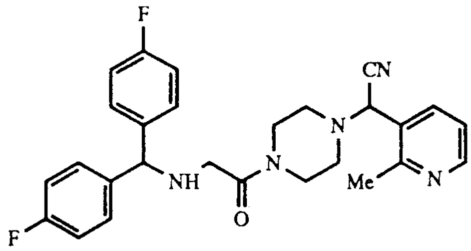

25

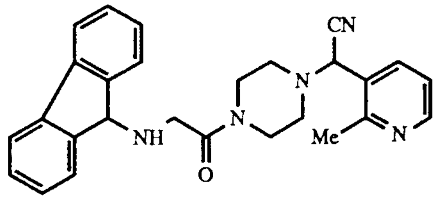

26

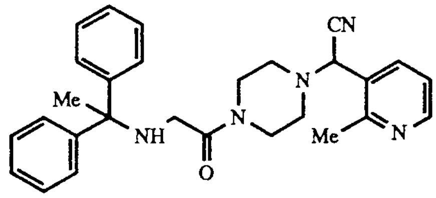

27

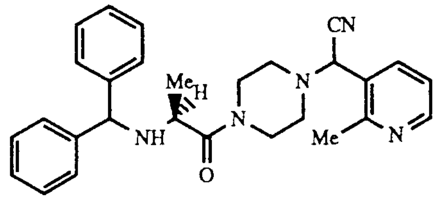

28

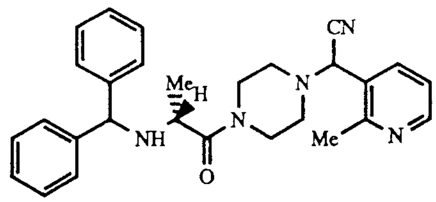

29

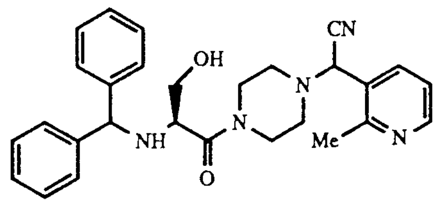

30

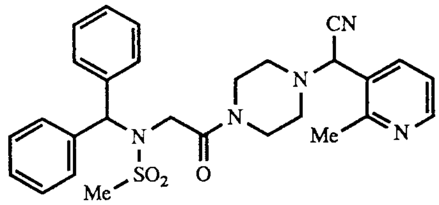
31

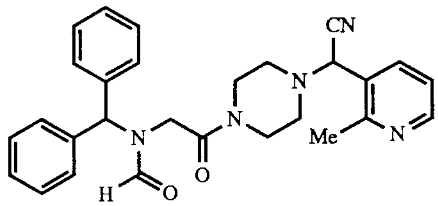
32

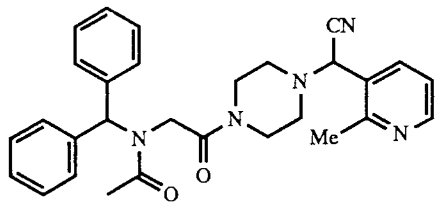
33

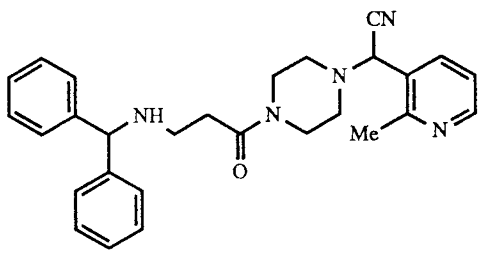
34

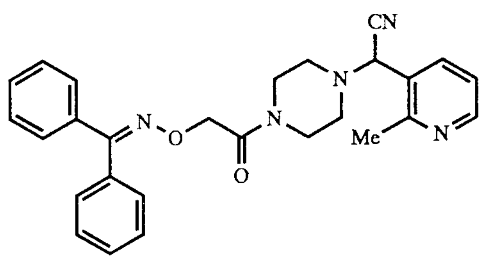
35

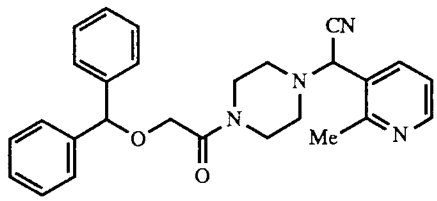
36

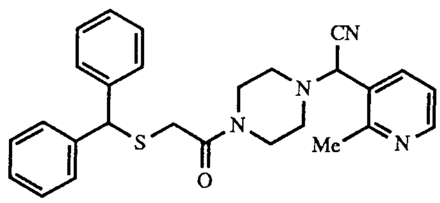

37

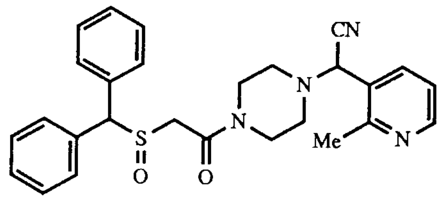

38

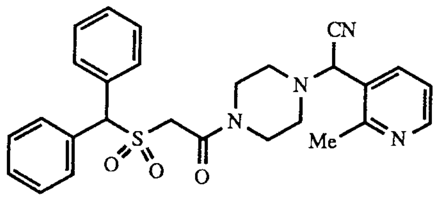

39

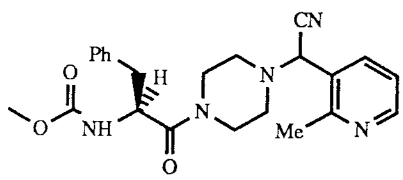

40

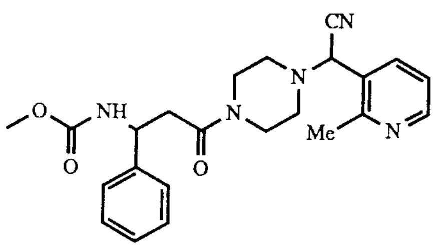

41

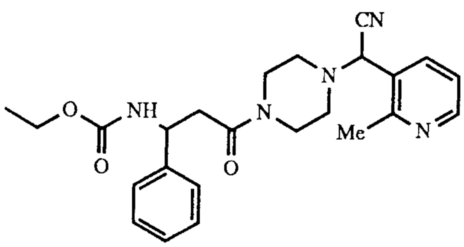

42

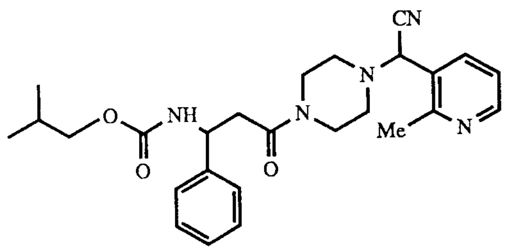

43

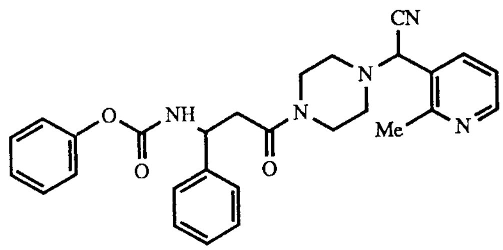

44

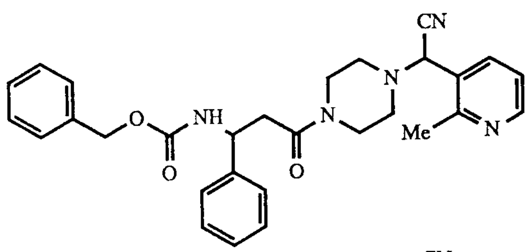

45

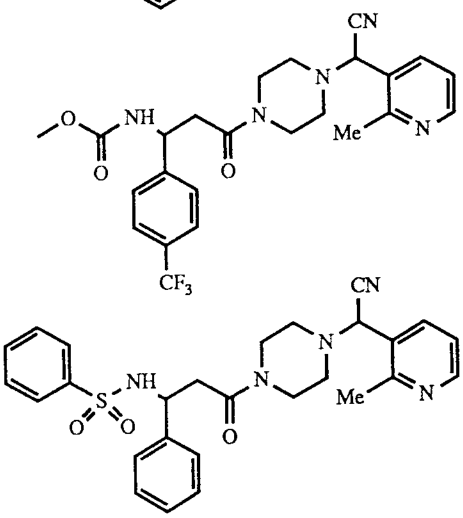

46

47

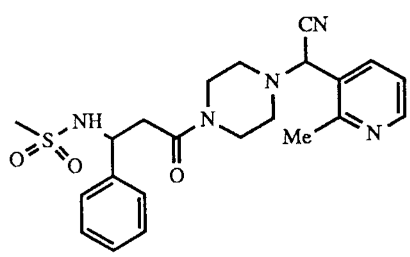

48

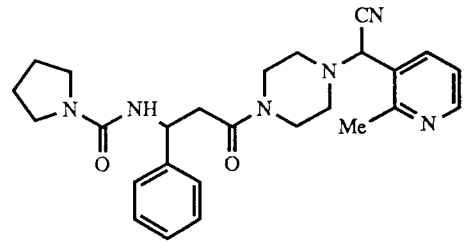

49

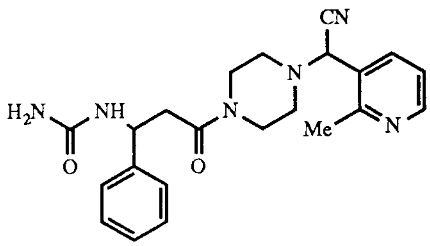

50

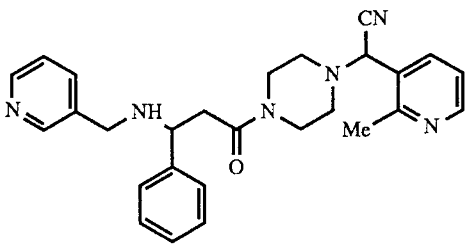

51

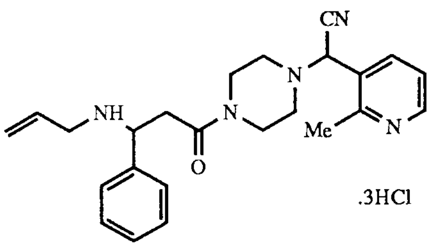

52

.3HCl

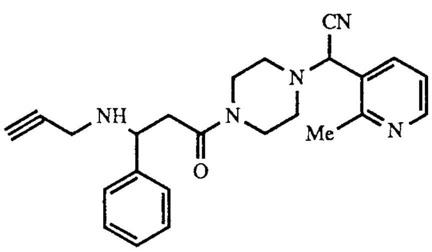

53

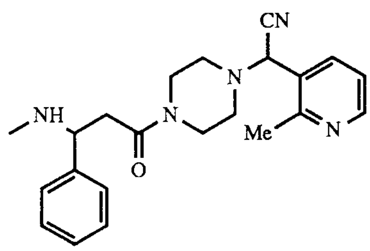

54

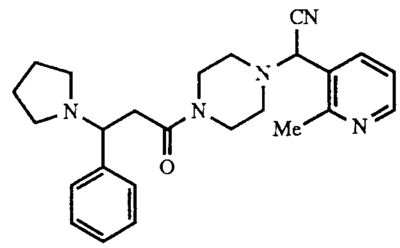

55

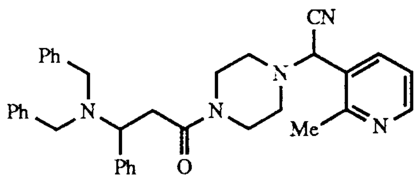

56

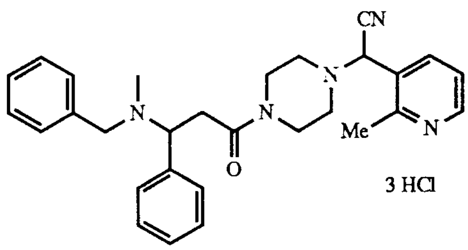

57

3 HCl

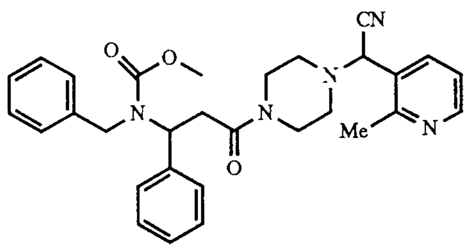

58

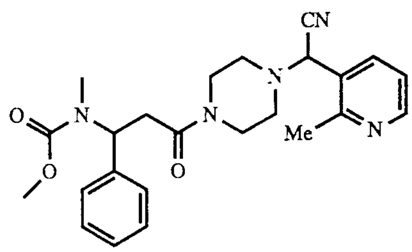

59

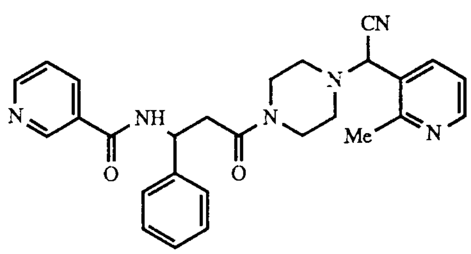

60

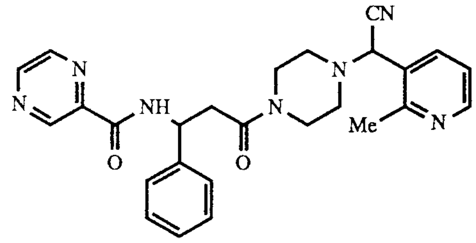

61

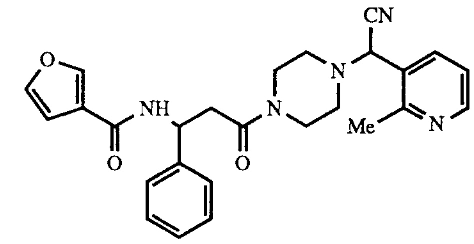

62

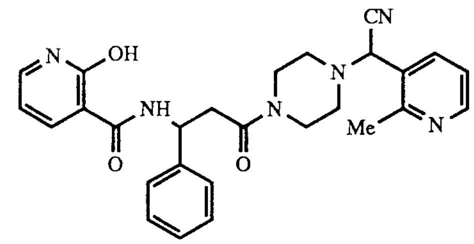

63

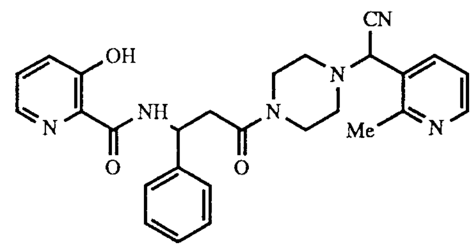

64

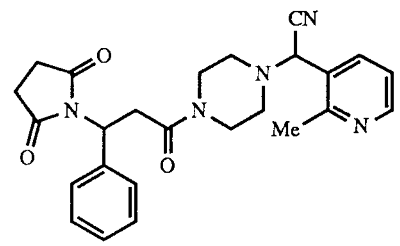

65

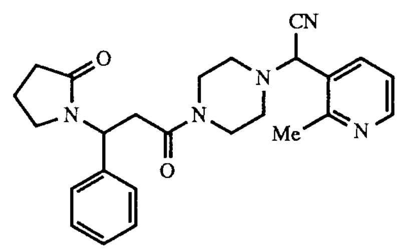

66

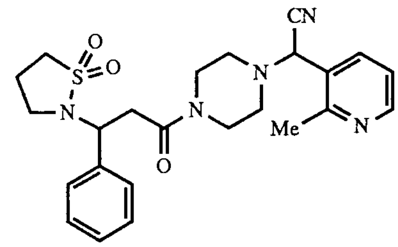

67

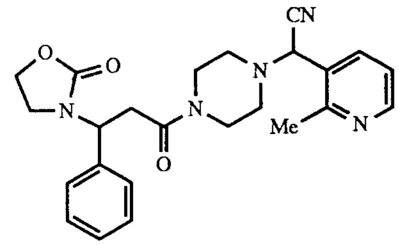

68

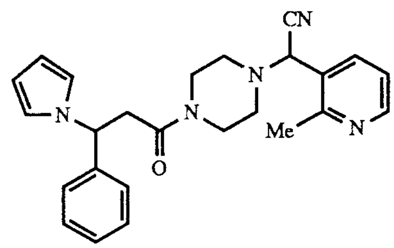

69

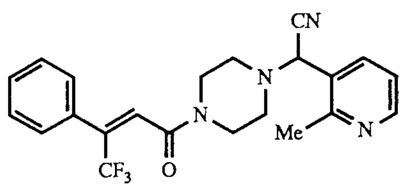

70

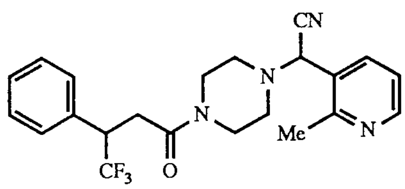

71

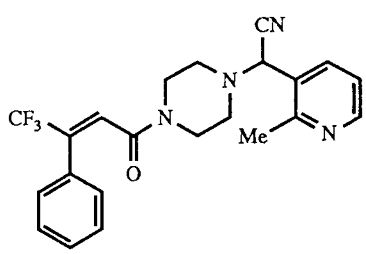

72

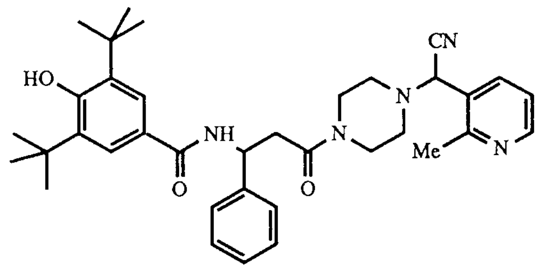

73

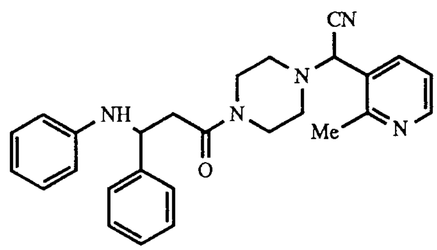

74

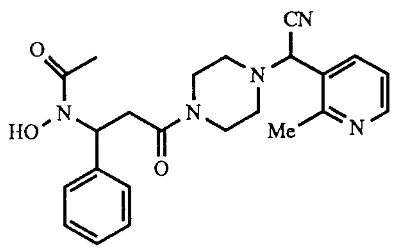

75

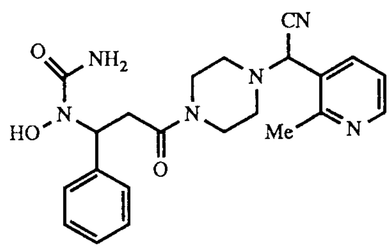

76

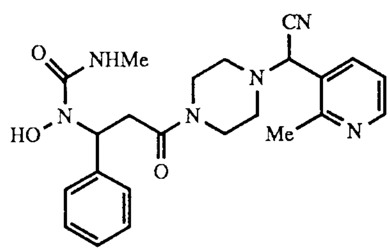

77

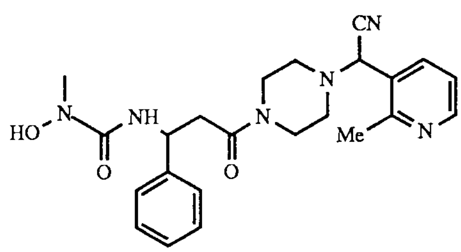

78

Of the compounds listed above, the following are preferred, that is to say Compounds No. 1, 4, 5, 11, 15, 16, 17, 22, 34, 41, 44, 48, 60, 66, 68, 74, 75, 76, 77 and 78.

The most preferred compounds are:

No. 4: 1-(3,3-Diphenyl-3-hydroxypropionyl)-4-[(2-methyl-3-pyridyl)cyanomethyl]piperazine;

No. 11: 1-(3-Phenyl-3-hydroxy-3-(trifluoromethyl)propionyl)-4-[(-2 methyl-3-pyridyl)cyanomethyl]-piperazine;

No. 15: 1-[3-(N-Benzylamino)-3-phenylpropionyl]-4-[(2-methyl-3-pyridyl)cyanomethyl]piperazine;

No. 17: 1-[3-(N-acetylamino)-3-phenylpropionyl]-4-[(2-methyl-3-pyridyl)cyanomethyl]piperazine;

No. 22: 1-[N-(Diphenylmethyl)aminoacetyl]-4-[(2-methyl-3-pyridyl)cyanomethyl]piperazine;

No. 41: 1-[3-(N-(Methoxycarbonyl)amino)-3-phenylpropionyl]-4-[(2-methyl-3-pyridyl)cyanomethyl]-piperazine;

No. 44: 1-[3-(N-(Phenoxycarbonyl)amino)-3-phenylpropionyl]-4-[(2-methyl-3-pyridyl)cyanomethyl]-piperazine;

No. 68: 1-[3-(2-oxo-3-oxazolidinyl)-3-phenylpropionyl]-4-[(2-methyl-3-pyridyl)cyanomethyl]piperazine;

No. 74: 1-[3-(N-phenylamino)-3-phenylpropionyl]-4-[(2-methyl-3-pyridyl)cyanomethyl]piperazine;

No. 75: 1-[3-(N-acetyl-N-hydroxyamino)-3-phenylpropionyl]-4-[(2-methyl-3-pyridyl)cyanomethyl]-piperazine;

No. 76: 1-[3-(N-carbamoyl-N-hydroxyamino)-3-phenylpropionyl]-4-[(2-methyl-3-pyridyl)cyanomethyl]-piperazine.

The compounds of the present invention contain one or more basic nitrogen atoms and, consequently, they can form salts, which are also included in the present invention. There is no limitation on the nature of these salts but pharmaceutically acceptable ones are preferred. Examples of these salts include: salts with an inorganic acid such as hydrochloric acid, hydrobromic acid, hydriodic acid, nitric acid, perchloric acid, sulfuric acid or phosphoric acid; and salts with an organic acid, such as methanesulfonic acid, trifluoromethanesulfonic acid, ethanesulfonic acid, benzenesulfonic acid, *p*-toluenesulfonic acid, fumaric acid, oxalic acid or maleic acid.

The compounds of the present invention can exist as different diastereoisomers and/or optical isomers because the carbon in the $\alpha$ position to the substituent $R^1$ is chiral and in some cases there are one or more additional chiral centers. Diastereoisomers can be separated by conventional techniques such as chromatography or fractional crystallization. The optical isomers can be resolved using any of the conventional techniques of optical resolution to give optically pure isomers. Such a resolution can be performed in any chiral synthetic intermediate as well as in the products of general formula **I**. The optically pure isomers can also be individually obtained using enantiospecific synthesis. The present invention covers the individual isomers as well as their mixtures (e.g. racemic mixtures), being irrelevant if they have been obtained by synthesis or have been prepared by physically mixing them up.

The invention also provides processes for preparing the compounds of formula **I**. The precise method used for the preparation of a given compound of the present invention may vary depending on its chemical structure. Scheme 1 illustrates the general method for their preparation.

SCHEME 1

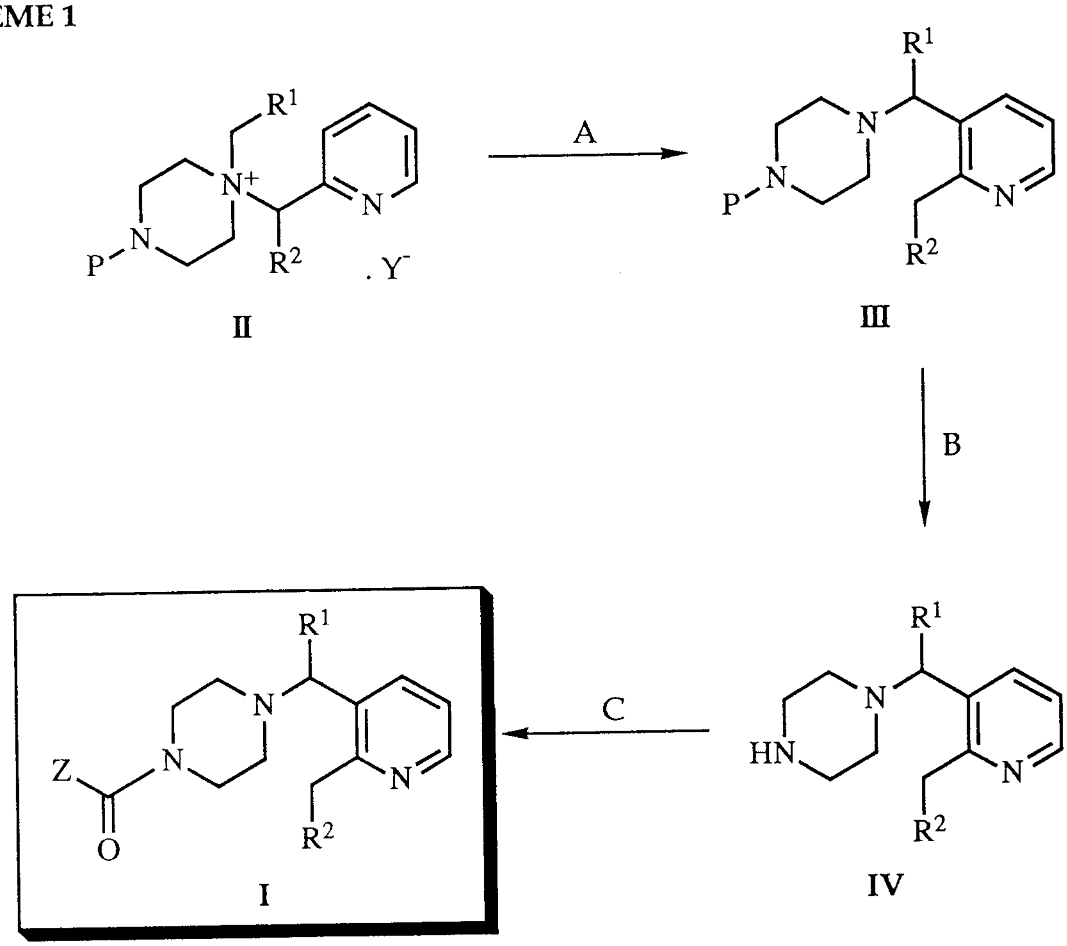


II III IV I

Wherein Z, $R^1$, $R^2$, P and Y have the previously defined meaning.

In Step A, compounds of formula **III** are prepared from compounds of formula **II** by treatment with a base in a solvent compatible with the reaction conditions. We have found that the reaction works well using as a base 1,8-diazabicyclo[5.4.0]undec-7-ene in acetonitrile although other combinations of base add solvent can also be used, such as potassium *tert*-butoxide in dimethylsulfoxide/*tert*-butanol, sodium hydride in dimethylformamide, sodium ethoxide in ethanol, sodium hydroxide in water or lithium diisopropylamide in tetrahydrofuran. The reaction is carried out at a temperature ranging from 0 ° C to room temperature, and during a period of time between 1 and 24 hours. The desired product can be isolated and purified by conventional methods such as flash chromatography.

When the starting product has P = ZCO, Step A leads directly to compounds of formula **I**.

When P is an amine protecting group, compounds of formula **III** are converted into the free amine **IV** - (Step B) by deprotection of the piperazine nitrogen. The reagents and the reaction conditions needed will depend on the nature of the protecting group used. If the protecting group is *tert*-butoxycarbonyl, deprotection can be carried out by treatment with a 3.7N dioxane/hydrochloric acid solution in a suitable solvent such as chloroform at a temperature between 0 ° C and room temperature and during a period of time from 30 min to 2 h. The desired compound is obtained by concentration to dryness, treatment with sodium hydroxide and extraction with chloroform.

Compounds of formula **I** are prepared by a dehydration procedure between amines of formula **IV** and the carboxylic acids of general formula **ZCOOH** (Step C), wherein Z has the previously defined meaning. This dehydration process can be carried out by using any conventional reaction of amide bond formation, such as the following processes:

a) By reaction between amines **IV** and an acid of general formula **ZCOOH** in the presence of dicyclohexylcarbodiimide and 1-hydroxybenzotriazole in a suitable solvent; as examples of suitable solvents can be mentioned dioxane, tetrahydrofuran, acetonitrile, chloroform and N,N-dimethylformamide. The reaction is performed at a temperature ranging from 0 to 60 ° C during a period of time from 6 to 24 hours.

b) By reaction of amines **IV** with an acid chloride or anhydride derived from an acid of general formula **ZCOOH** in the presence of a proton scavenger amine, such as pyridine or triethylamine, in a suitable solvent such as methylene chloride or chloroform, or the same proton scavenger amine can be used as solvent. The reaction is carried out at a temperature between 0 ° C and that of the boiling point of the solvent, during a period of time from 6 to 24 hours. The compounds thus obtained are purified by flash chromatography or recrystallization.

Furthermore, when in a compound of formula **I**, Z represents a group of formula

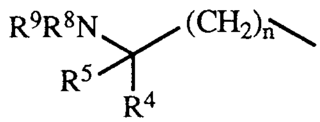

wherein $R^4$, $R^5$, $R^8$, $R^9$ and n have the above mentioned meaning, **I** may also be obtained from a compound of formula **I** wherein one of $R^8$ or $R^9$ is an amine protecting group, such as a *tert*-butoxycarbonil group, by performing the following steps:

a) Deprotection of the nitrogen atom of the group $NR^8R^9$ in the same reaction conditions mentioned above for Step B of Scheme 1; and

b) Subsequent reaction of the resulting free nitrogen atom; examples of said reactions include among others alkylation, acylation, condensation to form an heterocycle, formation of sulfonamide derivatives, formation of carbamates and ureas. These reactions can be carried out under the conventional reaction conditions used in organic chemistry for these kind of transformations.

The preparation of a compound of formula **II** starts from a compound of formula **V** and is outlined in Scheme 2:

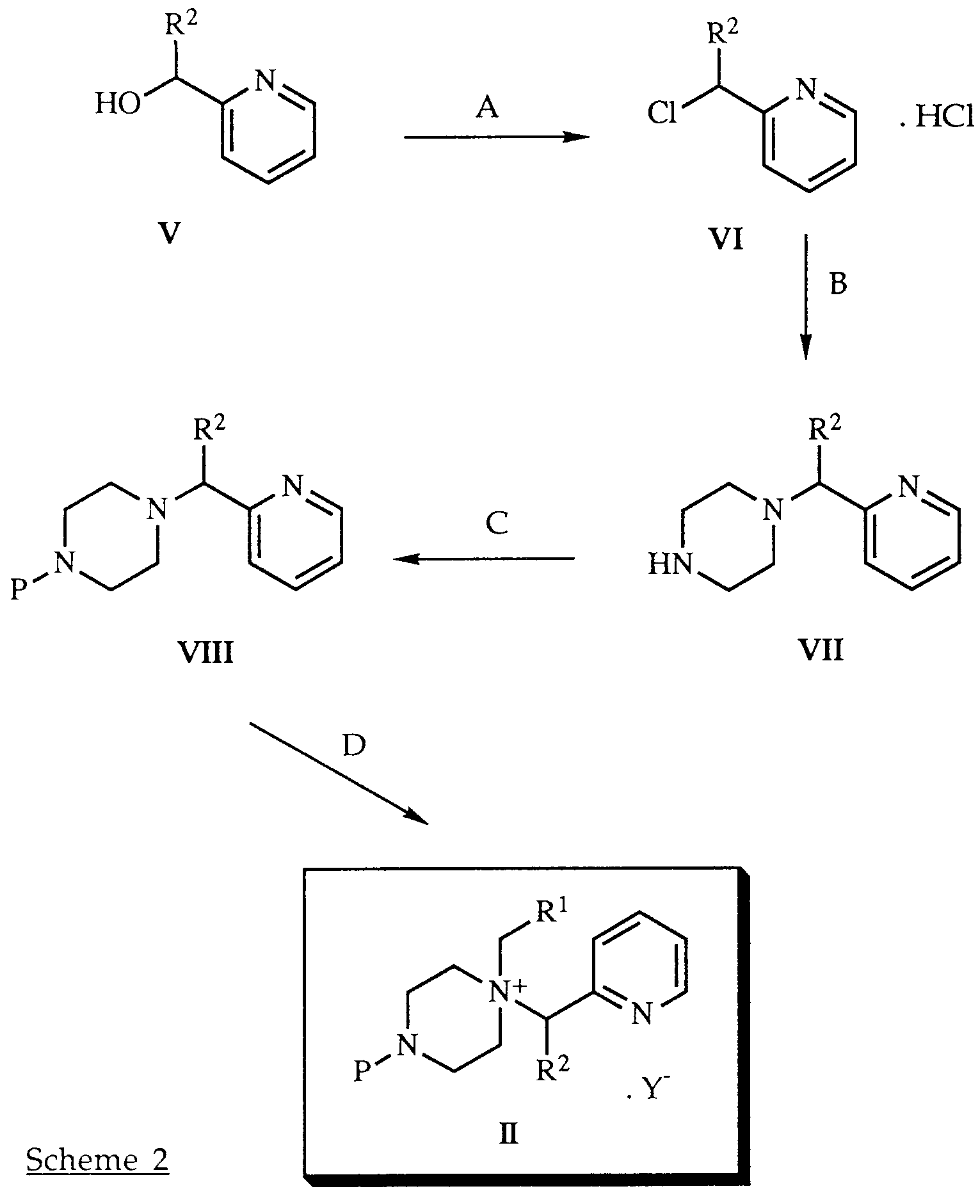


Scheme 2

Wherein: $R^1$, $R^2$, P and Y have the previously defined meaning.

The reaction of a compound of formula **V** (Step A of Scheme 2) with thionyl chloride in a suitable solvent such as methylene chloride at a temperature between 0° C and that of the boiling point of the solvent and during a period of time from 2 to 24 h leads to a compound of formula **VI**.

The reaction of a compound of formula **VI** (Step B) with an excess of piperazine monohydrochloride in aqueous solution leads to a compound of formula **VII**. The reaction is carried out at room temperature and during a period of time ranging from 6 to 48 hours.

In Step C, the amine of formula **VII** is allowed to react with an acid of general formula **ZCOOH**, wherein Z has the previously defined meaning, by a dehydration process as described above, or alternatively, the piperazine nitrogen of the compound of formula **VII** is protected to give a compound of formula **VIII**. Although any amine protecting group stable to the reaction conditions can be used in principle, we have found that *tert*-butoxycarbonyl group (BOC) works satisfactorily. The introduction of the *tert*-butoxycarbonyl group is performed by reacting the compound of formula **VII** with di-*tert*-butyl carbonate in the presence of sodium hydroxide in a suitable solvent, such as 1:1 mixtures of tetrahydrofuran and water, at a temperature between 0° C and room temperature and during a period of time from 30 min to 2 h.

The reaction of a compound of formula **VIII** (Step D) with an alkylating agent of general formula $Y-CH_2-R^1$ (wherein $R^1$ and Y have the previously defined meaning) in a suitable solvent such as acetonitrile at a temperature between 0° C and room temperature and during a period of time from 1 to 5 days leads to the compounds of general formula **II**. They can be purified by flash chromatography.

Acids of general formula **ZCOOH** are either commercial, or widely described in the literature or can be prepared by methods similar to those described, starting from commercially available products.

Compounds of general formula **I**, being potent PAF antagonists, are useful as a preventive and therapeutic drugs for the treatment of circulatory diseases caused by PAF, such as thrombosis, cerebral apoplexy ( e.g. cerebral hemorrhage, cerebral thrombosis), myocardial infarction, angina pectoris, thrombotic phlebitis, thrombocitopenic purple, nephritis (e.g. glomerular nephritis), diabetic nephrosis, shock (e.g. endotoxin shock observed after severe infection or postoperatively, intravascular agglutination syndrome caused by endotoxin, anaphylactic shock, hemorrhagic shock); digestive tract diseases caused by PAF (e.g. gastric ulcer, ulcerative colitis); diseases related to allergy and inflammation (e.g. asthma, dermatitis, urticaria, psoriasis); pneumonia; rejection due to increased PAF production after implantations of organs; and postoperative organodysfunction (e.g. in heart, liver and kidney). It can also be used for contraception of female mammals by suppressing cell division and/or ovoimplantation on the uterus, in the treatment of endometriosis and in the prevention or treatment of hyperendothelinemia induced by excess secretion of endothelin.

According to the activity of the compounds od formula **I**, the present invention further provides compositions that contain one or more of the compounds of the present invention, together with an excipient and optionally other auxiliary agents, if necessary. The compounds of the present invention can be administered in different pharmaceutical formulation, the precise nature of which, as it is well known, will depend upon the chosen route of administration and the nature of the pathology to be treated.

Thus, solid compositions according to the present invention for oral administration include compressed tablets, dispersible powders, granules and capsules. In tablets, one or more of the active component(s) is admixed with at least one inert diluent such as lactose, starch, mannitol, microcrystalline cellulose or calcium phosphate; granulating and disintegrating agents for example corn starch, gelatine, microcrystalline cellulose or polyvinylpyrrolidone; and lubricating agents for example magnesium stearate, stearic acid or talc. The tablets may be coated by known techniques to delay disintegration and absorption in the gastrointestinal tract and, thereby, provide a sustained action over a longer period. Gastric film-coated or enteric film-coated can be made with sugar, gelatin, hydroxypropylcellulose, or acrylic resins. Tablets with a sustained action may also be obtained using an excipient which provides regressive osmosis, such as the galacturonic acid polymers. Formulations for oral use may also be presented as hard capsules of absorbable material, such as gelatin, wherein the active ingredient is mixed with an inert solid diluent and lubricating agents, or pasty materials, such as ethoxylated saturated glycerides. Soft gelatin capsules are possible wherein the active ingredient is mixed with water or an oil medium, for example peanut oil, liquid paraffin or olive oil.

Dispersible powders and granules suitable for preparation of a suspension by the addition of water provide the active ingredient in admixture with a dispersing or wetting agent, a suspending agent, such as sodium carboxymethylcellulose, sodium alginate, polyvinylpirrolidone, gum tragacanth, xantham gum, gum acacia, and one or more preservatives, such as methyl or *n*-propyl-*p*-hydroxybenzoate. Additional excipients, for example sweetening, flavoring and coloring agents may also be present.

Liquid compositions for oral administration include emulsions, solutions, suspensions, syrups and elixirs containing commonly used inert diluents, such as distilled water, ethanol, sorbitol, glycerol, or propylene glycol. Such compositions may also comprise adjuvants such as wetting agents, suspending agents, sweetening, flavoring, perfuming, preserving agents and buffers.

Other compositions for oral administration include spray compositions, which may be prepared by known methods and which comprise one or more active compound(s). The spray compositions will contain a suitable propellent.

Preparations for injection according to the present invention for parenteral administration include sterile aqueous or non-aqueous solutions, suspensions or emulsions, in a non-toxic parentally-acceptable diluent or solvent. Examples of aqueous solvents or suspending media are distilled water for injection, the Ringer's solution, and isotonic sodium chloride solution. Examples of non-aqueous solvents or suspending media are propylene glycol, polyethylene glycol, vegetable oils such as olive oil, or alcohols such as ethanol. These compositions may also include adjuvants such as wetting, preserving, emulsifying and dispersing agents. They may be sterilized by one of the known methods or manufactured in the form of sterile solid compositions which can be dissolved in sterile water or some other sterile injectable medium immediately before use. When all of the components are sterile, the injectables will maintain the sterility if they are manufactured in sterile environment.

A compound of the invention may also administered in the form of suppositories for rectal administration of the drug, or as creams, ointments jellies, solutions or suspensions for topical use and pessaries for vaginal administration.

The dosage and frequency of dose may vary depending upon symptoms, age and body weight of the patient, as well as upon the route of administration, but, in general, the compounds of the invention may be administered orally in a daily dose of from 1-1000 mg for an adult, preferably a dosage from 5-250 mg, which may be administered either as a single dose or as divided doses. A preferred dosage for human patients is from 0.005 to 5 mg/kg of body weight, more preferably from 0.05 to 1 mg/kg of body weight.

Following are some representative preparations for tablets capsules, syrups, aerosols and injectables. They can be prepared following standard procedures and they are useful as inhibitors of the platelet activating factor.

| **Tablets** | |
|---|---|
| Compound of formula **I** | 100 mg |
| Dibasic calcium phosphate | 125 mg |
| Sodium starch glycolate | 10 mg |
| Talc | 12,5 mg |
| Magnesium stearate | 2,5 mg |
| | 250,0 mg |

| **Hard gelatin capsules** | |
|---|---|
| Compound of formula **I** | 100 mg |
| Lactose | 197 mg |
| Magnesium stearate | 3 mg |
| | 300 mg |

| **Syrup** | |
|---|---|
| Compound of formula **I** | 0,4 g |
| Sucrose | 45 g |
| Flavoring agent | 0,2 g |
| Sweetening agent | 0,1 g |
| Water to | 100 mL |

| **Aerosol** | |
|---|---|
| Compound of formula **I** | 4 g |
| Flavoring agent | 0,2 g |
| Propylene glycol to | 100 mL |
| Suitable propellent to | 1 unit |

| **Injectable preparation** | |
|---|---|
| Compound of formula **I** | 100 mg |
| Benzylic alcohol | 0,05 mL |
| Propylene glycol | 1 mL |
| Water to | 5 mL |

The compounds of the present invention are useful in the treatment of PAF-mediated illnesses, as illustrated in the following examples of pharmacological tests.

**EXAMPLE OF PHARMACOLOGICAL TEST 1**

Inhibition of platelet aggregation induced by PAF.

The blood is obtained by cardiac puncture of male New Zealand albino rabbits (between 2 and 2.5 Kg of weight) and coagulation is prevented by adding 1 part of 3.16% sodium citrate dihydrate in 9 parts of blood. The platelet rich plasma (PRP) is prepared by blood centrifugation at 250xg for 10 min. at 4°C and it is diluted with platelet poor plasma (PPP) obtained by additional centrifugation at 3000xg for 10 min. The amount of platelets is adjusted to $3x10^{-5}/mm^3$. The platelet aggregation induced by PAF ($C_{18}$, prepared in our laboratory) (16 nM, final) is determined by the Born nephelometric technique (*J. Physiol.*, **1962,** 162, 67) using an aggregometer Chrono-log 500. The activities of the inhibitors are expressed as $IC_{50}$ values, that is to say the concentration of the drug needed to inhibit the platelet aggregation in a 50%. The results are shown in table I:

TABLE I

| Compound Nº | $IC_{50}$ (μM) |
|---|---|
| 1 | 0.018 |
| 4 | 0.017 |
| 5 | 0.026 |
| 9 | 0.036 |
| 11 | 0.014 |
| 15 | 0.0091 |
| 16 | 0.0091 |
| 17 | 0.038 |
| 18 | 0.080 |
| 22 | 0.085 |
| 28 | 0.013 |
| 34 | 0.11 |
| 36 | 0.042 |
| 41 | 0.041 |
| 42 | 0.014 |
| 44 | 0.016 |
| 60 | 0.0089 |
| 66 | 0.029 |
| 68 | 0.063 |
| 74 | 0.028 |
| 75 | 0.062 |
| 76 | 0.069 |
| 77 | 3.0 |
| 78 | 0.082 |

**EXAMPLE OF PHARMACOLOGICAL TEST 2**

Inhibition of the hypotensive effect induced by PAF in normotense rats.

Male Sprage Dawley rats, of 180-220 g of weight, anesthetized with sodium pentobarbital (50 mg/Kg, i.p. 1 mL/100 g) have been used. In order to measure the average arterial pressure, a polyethylene catheter is introduced into the carotid artery. The arterial pressure is recorded with the help of a transducer connected with a R611 Beckman polygraph. The tested compounds are administered through the femoral vein 3 min. before the injection of PAF (0.5 mcg/Kg, i.v.) . The inhibition of the hypotension induced by PAF of the different compounds is expressed as $ID_{50}$, that is to say, the amount of compound by weight of animal (dose) needed to inhibit the hypotension induced by PAF in a 50%. Results are shown in Table II.

TABLE II

| Compound No | $ID_{50}$ (mg/Kg i.v.) |
|---|---|
| 1 | 0.015 |
| 4 | 0.039 |
| 5 | 0.038 |
| 11 | 0.013 |
| 15 | 0.035 |
| 16 | 0.056 |
| 17 | 0.035 |
| 18 | 0.028 |
| 22 | 0.021 |
| 28 | 0.025 |
| 34 | 0.044 |
| 36 | 0.048 |
| 41 | 0.015 |
| 42 | 0.025 |
| 44 | 0.036 |
| 60 | 0.029 |
| 66 | 0.026 |
| 68 | 0.029 |
| 74 | 0.042 |
| 75 | 0.027 |
| 77 | 0.045 |
| 78 | 0.055 |

**EXAMPLE OF PHARMACOLOGICAL TEST 3**

Mortality induced by PAF in mice (p.o.).

This test was performed according to the procedure described by Young et al. (*Prostaglandins*, **1985**, *30*, 545-551). Groups of 10 Swiss mice weighing from 22 to 26 g were used. 100 mcg/kg of PAF-$C_{18}$ and 1 mg/kg of propanolol were administered through a lateral tail vein 1 h after the oral administration of the compounds to be tested (20 mL/kg). Animals were examined 2 h after the injection of PAF. The percentage of the inhibition of the mortality induced by PAF was determined for all the tested compounds in comparison with the control group. The results are expressed as $ID_{50}$ values, that is to say, the amount of compound by weight of animal (dose) needed to inhibit the mortality induced by PAF in a 50%. The results are shown in table III.

TABLE III

| Compound Nº | $ID_{50}$ (mg/Kg p.o.) |
|---|---|
| 4 | 0.69 |
| 11 | 0.44 |
| 15 | 1.2 |
| 17 | 0.09 |
| 22 | 0.30 |
| 28 | 1.6 |
| 34 | 0.62 |
| 41 | 0.044 |
| 42 | 0.23 |
| 60 | 0.41 |
| 66 | 0.13 |
| 68 | 0.3-1 |
| 74 | 0.27 |
| 75 | 0.061 |

The following examples illustrate, but do not limit, the scope of the preparation of the compounds of the present invention.

**PREPARATION 1**

**2-(chloromethyl)pyridine, monohydrochloride**

To a cooled solution (ice bath) of 36 mL (0.372 mol) of 2-(hydroxymethyl)pyridinein 200 mL of anhydrous $CH_2Cl_2$, 27 mL of thionyl chloride were added dropwise. The ice bath was removed and the solution heated at reflux for 3 h. The solvent was evaporated, yielding a crude that was directly used in the next reaction (quantitative yield). IR (film) $\nu$ : 3024, 2945, 2537, 1619, 1606, 1530, 1461, 1421, 1231, 1162, 996, 769 $cm^{-1}$.

**PREPARATION 2**

**1-[(2-pyridyl)methyl]piperazine**

To a cooled solution (ice bath) of 64.08 g (0.744 mol) of anhydrous piperazine in 300 mL of water, 64.16 mL of fumant hydrochloric acid (11.6 N) were added dropwise and the mixture was stirred for 30 min. The ice bath was removed and a solution of the crude obtained in preparation 1 in 100 mL of water was added dropwise. The solution was stirred at room temperature for 48 h. The resulting solution was extracted with chloroform and, after a slight basification was extracted again. Finally, the solution was basified to pH > 10 with sodium hydroxide pellets, the aqueous phase satured with sodium chloride and extracted three times with chloroform. The organic phase of the extractions at pH > 10 was dried over sodium sulfate and the solvent was removed, yielding the title compound of this example as a brown oil (quantitative yield).
IR (film) $\nu$ : 3267, 3002, 2933, 2807, 1585, 1564, 1469, 1427, 1316, 1141, 757 $cm^{-1}$; $^1H$ NMR (80MHz, $CDCl_3$) $\delta$ (TMS): 8.55 (broad d, J = 4.8Hz, 1H, pyr), 7.24 (m, 3H, pyr), 3.64 (s, 2H, $CH_2N$), 2.95 (m, 4H, pip), 2.56 (m, 5H, pip, NH).

**PREPARATION 3**

**1-(*tert*-butoxycarbonyl)-4-[(2-pyridyl)methyl]piperazine**

To a cooled solution (ice bath) of 50 g (0.28 mol) of the product obtained in preparation 2 in 337 mL of water and 337 mL of tetrahydrofuran, 337 mL of 1N sodium hydroxide were added. Keeping the flask in the ice bath, 61.84 g of di-*tert*-butyl dicarbonate were added. The mixture was stirred at room temperature for 45 min, tetrahydrofuran was removed and the crude was extracted with chlorofom. The organic phase was dried over sodium sulfate and the solvent was removed, affording 92.48 g of crude as a brown oil that was purified by crystallization in hexane at 0 ° C, to yield 71.2 g of a white solid (91% yield).

IR (film) ν : 2970, 2805, 1691, 1416, 1361, 1243, 1121, 1004, 757 $cm^{-1}$; $^1$H NMR ($CDCl_3$) δ ppm (TMS): 8.56 (broad d, J = 4.4Hz, 1H, pyr), 7.70 (m, 1H, pyr), 7.40 (m, 1H, pyr), 7.16 (dd, $J_a$ = 7.2Hz, $J_b$ = 4.4Hz, 1H, pyr), 3.66 (s, 2H, $CH_2N$), 3.46 (m, 4H, pip), 2.45 (m, 4H, pip), 1.46 (s, 9H, $CH_3$).

## PREPARATION 4

### 1-(*tert*-butoxycarbonyl)-4-(cyanomethyl)-4-[(2-pyridyl)methyl]piperazinium, bromide

To a cooled (ice bath) solution of 44.5 g (0.16 mol) of the product obtained in preparation 3 in 40 mL of acetonitrile, 13 mL of bromoacetonitrile were added dropwise and the solution stirred at room temperature for 48 h. The solution was concentrated to dryness and the crude thus obtained was purified by chromatography on silica gel (chloroform : methanol 10%), to give 37.8 g of the title compound of this example (74% yield). An analytical sample was obtained by recrystallization in acetone/ ether.
m.p.: 130.7-137.5 ° C;
IR(KBr) ν: 3427, 2974, 2923, 1747, 1685, 1422, 1275, 1146, 877 $cm^{-1}$;
$^1$H NMR (80MHz, $CDCl_3$) δ (TMS): 8.68 (broad d, J = 4.7Hz, 1H, pyr), 8.18 (broad d, J = 7.5Hz, 1H, pyr), 7.85 (d of t, $J_a$ = 7.5Hz, $J_b$ = 1.6Hz, 1H, pyr), 7.46 (dd, $J_a$ = 7.5Hz, $J_b$ = 4.7Hz, 1H, pyr), 5.91 (s, 2H, $CH_2$ pyr), 5.42 (s, 2H, $CH_2CN$), 4.32-3.84 (complex signal, 8H, pip), 1.47 (s, 9H, $CH_3$).
Analysis Calcd. for $C_{17}H_{25}N_4O_2Br$: C 51.39%, H 6.34%, N 14.10%. Found: C 51.40%, H 6.29%, N 14.11%.

## PREPARATION 5

### 1-(*tert*-butoxycarbonyl)-4-[(2-methyl-3-pyridyl)cyanomethyl]piperazine

To a solution of 21 g (0.054 mol) of the product obtained in preparation 4 in 40 mL of acetonitrile, 8.02 mL (0.054 mol) of 1,8-diazabicyclo[5.4.0]undec-7-ene were added and the mixtures was stirred at room temperature for 18 h. The solution was concentrated to dryness, affording a crude that was chromatographed on silica gel (chloroform : methanol 1%), to give 15.26 g of the title compound of this example (94% yield).
m.p.: 186.6-186.7 ° C;
$^1$H NMR (80MHz, $CDCl_3$) δ (TMS): 8.53 (dd, $J_a$ = 4.9Hz, $J_b$ = 1.7Hz, 1H, pyr), 7.82 (dd, $J_a$ = 7.8Hz, $J_b$ = 1.7Hz, 1H, pyr), 7.21 (dd, $J_a$ = 7.8Hz, $J_b$ = 4.9Hz, 1H, pyr), 4.90 (s, 1H, CHCN), 3.43 (m, 4H, pip), 2.55 (m, 7H, pip, $CH_3$ pyr), 1.46 (s, 9H, $CH_3$);
$^{13}$C NMR (20.15 MHz, $CDCl_3$) δ (TMS): 157.96 (C), 154.90 (C), 149.52 (CH), 136.08 (CH), 126.41 (C), 120.93 (CH), 114.18 (C), 79.98 (C), 60.09 (CHCN), 49.29 ($CH_2$), 43.24 ($CH_2$), 28.33 ($CH_3$), 21.96 ($CH_3$ pyr).
Analysis Calcd. for $C_{17}H_{24}N_4O_2$: C 64.53%, H 7.65%, N 17.71%. Found: C 64.23%, H 7.77%, N 17.50%.

## PREPARATION 6

### 1-[(2-methyl-3-pyridyl)cyanomethyl]piperazine

To a cooled solution (0 ° C) of 21.41 g (0.071 mol) of the product obtained in preparation 5 in 150 mL of chloroform, 256 mL of a 3.7 N dioxane/HCl solution were added dropwise. After the addition was completed, the mixture was stirred 1 h at room temperature and then concentrated to dryness, yielding a yellow solid. 139 mL of 1N sodium hydroxide were added and the mixture was extracted three times with chloroform. The organic phase was separated, dried over sodium sulfate and the solvents were removed, yielding 12.1 g of a brown oil (79% yield).
IR (film) ν : 3269, 2944, 2910, 1569, 1438, 1322, 1119, 1101 $cm^{-1}$;
$^1$H NMR (80MHz, $CDCl_3$) δ (TMS): 8.52 (dd, $J_a$ = 4.8Hz, $J_b$ = 1.4Hz, 1H, pyr), 7.83 (dd, $J_a$ = 7.8Hz, $J_b$ = 1.4Hz, 1H, pyr), 7.19 (dd, $J_a$ = 7.8Hz, $J_b$ = 4.8Hz, 1H, pyr), 4.84 (s, 1H, CHCN), 2.88 (m, 4H, pip), 2.64 (s, 3H, $CH_3$), 2.58 (m, 4H, pip), 1.75 (s, 1H, NH).

## PREPARATION 7

### 2-(1-chloroethyl)pyridine, monohydrochloride

Following the procedure described in preparation 1, but using 2-(1-hydroxyethyl)pyridine instead of 2-(hydroxymethyl)pyridine, the title compound of this example was obtained as a crude that can be directly

used in the next reaction (quantitative yield).
IR (film) ν: 3391, 3041, 2561, 2055, 1986, 1608, 1525, 1461, 1240, 782 $cm^{-1}$.

**PREPARATION 8**

**1-[1-(2-pyridyl)ethyl]piperazine**

To a cooled solution (ice bath) of 15.5 g (0.18 mol) of anhydrous piperazine and 31.8 g (0.18 mol) of piperazine dihydrochloride in 150 mL of water, was added the crude obtained in preparation 7 dissolved in 100 mL of water. The mixture was stirred at room temperature for 48 h and the resulting solution was extracted with chloroform. The solution was slightly basified and extracted again. Finally, the solution was basified to pH > 10 with sodium hydroxide pellets, the aqueous phase saturated with sodium chloride and extracted 3 times with chlororform. The organic phase of the extractions at pH > 10 was dried over sodium sulfate and after removal of the solvent, 21.59 g of a brown oil were obtained (63% yield).
IR (film) ν: 3264, 3056, 2934, 2814, 1585, 1564, 1428, 1316, 1134, 750 $cm^{-1}$; $^1H$ NMR (80MHz, $CDCl_3$) δ -(TMS): 8.54 (broad d, J = 4.6Hz, 1H, pyr), 7.60 (m, 1H, pyr), 7.36 (broad d, J = 8Hz, 1H, pyr), 7.14 (dd, $J_a$ = 7.2Hz, $J_b$ = 4.6Hz, 1H, pyr), 3.55 (q, 1H, $\underline{CH}CH_3$), 2.88 (m, 4H, pip), 2.45 (m, 2H, pip), 2.16 (s, 1H, NH), 1.98 (m, 2H, pip), 1.38 (d, 3H, $CH_3$).

**PREPARATION 9**

**1-(*tert*-butoxycarbonyl)-4-[1-(2-pyridyl)ethyl]piperazine**

To a cooled solution (ice bath) of 21.59 g (0.113 mol) of the product obtained in preparation 8, in 130 mL of water and 130 mL of tetrahydrofuran, were added 130 mL of 1N sodium hydroxide. Keeping the flask in the ice bath, 25 g of di-*tert*-butyl dicarbonate were added and the solution was stirred at room temperature for 45 min. After evaporation of tetrahydrofuran, the resulting residue was extracted with chloroform and the organic phase was dried over sodium sulfate. Evaporation of the solvent afforded 37.84 g of a crude that was purified by chromatography on silica gel (hexane : ethyl acetate, 90%), to give 15.3 g of the title compound of this example (46% yield). IR (film) ν: 3408, 2969, 2925, 1685, 1585, 1416, 1360, 1244, 1170 $cm^{-1}$;
$^1H$ NMR (80MHz, $CDCl_3$) δ (TMS): 8.55 (broad d, J = 4.6Hz, 1H, pyr), 7.65 (m, 1H, pyr), 7.36 (broad d, J = 8Hz, 1H, pyr), 7.13 (dd, $J_a$ = 8Hz, $J_b$ = 4.6Hz, 1H, pyr), 3.58 (q, 1H, $\underline{CH}CH_3$), 3.42 (m, 4H, pip), 2.43 (m, 4H, pip), 1.45 (complex signal, 12H, $CH_3$ (BOC), $\underline{CH_3}CH$).

**PREPARATION 10**

**1-(*tert*-butoxycarbonyl)-4-(cyanomethyl)-4-[1-(2-pyridyl)ethyl]piperazinium, bromide**

To a mixture of 15.3 g (0.053 mol) of the product obtained in preparation 9 and 15 mL of acetonitrile, 4 mL (0.055 mol) of bromoacetonitrile were added and the solution was stirred at room temperature for 5 days. The crude obtained was purified by chromatography on silica gel (chloroform : methanol 10%), to give 3.65 g of the title compound of this example (17% yield).
IR (film) ν: 3402, 2973, 2925, 1619, 1586, 1421, 1249, 1148, 753 $cm^{-1}$;
$^1H$ NMR (80MHz, $CDCl_3$) δ (TMS): 8.68 (broad d, J = 4.9Hz, 1H, pyr), 8.17 (broad d, J = 7.5Hz, 1H, pyr), 7.86 (d of t, $J_a$ = 7.5Hz, $J_b$ = 1.5Hz, 1H, pyr), 7.41 (dd, $J_a$ = 7.5Hz, $J_b$ = 4.9Hz, 1H, pyr), 6.00 (m, 3H, $\underline{CH}CH_3$, $CH_2CN$), 3.38-4.36 (complex signal, 8H, pip), 1.96 (d, 3H, $CH\underline{CH_3}$), 1.45 (s, 9H, $CH_3$ (BOC)).

**PREPARATION 11**

**1-(*tert*-butoxycarbonyl)-4-[(2-ethyl-3-pyridyl)cyanomethyl]piperazine**

To a cooled solution (ice bath) of 3.65 g (9 mmol) of the product obtained in preparation 10 in 30 mL of acetonitrile, 1.3 mL (9 mmol) of 1,8-diazabicyclo[5.4.0]undec-7-ene were added. The ice bath was removed and the solution stirred at room temperature for 18 h, under argon atmosphere. The solution was concentrated to dryness, affording a crude that was chromatographed on silica gel eluting with hexane-ethyl acetate mixtures of increasing polarity. 1.4 g of the desired product were obtained (47% yield).
IR (film) ν: 2970, 2928, 1690, 1566, 1416, 1363, 1245, 1169, 1001 $cm^{-1}$;

$^1$H NMR (80MHz, $CDCl_3$) δ (TMS): 8.57 (dd, $J_a$ = 4.6Hz, $J_b$ = 1.7Hz, 1H, pyr), 7.82 (dd, $J_a$ = 8.1Hz, $J_b$ = 1.7Hz, 1H, pyr), 7.27 (dd, $J_a$ = 8.1Hz, $J_b$ = 4.6Hz, 1H, pyr), 4.97 (s, 1H, CHCN), 3.46 (m, 4H, pip), 2.88 (q, 2H, $\underline{CH_2}CH_3$), 2.53 (m, 4H, pip), 1.17-1.46 (complex signal, 12H, $CH_3$, $CH_2\underline{CH_3}$).

**PREPARATION 12**

**1-[(2-ethyl-3-pyridyl)cyanomethyl]piperazine**

Following the procedure described in preparation 6, but using the product obtained in preparation 11 instead of the compound obtained in preparation 5, the title compound of this example was obtained (93% yield).
$^1$H NMR (80MHz, $CDCl_3$) δ (TMS): 8.58 (dd, $J_a$ = 4.8Hz, $J_b$ = 1.4Hz, 1H, pyr), 7.85 (dd, $J_a$ = 7.8Hz, $J_b$ = 1.4Hz, 1H, pyr), 7.20 (dd, $J_a$ = 7.8Hz, $J_b$ = 4.8Hz, 1H, pyr), 4.91 (s, 1H, CHCN), 2.89 (m, 6H, 4H pip, $\underline{CH_2}CH_3$), 2.53 (m, 5H, 4H pip, NH), 1.34 (t, J = 7.2Hz, 3H, $CH_2\underline{CH_3}$).

**PREPARATION 13**

**1-(3,3-diphenylpropionyl)-4-[(2-pyridyl)methyl]piperazine**

To a mixture of 8.67 g (0.049 mol) of the product obtained in preparation 2, 11.1 g (0.049 mol) of 3,3-diphenylpropionic acid and 8.3 g (0.052 mol) of 1-hydroxybenzotriazole solved in 250 mL of anhydrous dimethylformamide, 10.11 g (0.049 mol) of dicyclohexylcarbodiimide were added at 0 °C and under nitrogen atmosphere, and the resulting solution was stirred at room temperature for 18 h. The solvents were removed under vacuum, the residue was stirred with ethyl acetate and the white solid formed was filtered. The organic solution was washed with a saturated solution of sodium bicarbonate, with water and finally with a saturated solution of sodium chloride, dried over sodium sulfate and evaporated, yielding 18.9 g of a crude that was purified by chromatography on silica gel eluting with ethyl acetate, to give 8.19 g of the title compound of this example (43% yield).
IR (film) ν: 3053, 3018, 2929, 2803, 1622, 1463, 1447, 1427, 1224, 1004, 751 $cm^{-1}$;
$^1$H NMR (80MHz, $CDCl_3$) δ (TMS): 8.50 (broad d, J = 5Hz, 1H, pyr), 7.65 (m, 1H, pyr), 7.21 (m, 12H, pry + Ph), 4.60 (t, J = 7.6Hz, 1H, $Ph_2CH$), 3.59 (s, 2H, $CH_2N$), 3.34 (m, 4H, pip), 3.02 (d, J = 7.6Hz, 2H, $CH_2CO$), 2.29 (m, 4H, pip).

**PREPARATION 14**

**1-(3,3-diphenylpropionyl)-4-(ethoxycarbonylmethyl)-4-[(2-pyridyl)methyl]piperazinium, bromide**

Following a procedure analogous to the one described in preparation 4, but starting from the product obtained in preparation 13 and using ethyl bromoacetate instead of bromoacetonitrile, the title compound of this example was obtained (27% yield).
IR (film) ν: 3423, 2994, 1738, 1637, 1433, 1214, 1023, 952, 705 $cm^{-1}$;
$^1$H NMR (80MHz, $CDCl_3$) δ (TMS): 8.61 (broad d, J = 4.2Hz, 1H, pyr), 7.83 (m, 2H, pyr), 7.31 (m, 11H, pyr + Ph), 5.29 (s, 2H, $CH_2$ pyr), 4.79 (s, 2H, $CH_2COOEt$), 4.66 (t, J = 7.4Hz, 1H, $Ph_2\underline{CH}CH_2$), 4.14 (q, J = 7.2Hz, 2H, $O\underline{CH_2}CH_3$), 3.97 (broad s, 8H, pip), 3.11 (d, J = 7.4Hz, 2H, $Ph_2CH\underline{CH_2}$), 1.28 (t, J = 7.2Hz, 3H, $OCH_2\underline{CH_3}$).

**PREPARATION 15**

**1-(3,3-diphenylpropionyl)-4-[2-(methoxyimino)propyl]-4-[(2-pyridyl)methyl]piperazinium, iodide**

To a cooled mixture (ice bath) of 1.675 g of the product obtained in preparation 13, solved in acetonitrile, and 0.44 g of potassium iodide, 0.316 g of chloroacetone O-methyloxime were added dropwise. The mixture was stirred for 48 h and then concentrated to dryness. The resulting crude was purified by chromatography on silica gel (chloroform : methanol 5%), to give 0.33 g of the desired product (21% yield).
IR (film) ν: 3483, 3019, 2931, 1642, 1585, 1433, 1246, 1045, 1032, 753, 736, 702 $cm^{-1}$;
$^1$H NMR (80MHz, $CDCl_3$) δ (TMS): 8.63 (broad d, J = 3.9 Hz, 1H, pyr), 8.08 (broad d, J = 8Hz, 1H, pyr), 7.82 (d of t, $J_a$ = 8Hz, $J_b$ = 1.7Hz, 1H, pyr), 7.23 (m, 11H, pyr + Ph), 5.20 (s, 2H, $CH_2$ pry), 4.57 (m, 3H, $Ph_2CH$, $\underline{CH_2}C=N$), 3.41-3.95 (complex signal, 11H, $OCH_3$, pip), 3.11 (d, J = 7.5Hz, 2H, $Ph_2CH\underline{CH_2}$), 2.13 (s,

3H, $CH_3$).

**PREPARATION 16**

**1-(*tert*-butoxycarbonyl)-4-(2-propyn-1-yl)-4-[(2-pyridyl)methyl]piperazinium, phenylsulfonate**

To a cooled solution (ice bath) of 8 g (0.029 mol) of the product obtained in preparation 3 in 10 mL of acetonitrile, 4.55 mL of propargyl phenylsulfonate were added and the solution was stirred at room temperature for 72 h. After concentration to dryness, the resulting residue was purified by chromatography on silica gel eluting with chloroform-methanol mixtures of increasing polarity, yielding 5.9 g of the title compound of this example as a dark oil (43% yield).
IR (film) $\nu$: 3443, 3197, 2973, 2122, 1691, 1585, 1416, 1213, 1192, 1016 $cm^{-1}$;
$^1H$ NMR (80MHz, $CDCl_3$) $\delta$ (TMS): 8.61 (broad d, J = 5Hz, 1H, pyr), 7.90 (m, 4H), 7.30 (m, 4H), 5.12 (s, 2H, $CH_2$ pyr), 4.76 (d, J = 2.1Hz, 2H, $\underline{CH_2}C{\equiv}CH$), 3.89 (s, 8H, pip), 2.88 (t, J = 2Hz, 1H, $C{\equiv}CH$), 1.46 (s, 9H, $CH_3$).

**PREPARATION 17**

**1-(*tert*-butoxycarbonyl)-4-[1-(2-methyl-3-pyridyl)-2-propyn-1-yl]piperazine**

To a cooled solution (-10 °C) of 3 g (6.8 mmol) of the product obtained in preparation 16 in 15 mL of dry tetrahydrofuran and 3 mL of anhydrous dimethylsulfoxide, were added 1.25 g of potassium *tert*-butoxide and the mixture was stirred at room temperature for 18 h. After addition of water, extraction with chloroform and evaporation of the solvents, were obtained 1.3 g of a crude that was purified by chromatography on silica gel eluting with ethyl acetate, to give 250 mg of the title compound of this example as a dark oil (12% yield).
$^1H$ NMR (80MHz, $CDCl_3$) $\delta$ (TMS): 8.42 (dd, $J_a$ = 4.8Hz, $J_b$ = 1.5Hz, 1H, pyr), 7.89 (dd, $J_a$ = 7Hz, $J_b$ = 1.5Hz, 1H, pyr), 7.16 (dd, $J_a$ = 7Hz, $J_b$ = 4.8Hz, 1H, pyr),4.71 (d, J = 2Hz, 1H, CHN), 3.40 (m, 4H, pip), 2.66 (s, 3H, $CH_3$ pyr), 2.65 (d, J = 2Hz, 1H, $C{\equiv}CH$), 2.47 (m, 4H, pip), 1.46 (s, 9H, $CH_3$ (BOC)).

**PREPARATION 18**

**1-[1-(2-methyl-3-pyridyl)-2-propyn-1-yl]piperazine**

Following the procedure described in preparation 6, but starting from the compound obtained in preparation 17 instead of the compound obtained in preparation 5, the title compound of this example was obtained, which was purified by cromatography on silica gel (chloroform : methanol 10%), to give 40 mg of the desired product (22% yield).
$^1H$ NMR (80MHz, $CDCl_3$) $\delta$ (TMS): 8.44 (dd, $J_a$ = 4.8Hz, $J_b$ = 1.9Hz, 1H, pyr), 7.90 (dd, $J_a$ = 8.1Hz, $J_b$ = 1.9Hz, 1H, pyr), 7.11 (dd, $J_a$ = 8.1Hz, $J_b$ = 4.8Hz, 1H, pyr), 4.66 (d, J = 2.2Hz, 1H, CHN), 2.87 (m, 4H, pip), 2.64 (s, 3H, $CH_3$ pyr), 2.58 (m, 5H, pip, $C{\equiv}CH$), 2.24 (s, 1H, NH).

**PREPARATION 19**

**3-(N-(*tert*-butoxycarbonyl)amino)-3-phenylpropionic acid**

Following the procedure described in preparation 3, but using the 3-amino-3-phenylpropionic acid instead of the product obtained in preparation 2, the title compound of this example was obtained (80% yield). m.p.: 141-142 °C

**PREPARATION 20**

**3,3-Diphenyl-3-ethoxycarbonylpropionic acid**

**a) Ethyl diphenylacetate**

To a solution of 20 g (0.094 mol) of diphenylacetic acid in 70 mL ethanol, were added 70 mL of toluene. Then, 3 mL of sulfuric acid were added dropwise and the mixture was heated to reflux for 18 h. To the

resulting solution was added water and ethyl acetate, and the organic phase was separated, washed three times with a saturated solution of sodium bicarbonate and dried over sodium sulfate. Removal of the solvents afforded 23.2 g of a white solid that was directly used in the next step.
IR(KBr) ν: 3056, 2975, 1728, 1491, 1448, 1364, 1189, 1148, 1025 $cm^{-1}$;
$^1H$ NMR (80MHz, $CDCl_3$) δ (TMS): 7.28 (m, 10H, Ph), 5.00 (s, 1H, $Ph_2CH$), 4.19 (q, J = 7Hz, 2H, $O\underline{CH_2}CH_3$), 1.22 (t, J = 7Hz, 3H, $OCH_2\underline{CH_3}$).

**b) *tert*-butyl 3,3-diphenyl-3-ethoxycarbonylpropionate**

To a solution of 4.25 g of sodium hydride (60% in parafine) in 100 mL of dimethylformamide, were added 23.2 g (0.965 mol) of the product obtained in preparation 20a, solved in 50 mL of dimethylformamide. Then, the mixture was stirred at room temperature for 1 h. 13.5 mL (0.965 mol) of *tert*-butyl bromoacetate were added dropwise and the mixture was stirred at 60 ° C for 18 h. To the resulting solution was added 1 mL of water and the solvents were removed. More water was added and the solution was extracted with ethyl acetate, at basic pH. The organic phase was dried over sodium sulfate and the solvent was removed, yielding the title compound of this example as a dark oil.
IR(KBr) ν: 3053, 2973, 2925, 1729, 1442, 1364, 1224, 1145, 1028 $cm^{-1}$;
$^1H$ NMR (80MHz, $CDCl_3$) δ (TMS): 7.26 (s, 10H, Ph), 4.21 (q, J = 7.2Hz, 2H, $O\underline{CH_2}CH_3$), 3.43 (s, 2H, $CH_2$), 1.30 (s, 9H, $CH_3$), 1.16 (t, J = 7.2Hz, 3H, $OCH_2\underline{CH_3}$).

**c) 3,3-Diphenyl-3-ethoxycarbonylpropionic acid**

To a cooled solution (0 ° C) of 6 g (0.017 mol) of the product obtained in preparation 20b in 20 mL of dichloromethane, were added dropwise 2.6 mL of trifluoroacetic acid and the mixture was stirred at room temperature for 18 h. Concentration to dryness afforded the title compound of this example (85% yield).
IR(KBr) ν: 3600-2400, 1728, 1491, 1442, 1366, 1162, 1026, 698 $cm^{-1}$;
$^1H$ NMR (80MHz, $CDCl_3$) δ (TMS): 11.26 (broad s, 1H, COOH), 7.25 (s, 10H, Ph), 4.21 (q, J = 7.1Hz, 2H, $O\underline{CH_2}CH_3$), 3.54 (s, 2H, $CH_2CO$), 1.17 (t, J = 7.1Hz, 3H, $OCH_2\underline{CH_3}$).

## PREPARATION 21

### 3-Phenyl-3-hydroxy-3-(trifluoromethyl)propionic acid

To a cooled solution (ice bath) of 40 mL of butyl lithium 1.6 M in hexane in 90 mL of dry tetrahydrofuran, were added dropwise 9.45 mL of diisopropylamine and the solution was stirred for 5 min. Keeping the solution at 0 ° C, 1.92 mL (0.0336 mol) of acetic acid were added dropwise and the mixture was stirred for 10 min. Then, the mixture was heated at 50 ° C for 30 min and finally it was allowed to cool. A solution of 4.76 mL (0.0336 mol) of 2,2,2-trifluoroacetophenone in 15 mL of dry tetrahydrofuran was added at 0 ° C and the mixture was stirred at room temperature overnight. Finally, 150 mL of diethyl ether and 50 mL of water were added, the aqueous phase was separated, acidified with HCl and extracted three times with ethyl acetate. After removal of the solvents, 3.88 g of the title compound of this example were obtained as an orange solid (49% yield).
IR(KBr) ν: 3700-2300, 1702, 1447, 1420, 1265, 1163, 1074, 700 $cm^{-1}$; $^1H$ NMR (80MHz, $CDCl_3$) δ (TMS): 9.0 (complex signal, 2H, COOH, OH), 7.38 (m, 5H, Ph), 3.2 (s, 2H, $CH_2CO$).

## PREPARATION 22

### N-(1,1-Diphenylethyl)aminoacetic acid

**a) 2,2-Diphenylpropionyl chloride**

A mixture of 20 g of 2,2-diphenylpropionic acid and 40 mL of thionyl chloride was heated at reflux for 5 h. 60 mL of toluene were added and the mixture was distilled in vacuo (water pump), yielding a crude that was directly used in the next step.
IR(KBr) ν: 3054, 2994, 1768, 1490, 1440, 1374, 1027, 931, 904, 731 $cm^{-1}$.

**b) N-(1,1-Diphenylethyl)amine**

To a cooled solution (ice bath) of 6.3 g (0.097 mol) of sodium azide in 30 mL of water, was added dropwise a solution of the product obtained in preparation 22a in 30 mL of acetone, and the mixture was stirred at 10 ° C for 1 h. The two layers were separated and the organic phase was poured into 100 mL of toluene at 60 ° C. The heating was mantained during 1.5 h at 60-70 ° C until complete solubilization. 50 mL of conc. hydrochloric acid were added and the mixture heated at reflux for 2.5 h. The solid formed was filtered, washed with ether and dried, yielding 16.7 g of the title compound of this example, as a hydrochloride (81% yield from 2,2-diphenylpropionic acid).
IR(KBr) $\nu$: 3100-2700, 1605, 1510, 1444, 1253, 771, 697 $cm^{-1}$;
$^1H$ NMR (80MHz, $CDCl_3$) $\delta$ (TMS): 9.51 (broad s, 3H, $^+NH_3$), 7.33 (s, 10H, Ph), 2.08 (s, 3H, $CH_3$).

**c) *tert*-butyl N-(1,1-diphenylethyl)aminoacetate**

To a solution of 2.7 g (10 mmol) of the product obtained in preparation 22b and 1.6 g of potassium carbonate in dimethylformamide, were added 1.6 mL of *tert*-butyl bromoacetate and the mixture was stirred at 80 ° C for 12 h. The solvent was removed and the residue was worked up with water and chloroform. The organic phase was dried over sodium sulfate and the solvent was evaporated, yielding 3.4 g of the title compound of this example (95% yield).
IR(KBr) $\nu$: 3321, 3052, 2972, 1725, 1674, 1365, 1154, 701 $cm^{-1}$;
$^1H$ NMR (80MHz, $CDCl_3$) $\delta$ (TMS): 7.29 (s, 10H, Ph), 3.13 (s, 2H, $CH_2CO$), 2.30 (broad s, 1H, NH), 1.77 (s, 3H, $CH_3CPh_2$), 1.41 (s, 9H, $CH_3$).

**d) N-(1,1-diphenylethyl)aminoacetic acid**

To a solution of 1.5 g (4.8 mmol) of the product obtained in preparation 22c in 100 mL of methanol, was added a solution of 3.33 g of potassium carbonate in 50 mL of water and the mixture was stirred at room temperature for 1 h and at reflux for 2 h. Methanol was evaporated in vacuo and the resulting solution was neutralized with hydrochloric acid to pH = 6. The solution was extracted with chloroform and the organic sphase was dried over sodium sulfate. Evaporation of the solvent afforded 0.98 g of a crude that was purified by chromatography on silica gel eluting with chloroform-methanol mixtures of increasing polarity, to yield 0.43 g of the title compound of this example (35% yield).
IR(KBr) $\nu$: 3600-2300, 1723, 1670, 1365, 1153, 757, 700 $cm^{-1}$;
$^1H$ NMR (80MHz, $CDCl_3$) $\delta$ (TMS): 7.28 (s, 10H, Ph), 6.75 (s, 2H), 3.37 (s, 2H, $CH_2CO$), 1.98 (s, 3H, $CH_3$).

## PREPARATION 23

## N-(diphenylmethyl)-N-(methylsulfonyl)aminoacetic acid

**a) N-(diphenylmethyl)methanesulfonamide**

To a solution of 4 g (0.02 mol) of (diphenylmethyl)amine in 50 mL of anhydrous dichloromethane, 10 mL (0.072 mol) of triethylamine were added. The mixture was cooled with an ice bath and 1.8 mL (0.024 mol) of methanesulfonyl chloride were added under nitrogen atmosphere and the mixture was stirred at room temperature for 18 h. The resulting solution was diluted with dichloromethane, washed with water and 0.1 N hydrochloric acid and the organic phase was dried over sodium sulfate. Evaporation of the solvent afforded 5.22 g of a crude that was purified by chromatography on silica gel (hexane : ethyl acetate, 30%), to give 3.36 g of the product (64% yield).
IR(KBr) $\nu$: 3290, 3054, 3018, 2920, 1595, 1487, 1446, 1317, 1147, 742, 698 $cm^{-1}$;
$^1H$ NMR (80MHz, $CDCl_3$) $\delta$ (TMS): 7.32 (s, 10H, Ph), 5.73 (broad s, 1H, NH), 5.19 (s, 1H, CH), 2.63 (s, 3H, $CH_3$).

**b) Ethyl N-(diphenylmethyl)-N-(methylsulfonyl)aminoacetate**

To a suspension of 0.7 g (0.014 mol) of sodium hydride (60% in parafine) in 10 mL of dimethylformamide, was added a solution of 3.36 g (0.018 mol) of the product obtained in preparation 23a in 5 mL of dimethylformamide. After the addition was completed, 1.5 mL (0.014 mol) of ethyl bromoacetate were added and the mixture was stirred at 60 ° C for 18 h under argon atmosphere. Dimethylformamide was

removed and the resulting residue was worked up with phosphates buffer and ethyl acetate, and extracted two more times with ethyl acetate. The organic phase was dried over sodium sulfate and the solvent was evaporated, affording 4.01 g of a crude that was purified by chromatography on silica gel (hexane : ethyl acetate, 20%), to give 1.47 g of the product (30% yield).
IR(KBr) ν: 3051, 3025, 2974, 1734, 1488, 1443, 1314, 1260, 1161, 1144, 1066, 1028 $cm^{-1}$.
$^1H$ NMR (80MHz, $CDCl_3$) δ (TMS): 7.31 (s, 10H, Ph), 6.32 (s, 1H, CH), 4.09 (s, 2H, $CH_2N$), 3.89 (q, J= 7.2Hz, 2H, $OCH_2CH_3$), 3.15 (s, 3H, $SO_2CH_3$), 1.09 (t, J= 7.2Hz, 3H, $OCH_2CH_3$).

**c) N-(diphenylmethyl)-N-(methylsulfonyl)aminoacetic acid**

To a solution of 1.47 g of the product obtained in preparation 23b in 5 mL of tetrahydrofuran, were added 7 mL of 5% sodium hydroxide and the mixture was stirred at 70°C for 1 h. 10 mL of water were added and the resulting solution was extracted with diethyl ether. The aqueous phase was acidified to acid pH and extracted with chloroform. The organic phase was dried over sodium sulfate and the solvents were removed, affording 0.96 g of a crude that was purified by chromatography on silica gel (chloroform : methanol, 5%), to give 0.98 g of the title compound of this example (73% yield).
m.p.: 153-154°C;
IR(KBr) ν: 3500-2400, 1723, 1313, 1231, 1165, 1145, 1063, 941 $cm^{-1}$;
$^1H$ NMR (80MHz, $CDCl_3$) δ (TMS): 8.96 (complex signal, 1H, COOH), 7.31 (s, 10H, Ph), 6.32 (s, 1H, CH), 4.11 (s, 2H, $CH_2N$), 3.05 (s, 3H, $SO_2CH_3$).

## PREPARATION 24

## (Diphenylmethylen)aminooxyacetic acid

**a) Benzophenone oxime**

To a mixture of 15 g (0.083 mol) of benzophenone, 28.9 g (0.42 mol) of hydroxylamine hydrochloride and 49.32 g (0.25 mol) of barium carbonate, 250 mL of abs. ethanol were added and the mixture was stirred at reflux for 18 h. The resulting solution was filtered, washed with hot ethanol and the solvent was removed. The residue was solved in diethyl ether and washed with water. The organic phase was dried over sodium sulfate and the solvent was evaporated, yielding 17.65 g of the title compound of this example (quantitative yield).
IR(KBr) ν: 3500-2700, 1439, 1326, 1158, 996, 918, 766, 696 $cm^{-1}$;
$^1H$ NMR (80MHz, $CDCl_3$) δ (TMS): 7.39 (m, 10H, Ph), 5.9 (broad s, 1H, NOH).

**b) Ethyl (diphenylmethylen)aminooxyacetate**

To a solution of 2.38 g of sodium ethoxide in 25 mL of absolute ethanol, were added 7 g (0.035 mol) of the product obtained in preparation 24a solved in 20 mL of ethanol. The mixture was stirred at room temperature for 10 min and at 80°C for 15 min until the precipitated formed was dissolved. Then, the mixture was allowed to cool and a solution of 3.9 mL (0.035 mol) of ethyl bromoacetate in 5 mL of absolute ethanol were added dropwise. The resulting solution was heated at reflux for 18 h. After concentration to dryness, 0.1 N sodium hydroxide was added and the solution was extracted with chloroform. The organic phase was dried over sodium sulfate and the solvent was removed, affording 8.9 g of a crude that was purified by chromatography on silica gel (hexane : ethyl acetate, 5%), to give 1.87 g of the title compound of this example (20% yield).
IR(KBr) ν: 3053, 2975, 2925, 1752, 1743, 1440, 1203, 1098, 1025, 696 $cm^{-1}$;
$^1H$ NMR (80MHz, $CDCl_3$) δ (TMS): 7.42 (m, 10H, Ph), 4.69 (s, 2H, $CH_2CO$), 4.21 (q, J = 7.1Hz, 2H, $OCH_2CH_3$), 1.26 (t, J = 7.1Hz, 3H, $OCH_2CH_3$).

**c) (Diphenylmethylen)aminooxyacetic acid**

To a solution of 1.87 g (6.6 mmol) of the product obtained in preparation 24b in 5 mL of tetrahydrofuran, were added 7 mL of 5% sodium hydroxide and the mixture was heated at 70°C for 1 h. The solvent was removed and the residue extracted with diethyl ether. The aqueous phase was acidified with hydrochloric acid and extracted with chloroform. The organic phase was dried over sodium sulfate and the solvent was removed, yielding 1.1 g of the title compound of this example (65% yield).

IR(KBr) ν: 3400-2400, 1718, 1702, 1440, 1242, 1100, 1023, 694 $cm^{-1}$;
$^1H$ NMR (80MHz, $CDCl_3$) δ (TMS): 9.48 (broad s., 1H, COOH), 7.43 (m, 10H, Ph), 4.75 (s, 2H, $CH_2CO$).

## PREPARATION 25

## Diphenylmethoxyacetic acid

### a) Diphenylmethanol

To a cooled solution (0 °C) of 10 g (0.05 mol) of benzophenone in 40 mL of methanol, were added 3 g of sodium borohydride and the mixture was stirred at room temperature for 2 days. 10 mL of water were added, methanol was removed in vacuo and the residue was worked up with water and chloroform. The organic phase was dried over sodium sulfate and the solvent was removed, yielding 9.71 g of the title compound of this example (quantitative yield).
IR(KBr) ν: 3277, 3051, 3018, 1591, 1487, 1442, 1271, 1182, 1031, 1016, 739, 700 $cm^{-1}$;
$^1H$ NMR (80MHz, $CDCl_3$) δ (TMS): 7.29 (m, 10H, Ph), 5.76 (d, J= 3.7Hz, 1H, CH), 2.35 (d, J= 3.7Hz, 1H, OH).

### b) Diphenylmethoxyacetic acid

To 2 g (0.01 mol) of the product obtained in preparation 25a, were added 0.76 g (0.01 mol) of glycolic acid and 10 mL of trifluoroacetic acid and the mixture was stirred at room temperature for 18 h. The solvents were evaporated and chloroform was added. The product obtained was then washed with 2N sodium hydroxide solution and the aqueous layer was separated, acidified with 5N hydrochloric acid and extracted twice with ethyl acetate. The organic phase was dried over sodium sulfate and the solvent was removed, yielding 0.8 g of the title compound of this example (33% yield).
IR(KBr) ν: 3600-2400, 1721, 1445, 1244, 1183, 1118, 694 $cm^{-1}$;
$^1H$ NMR (80MHz, $CDCl_3$) δ (TMS): 8.87 (broad s, 1H, COOH), 7.25 (m, 10H, Ph), 5.54 (s, 1H, CH), 4.11 (s, 2H, $CH_2$).

## PREPARATION 26

## (Diphenylmethyl)thioacetic acid

A mixture of 2 g (0.01 mol) of the product obtained in preparation 25a and 0.7 mL (0.01 mol) of thioacetic acid in 10 mL of trifluoroacetic acid was stirred at room temperature for 30 min and insoluble material was removed by filtration. The filtrate was evaporated, affording 1.8 g of the title compound of this example as a white solid (66% yield).
m.p.: 123-124 °C;
IR(KBr) ν: 3600-2400, 1692, 1446, 1301, 1138, 746, 700 $cm^{-1}$;
$^1H$ NMR (80MHz, $CDCl_3$) δ (TMS): 9.47 (s, 1H, COOH), 7.35 (m, 10H, Ph), 5.41 (s, 1H, CH), 3.09 (s, 2H, $CH_2$).

## PREPARATION 27

## (Diphenylmethyl)sulfinylacetic acid

To a solution of 1 g (0.00387 mol) of the product obtained in preparation 26 in methanol, were added 0.89 mL of a solution prepared mixing 3 g of 2-propanol and 0.15 mL of 96% $H_2SO_4$. Then, 0.82 mL of 33% $H_2O_2$ were added. After stirring for 1 h, the solution was saturated with sodium chloride and extracted with chloroform. The organic phase was dried over sodium sulfate and evaporated, yielding 1.53 g of a crude that was purified by chromatography on silica gel (chloroform : methanol, 5%), to give 0.71 g of the title compound of this example (63% yield).
IR(KBr) ν: 3400-2300, 1718, 1447, 1270, 1123, 1027, 754, 704 $cm^{-1}$;
$^1H$ NMR (80MHz, $CDCl_3$) δ (TMS): 7.42 (m, 10H, Ph), 5.27 (s, 1H, CH), 4.03 (s, 1H, OH), 3.47 (s, 2H, $CH_2CO$).

**PREPARATION 28**

**(Diphenylmethyl)sulfonylacetic acid**

To a solution of 1 g (3.87 mmol) of the product obtained in preparation 26 in 6 mL of glacial acetic acid, were added 1.6 mL of 33% $H_2O_2$. The mixture was stirred for 18 h and extracted with chloroform. The organic phase was dried over sodium sulfate and evaporated, yielding 1.43 g of a crude that was purified by chromatography on silica gel (chloroform : methanol, 5%), to give the title compound of this example (37% yield).

IR(KBr) $\nu$: 3500-2700, 1726, 1448, 1308, 1219, 1136, 701 $cm^{-1}$;

$^1$H NMR (80MHz, $CDCl_3$) $\delta$ (TMS): 7.70 (m, 4H, Ph), 7.40 (m, 6H, Ph), 6.02 (s, 1H, CH), 3.95 (s, 1H, OH), 3.82 (s, 2H, $CH_2CO$).

**PREPARATION 29**

**a) (R)-2-(N-(Diphenylmethyl)amino)propionic acid**

A solution of 4.45 g (50 mmol) of D-alanine in 10 mL of water was added to a solution of 9.1 g (50 mmol) of benzophenone in 100 mL of methanol. To this mixture heated at 90 °C, 4.7 g (75 mmol) of $NaBH_3CN$ were added in batches. The resulting mixture was heated at reflux for 18 h, the mixture was concentrated, and water (100 mL) and 10% NaOH solution (20 mL) were added. The resulting solution was whased with ether and adjusted to pH 5 with 10% HCl solution. The precipitated solid was collected and dried (2.6 g, 20%).

m.p.: 204-206 °C;

IR(KBr) n 3500-2500, 1614, 1583, 1565, 1387, 1360 $cm^{-1}$;

$^1$H NMR (80MHz, $CDCl_3$) $\delta$ (TMS): 7.39 (broad s, 10 H), 5.21 (s, 1H), 3.80 (s, NH, OH), 3.38 (q, J = 7.3 Hz, 1H), 1.40 (d, J = 7.2Hz, 3H).

The absence of racemization was tested by preparation of the corresponding $\alpha$-methylbenzylamide with (D)-(+)-$\alpha$-methylbenzylamine: $^1$H NMR (80MHz, $CDCl_3$) $\delta$ (TMS): 7.17 (broad s, 15 H), 5.05 (qint, J = 1.8 Hz, 1H), 4.63 (s, 1H), 3.08 (q, J = 7.1 Hz, 1H), 1.92 (s, 3H), 1.38 (d, J = 7.1 Hz, 3H), 1.22 (d, J = 7.1 Hz, 3H).

**b) (S)-2-(N-(Diphenylmethyl)amino)propionic acid**

The procedure was repeated with L-alanine to give (S)-2-(diphenylmethylamino)propionic acid as a white solid.

m.p.: 198-199 °C.

**PREPARATION 30**

**N-Formyl-N-(diphenylmethyl)glycine.**

A mixture of 5 g (27 mmol) of (diphenylmethyl)amine and 5.4 g (59 mmol) of glyoxylic acid monohydrate in 25 mL of formic acid was heated at 70ºC for 90 min. After removal of the solvent, the residue was partitioned between water and chloroform, and the aqueous phase was reextracted twice with chloroform. The organic phases were dried and evaporated to give an orange solid (7.2 g), which was purified by flash chromatography ($CHCl_3$: MeOH, 3%) to afford a white solid (5.3 g, 74%). Crystallization of a portion from ethyl acetate/hexane furnished an analytical sample.

m.p.: 117-118 °C;

IR(KBr) n 3200-3500, 1730, 1658, 1631, 1388 $cm^{-1}$;

$^1$H NMR (80MHz, $CDCl_3$) $\delta$ (TMS): 8.13 (s, 1H, CHO), 7.30 (m, 10H), 6.58 (m, OH), 5.90 (s, 1H, CHN), 4.12 (s, 2H, $CH_2CO$).

**PREPARATION 31**

***Cis* and *trans* 3-phenyl-3-(trifluoromethyl)propenoic acid**

**a) Ethyl *cis* and *trans*-3-phenyl-3-(trifluoromethyl)propenoate**

To a cooled (0 °C) suspension of 1.5 g of NaH in 50 mL of DME, were added 7.1 mL of triethylfosfonoacetate and the resulting mixture was stirred for 0.5 h at room temperature. Then, were added dropwise 5 mL (35.6 mmol) of 2,2,2-trifluoroacetophenone and the resulting mixture was refluxed for 1 h. After removal of the solvent, the crude was partitioned between water and ether. The organic solution was dried over sodium sulfate and evaporated, affording 8.9 g of a crude that was purified by chromatography on silica gel (hexane : ethyl acetate, 2%), to give 3.54 g of ethyl *cis*-3-phenyl-3-trifluoromethylpropenoate (41% yield) and 4.0 g of ethyl *trans*-3-phenyl-3-trifluoromethylpropenoate (46% yield).

**Isomer cis:**

$^1H$ NMR (80MHz, $CDCl_3$) δ (TMS): 7.33 (s, 5H, Ph), 6.59 (s, 1H, CH), 4.00 (q, J = 7.1 Hz, 2H, $\underline{CH_2}CH_3$), 1.00 (t, J = 7.2 Hz, 3H, $CH_2\underline{CH_3}$).

**Isomer trans:**

$^1H$ NMR (80MHz, $CDCl_3$) δ (TMS): 7.40 (s, 5H, Ph), 6.32 (s, 1H, CH), 4.30 (q, J = 7.1 Hz, 2H, $\underline{CH_2}CH_3$), 1.34 (t, J = 7.2 Hz, 3H, $CH_2\underline{CH_3}$).

**b) *trans*-3-phenyl-3-(trifluoromethyl)propenoic acid**

Following the procedure described in preparation 22c, but starting from the product obtained in preparation 31a (isomer trans), the title compound of this example was obtained (92% yield).
$^1H$ NMR (80MHz, $CDCl_3$) δ (TMS): 10.26 (m, 1H, OH), 7.41 (m, 5H, Ph), 6.35 (s, 1H, CH).

**c) *cis*-3-phenyl-3-(trifluoromethyl)propenoic acid**

Following the procedure described in preparation 22c, but starting from the product obtained in preparation 31a (isomer cis), the title compound of this example was obtained (80% yield).
$^1H$ NMR (80MHz, $CDCl_3$) δ (TMS): 8.33 (m, 1H, OH), 7.35 (m, 5H, Ph), 6.57 (s, 1H, CH).

**PREPARATION 32**

**3-phenyl-3-(trifluoromethyl)propionic acid**

A mixture of 0.7 g (3.5 mmol) of the compound obtained in preparation 31a (isomer trans) and 0.23 g of Pd/C 5% in 40 mL of methanol was hydrogenated at atmospheric pressure for 18 h. The insoluble material was removed by filtration and the filtrate evaporated, to give 0.7 g of a white solid (92% yield).
$^1H$ NMR (80MHz, $CDCl_3$) δ (TMS): 7.32 (m, 5H, Ph), 6.57 (m, OH), 3.80 (dt, J = 9.0 Hz, J = 5.8 Hz, 1H, $CHCF_3$), 2.98 (m, 2H, $CH_2CO$).

**PREPARATION 33**

**3-(1-pyrrolyl)-3-phenylpropionic acid**

To a solution of 1.5 g (8.8 mmol) of D,L-3-amino-3-phenylpropionic acid in 20 mL of AcOH glacial, were added 1.15 mL (8.8 mmol) of 2,5-dimethoxytetrahydrofuran. The mixture was then refluxed for 1 h. After removal of the solvents, the crude was purified by chromatography on silica gel (hexane : ethyl acetate, 20%) to give 1.04 g of the title compound of this example as an oil (55% yield).
$^1H$ NMR (80MHz, $CDCl_3$) δ (TMS): 10.87 (s, 1H, OH), 7.22 (m, 5H, Ph), 6.72 (t, J = 2Hz, 2H, pyrr), 6.16 (t, J = 2Hz, 2H, pyrr), 5.63 (t, J = 7.6Hz, 1H, CHN), 3.22 (d, J = 7.7Hz, 2H, $CH_2CO$).

**PREPARATION 34**

**3-(2-oxo-3-oxazolidinyl)-3-phenylpropionic acid**

To a cooled (0 °C) solution of 1.5 g (8.8 mmol) of D,L-3-amino-3-phenylpropionic acid in 11 mL of tetrahydrofuran, 11 mL of water and 11 mL of 2N NaOH solution, were added 0.95 mL (8.8 mmol) of 2-bromoethyl chloroformate. The mixture was stirred at room temperature for 18 h. The mixture was then acidified with 5N HCl solution and extracted with ethyl acetate. The organic phase was separated, dried over sodium sulphate and the solvent was removed. The resulting crude was dissolved in 25 mL of DMF and cooled to 0 °C. To that solution, 0.63 g (16 mmol) of 50% NaH were added and the resulting mixture was heated at 80°C for 18 h. After removing the solvents, 0.5 N HCl solution and ethyl acetate were added. The organic phase was separated, dried over sodium sulphate and evaporated, to give a crude that was purified by chromatography on silica gel eluting with ethyl acetate, to afford 0.75 g of the title compound of this example as a white solid (38% yield). $^1$H NMR (80MHz, $CDCl_3$) δ (TMS): 9.11 (s, 1H, OH), 7.34 (m, 5H, Ph), 5.41 (dd, J = 8.9Hz, J = 6.7Hz, 1H, CHN), 4.36 (m, 2H), 3.55 (m, 2H), 3.12 (m, 2H).

**PREPARATION 35**

**3-Phenyl-(3-phenylamino)propionic acid**

**a) Ethyl 3-Phenyl-(3-phenylamino)propionate**

To a solution of 3.37 g (26 mmol) of aniline hydrochloride and 5 mL (26 mmol) of ethyl benzoylacetate in 70 mL of methanol, were added 1.75 g of $NaBH_3CN$. After stirring at room temperature for 18 h, the solvent was removed and 0.5 N HCl and ether were added. The aqueous phase was separated, basified and extracted with chloroform. The organic phase was separated, dried and evaporated to give a crude that was purified by chromatography on silica gel (hexane : ethyl acetate, 5%), to afford 4.3 g of the title compound of this example as a white solid (62% yield).
$^1$H NMR (80MHz, $CDCl_3$) δ (TMS): 7.25 (m, 8H, Ph), 6.60 (m, 3H, Ph), 4.82 (t, J = 6.3Hz, 1H, CHN), 4.50 (m, NH), 4.10 (q, J = 7.2 Hz, 2H, $\underline{CH_2}CH_3$), 2.79 (d, J = 6.4 Hz, 2H, $CH_2CO$), 1.16 (t, J = 7.2 Hz, 3H, $CH_2\underline{CH_3}$).

**b) 3-Phenyl-(3-phenylamino)propionic acid**

Following the procedure described in preparation 22d, but starting from the product obtained in preparation 35a, the title compound of this example was obtained (76% yield).
m.p.: 110-111 °C;
$^1$H NMR (80MHz, $CDCl_3$) δ (TMS): 7.10 (m, 8H, Ph), 6.63 (m, 3H, Ph), 4.85 (t, J = 6.3Hz, 1H, CHN), 4.05 (m, OH, NH), 2.85 (d, J = 6.5 Hz, 2H, $CH_2CO$).

**PREPARATION 36**

**3-(N-acetyl-N-hydroxyamino)-3-phenylpropionic acid**

**a) 3-(N-hydroxyamino)-3-phenylpropionic acid**

A hot solution of 13.9 g (0.2 mol) of hydroxylamine hydrochloride in 10 mL of water was added to a hot solution prepared from 4.3 g (0.19 mol) of Na and 160 mL of ethanol. After cooling, the sodium chloride formed was removed by filtration. 18 g (0.12 mol) of cinnamic acid were added and the mixture was heated at reflux for 1 h. The mixture was allowed to cool to room temperature and the white precipitate formed was separated and dried (9.9 g, 47%).
$^1$H NMR (80MHz, DMSO-$d_6$) δ (TMS): 7.30 (m, 5H, Ph), 5.30 (m, OH, NH), 4.19 (t, J = 7.1 Hz, 1H, CHNH), 2.82 (dd, J = 6.9 Hz, J = 15.5 Hz, 1H, $CH_2CO$), 2.40 (dd, J = 7.6 Hz, J = 15.5 Hz, 1H, $CH_2CO$).

**b) 3-(N-acetyl-N-hydroxyamino)-3-phenylpropionic acid**

To a solution of 13 g (72 mmol) of the product obtained in preparation 36a in 60 mL of pyridine, were added 30 mL of $Ac_2O$. After stirring for 18 h at room temperature, the solvents were removed. To a cooled

(0 °C) solution of the crude in 97 mL of ethanol, were added dropwise 5.83 g of LiOH in 60 mL of water. The resulting mixture was stirred at room temperature for 18 h. The solvent was removed, and cooled 5N HCl solution and chloroform were added. The organic phase was separated, dried and evaporated to give 13.5 g of a white solid (84%).
$^1$H NMR (80MHz, $CD_3OD$, $CDCl_3$) δ (TMS): 7.34 (m, 5H, Ph), 5.99 (m, 1H, CHN), 4.12 (s, OH), 2.96 (m, 2H, $CH_2CO$), 2.13 (s, 3H, MeCO).

**PREPARATION 37**

**3-(N-(N'-trimethylsilylcarbamoyl)-N-hydroxyamino)-3-phenylpropionic acid**

To a suspension of 2.8 g (27.5 mmol) of the product obtained in preparation 36a in 30 mL of dimethylformamide, were added 2.14 mL of trimethylsilylisocyanate. The resulting mixture was heated to 60°C for 1.5 h. After removing the solvent, 5.1 g of a violet solid were obtained.
$^1$H NMR (80MHz, DMSO-$d_6$) δ (TMS): 10.14 (m, OH), 7.29 (m, 5H, Ph), 6.22 (m, OH, NH), 5.58 (t, J = 7.3 Hz, 1H, CHNH), 2.72 (m, 2H, $CH_2CO$), 0.06 (s, 9H, $Me_3Si$).

**PREPARATION 38**

**3-(N-methylcarbamoyl-N-hydroxyamino)-3-phenylpropionic acid**

Following the procedure described in preparation 37, but using methylisocyanate, the title compound of this example was obtained (85% yield).
$^1$H NMR (80MHz, $CD_3OD$, $CDCl_3$) δ (TMS): 7.33 (m, 5H, Ph), 5.71 (t, J = 7.5 Hz, 1H, CHN), 4.34 (m, OH, NH), 2.87 (m, 2H, $CH_2CO$), 2.72 (s, 3H, MeNH).

**PREPARATION 39**

**3-((N'-hydroxy-N'-methylcarbamoyl)amino)-3-phenylpropionic acid**

**a) *t*-butyl 3-((N'-hydroxy-N'-methylcarbamoyl)amino)-3-phenylpropionate**

To a solution of 2.75 g (11 mmol) of *t*-butyl 3-carboxy-3-phenylpropionate in 90 mL of benzene and 1.53 mL (11 mmol) of triethylamine, were added 2.38 mL (11 mmol) of diphenylphosporylazide. After heating the mixture to 90°C for 1 h, a solution of 1.88 g (22 mmol) of N-methylhydroxylamine hydrochloride in 0.8 mL of water and 3.06 mL of triethylamine was added. The resulting mixture was then heated to 90°C for 18 h. 55 mL of saturated ammonium chloride solution were added and the mixture was extracted with ethyl acetate. The organic phase was separated, dried and evaporated to give a crude that was purified by chromatography on silica gel (chloroform : methanol, 3%), yielding 2.34 g of the title compound of this example as a white solid (73% yield).
$^1$H NMR (80MHz, $CDCl_3$) δ (TMS): 7.26 (m, 6H, Ph, OH), 6.90 (broad d, J = 8.9 Hz, 1H, NH), 5.19 (q, J = 8.1 Hz, 1H, CHN), 3.02 (s, 3H, MeN), 2.73 (d, J = 6.3 Hz, 2H, $CH_2CO$), 1.31 (s, 9H, *t*-Bu).

**b) 3-((N'-hydroxy-N'-methylcarbamoyl)amino)-3-phenylpropionic acid**

Following the procedure described in preparation 20c, but using the product obtained in preparation 39a, the title compound of this example was obtained (80% yield).
$^1$H NMR (80MHz, $CDCl_3$) δ (TMS): 7.29 (m, 5H, Ph), 5.22 (t, J = 5.8 Hz, 1H, CHN), 4.54 (m, NH, OH), 3.09 (s, 3H, MeN), 2.80 (d, J = 6.4 Hz, 2H, $CH_2CO$).

**EXAMPLE 1**

**1-(3,3-Diphenylpropionyl)-4-[(2-methyl-3-pyridyl)cyanomethyl]piperazine, dihydrochloride**

**a) 1-(3,3-Diphenylpropionyl)-4-[(2-methyl-3-pyridyl)cyanomethyl]piperazine**

Following the procedure described in preparation 13, but using the product obtained in preparation 6 instead of the product obtained in preparation 2, the title compound of this example was obtained (53%

yield).
m.p.: 101.2-102.1 ° C;
IR(film) ν: 3019, 2913, 2818, 1635, 1437, 1241, 998, 700 $cm^{-1}$;
$^1H$ NMR (80MHz, $CDCl_3$) δ (TMS): 8.52 (broad d, J = 4.9Hz, 1H, pyr), 7.78 (broad d, J = 8Hz, 1H, pyr), 7.24 (s, 11H, pyr + Ph), 4.83 (s, 1H, CHCN), 4.64 (t, J = 7.6Hz, 1H, $CHPh_2$), 3.42 (m, 4H, pip), 3.04 (d, J = 7.6Hz, 2H, $CH_2CO$), 2.60 (s, 3H, $CH_3$ pyr), 2,32 (m, 4H, pip).
Analysis Calcd. for $C_{27}H_{28}N_4O$ . 1/4 $H_2O$: C 75.59%, H 6.69%, N 13.06%. Found: C 75.59%, H 6.80%, N 12.76%.

**b) Title compound of this example**

To a cooled (0 ° C) solution of 0.5 g of the compound obtained in example 1a in 5 mL of ethyl acetate, were added 1.5 mL of a diethyl ether solution saturated with hydrochloric acid gas, yielding 0.36 g of a white solid (72% yield).
m.p.: 99.3-108.7 ° C;
Analysis Calcd. for $C_{27}H_{28}N_4O.2HCl.1/2H_2O$: C 64.03%, H 6.17%, N 11.06%. Found: C 64.20%, H 6.13%, N 10.86%.

**EXAMPLE 2**

**1-(3,3-Diphenylpropionyl)-4-[(2-methyl-3-pyridyl)ethoxycarbonylmethyl]piperazine, dihydrochloride**

**a) 1-(3,3-diphenylpropionyl)-4-[(2-methyl-3-pyridyl)ethoxycarbonylmethyl]piperazine**

To 0.54 mL of a solution of 1 g of sodium in 30 mL of ethanol, cooled with an ice bath, were added dropwise 0.9 g of the product obtained in preparation 14 solved in 0.5 mL of abs. ethanol and the mixture was stirred for 18 h. Ethanol was evaporated, 5 mL of water were added and the resulting solution was extracted with chloroform. Evaporation of the solvent afforded 414 mg of a crude that was purified by chromatography on silica gel (chloroform : methanol 1%) to give 0.100 g of the product (13% yield).
$^1H$ NMR (80MHz, $CDCl_3$) δ (TMS): 8.42 (dd, $J_a$ = 4.6Hz, $J_b$ = 1.7Hz, 1H, pyr), 7.78 (dd, $J_a$ = 7.5Hz, $J_b$ = 1.7Hz, 1H, pyr), 7.25 (m, 11H, pyr + Ph), 4.62 (t, J = 7.4Hz, 1H, $CHPh_2$), 4.17 (s, 1H, CHCOOEt), 4.14 (q, J = 6.8Hz, 2H, $OCH_2CH_3$), 3.6-3.2 (complex signal, 4H, pip), 3.02 (d, J = 7.4Hz, 2H, $CH_2CO$), 2.63 (s, 3H, $CH_3$), 2,26 (m, 4H, pip), 1.19 (t, J = 6.8Hz, 3H, $OCH_2CH_3$).

**b) Title compound of this example**

Following the procedure described in example 1b, but using the compound obtained in example 2a, the title compound of this example was obtained as a white solid (63% yield).
m.p.: 130.0-133.3 ° C;
Analysis Calcd. for $C_{29}H_{33}N_3O_3.2HCl.1/4H_2O$ : C 62.31%, H 6.38%, N 7.51%. Found: C 62.50%, H 6.52%, N 7.36%.

**EXAMPLE 3**

**1-(3,3-Diphenylpropionyl)-4-[1-(2-methyl-3-pyridyl)-2-(methoxyimino)propyl]piperazine**

To a cooled solution (ice bath) of 0.3 g (0.5 mmol) of the product obtained in preparation 15 in tetrahydrofuran, were added dropwise 0.093 mL (0.6 mmol) of 1,8-diazabicyclo[5.4.0]undec-7-ene and the mixture was stirred for 1 h at room temperature. Concentration to dryness afforded a crude that was purified by chromatography on silica gel eluting with ethyl acetate, to give 23 mg of the product (10% yield).
m.p.: 49.7-54.3 ° C;
$^1H$ NMR (80MHz, $CDCl_3$) δ ppm (TMS): 8.39 (dd, $J_a$ = 4.5Hz, $J_b$ = 1.6Hz, 1H, pyr), 7.84 (dd, $J_a$ = 7.3Hz, $J_b$ = 1.6Hz, 1H, pyr), 7.23 (m, 11H, pyr + Ph), 4.66 (t, J = 7.5Hz, 1H, $CHCH_2$), 4.00 (s, 1H, CHN), 3.85 (s, 3H, $OCH_3$), 3.45 (m, 4H, pip), 3.00 (d, J = 7.4Hz, 2H, $CH_2CO$), 2.59 (s, 3H, $CH_3$ pyr), 2.20 (m, 4H, pip), 1.63 (s, 3H, $CH_3C=N$).
Analysis Calcd. for $C_{29}H_{34}N_4O_2.H_2O$: C 71.28%, H 7.43%, N 11.47%. Found: C 71.90%, H 7.31%, N 11.07%.

## EXAMPLE 4

### 1-(3,3-Diphenyl-3-hydroxypropionyl)-4-[(2-methyl-3-pyridyl)cyanomethyl]piperazine

Following the procedure described in preparation 13, but using 3,3-diphenyl-3-hydroxypropionic acid and the product obtained in preparation 6, the title compound of this example was obtained (68% yield).
m.p.: 189.3-189.8 °C;
IR (film) ν: 3320, 3050, 2920, 1612, 1444, 1229, 997, 700 $cm^{-1}$;
$^1H$ NMR (80MHz, $CDCl_3$) δ ppm (TMS): 8.55 (broad d, J = 4Hz, 1H, pyr), 7.83 (dd, $J_a$ = 6.4Hz, $J_b$ = 1.6Hz, 1H, pyr), 7.32 (m, 11H, pyr + Ph), 6.34 (broad s, 1H, OH), 4.89 (s, 1H, CHCN), 3.50 (m, 4H, pip), 3.21 (s, 2H, $CH_2CO$), 2.64 (s, 3H, $CH_3$), 2.45 (m, 4H, pip).
Analysis Calcd. for $C_{27}H_{28}N_4O_2$.1/4$Et_2O$: C 73.25%; H 6.69%; N 12.20%. Found: C 73.21%; H 6.93%; N 12.30%.

## EXAMPLE 5

### 1-(3,3-Diphenylpropenoyl)-4-[(2-methyl-3-pyridyl)cyanomethyl]piperazine

Following the procedure described in preparation 13, but using 3,3-diphenylpropenoic acid and the product obtained in preparation 6, the title compound of this example was obtained (52% yield).
m.p.: 75.1-80.4 °C;
IR(KBr) ν: 3048, 2916, 1625, 1438, 1246, 997, 699 $cm^{-1}$;
$^1H$ NMR (80MHz, $CDCl_3$) δ (TMS): 8.50 (dd, $J_a$ = 4.4Hz, $J_b$ = 1.4Hz, 1H, pyr), 7.73 (dd, $J_a$ = 8.0Hz, $J_b$ = 1.4Hz, 1H, pyr), 7.31 (m, 11H, pyr + Ph), 6.28 (s, 1H, CHCO), 4.76 (s, 1H, CHCN), 3.52 (m, 2H, pip), 3.25 (m, 2H, pip), 2.56 (s, 3H, $CH_3$ pyr), 2.37 (m, 2H, pip), 1.94 (m, 2H, pip).
Analysis Calcd. for $C_{22}H_{26}N_4O_4$ . 1/2 $H_2O$: C 63.00%; H 6.44%; N 13.36%. Found: 63.02%; H 6.20%; N 13.14%.

## EXAMPLE 6

### 1-[3,3-bis(4-fluorophenyl)-3-hydroxypropionyl]-4-[(2-methyl-3-pyridyl)cyanomethyl]piperazine

Following the procedure described in preparation 13, but using 3,3-bis(4-fluorophenyl)-3-hydroxypropionic acid and the product obtained in preparation 6, the title compound of this example was obtained (93% yield).
m.p.: 70.8-77.8 °C;
IR (film) ν: 3319, 2922, 1727, 1612, 1500, 1439, 1228 $cm^{-1}$;
$^1H$ NMR (80MHz, $CDCl_3$) δ (TMS): 8.55 (broad d, 1H, pyr), 7.78 (broad d, J = 7.2Hz, 1H, pyr), 7.29 (m, 9H, pyr + Ph), 6.40 (s, 1H, OH), 4.94 (s, 1H, CHCN), 3.52 (m, 4H, pip), 3.16 (s, 2H, $CH_2CO$), 2.63 (s, 3H, $CH_3$), 2.53 (m, 4H, pip).
Analysis Calcd. for $C_{27}H_{26}F_2N_4O_2$.1/2$H_2O$: C 66.79%; H 5.61%; N 11.54%. Found: C 66.99%; H 5.88%; N 11.32%.

## EXAMPLE 7

### 1-[3-hydroxy-3-phenyl-3-(3-pyridyl)propionyl]-4-[(2-methyl-3-pyridyl)cyanomethyl]piperazine

Following the procedure described in preparation 13, but using 3-hydroxy-3-phenyl-3-(3-pyridyl)-propionic acid and the product obtained in preparation 6, the title compound of this example was obtained (47% yield).
m.p.: 101-105 °C;
Analysis Calcd. for $C_{26}H_{27}N_5O_2$.1/2$H_2O$.1/3$Et_2O$: C 69.07%, H 6.59%, N 14.74 %. Found: C 69.46%, H 6.64%, N 14.40%.

**EXAMPLE 8**

**1-(3,3-Diphenyl-3-ethoxycarbonylpropionyl)-4-[(2-methyl-3-pyridyl)cyanomethyl]piperazine**

Following the procedure described in preparation 13, but starting from the acid obtained in preparation 20 and the product obtained in preparation 6, the title compound of this example was obtained (26% yield).
m.p.: 82.9-91.1 °C;
IR(KBr) $\nu$: 2925, 1725, 1642, 1439, 1231, 1190, 998, 698 $cm^{-1}$;
$^1H$ NMR (80MHz, $CDCl_3$) $\delta$ (TMS): 8.53 (broad d, J = 4Hz, 1H, pyr), 7.78 (broad d, J = 6Hz, 1H, pyr), 7.27 (m, 11H, pyr + Ph), 4.84 (s, 1H, CHCN), 4.23 (q, J = 7.2Hz, 2H, $OCH_2CH_3$), 3.47 (s, 2H, $CH_2CO$), 3.32 (m, 4H, pip), 2.60 (s, 3H, $CH_3$ pyr), 2.35 (complex signal, 4H, pip), 1.19 (t, J = 7.2Hz, 3H, $OCH_2CH_3$).
Analysis Calcd. for $C_{30}H_{32}N_4O_3$ . 1/4$H_2O$: C 71.90%; H 6.53%; N 11.18%. Found: C 71.74%; H 6.71%; N 10.93%.

**EXAMPLE 9**

**1-(3-Phenyl-3-hydroxybutanoyl)-4-[(2-methyl-3-pyridyl)cyanomethyl]piperazine**

Following the procedure described in preparation 13, but using 3-phenyl-3-hydroxybutyric acid and the product obtained in preparation 6, the title compound of this example was obtained (44% yield).
m.p.: 56.7-64.3 °C;
IR(KBr) $\nu$: 3393, 2919, 2821, 1612, 1439, 1232, 998 $cm^{-1}$;
$^1H$ NMR (80MHz, $CDCl_3$) $\delta$ (TMS): 8.53 (broad d, J = 4.4Hz, 1H, pyr), 7.80 (broad d, J = 7.8Hz, 1H, pyr), 7.36 (m, 6H, pyr + Ph), 5.69 (broad s, 1H, OH), 4.85 (s, 1H, CHCN), 3.41 (m, 4H, pip), 2.7-2.2 (complex signal, 9H, 4H pip, $CH_2CO$, $CH_3$ pyr), 1.59 (s, 3H, $CH_3$).
Analysis Calcd. for $C_{22}H_{26}N_4O_2$ . 1/4$H_2O$: C69.00%; H 6.97%; N 14.63%. Found: C 69.14%; H 6.92%; N 14.25%.

**EXAMPLE 10**

**1-(3-Phenylbutanoyl)-4-[(2-methyl-3-pyridyl)cyanomethyl]piperazine**

Following the procedure described in preparation 13, but using 3-phenylbutyric acid and the product obtained in preparation 6, the title compound of this example was obtained (70% yield).
m.p.: 103.7-110.6 °C;
IR(KBr) $\nu$: 3020, 2953, 2919, 1632, 1437, 1221, 1126, 998, 700 $cm^{-1}$;
$^1H$ NMR (80MHz, $CDCl_3$) $\delta$ (TMS): 8.52 (broad d, J = 4Hz, 1H, pyr), 7.78 (broad d, J = 7.2Hz, 1H, pyr), 7.25 (m, 6H, pyr + Ph), 4.85 (s, 1H, CHCN), 3.42 (m, 5H, 4H pip, CHPh), 2.61 (s, 3H, $CH_3$ pyr), 2.46 (m, 6H, 4H pip, $CH_2CO$), 1.34 (d, J = 6.4Hz, 3H, $CH_3CH$).
Analysis Calcd. for $C_{22}H_{26}N_4O$ . 1/4 $H_2O$: C 72.00%; H 7.28%; N 15.26%. Found: C 72.04%; H 7.11%; N 14.94%.

**EXAMPLE 11**

**1-(3-Phenyl-3-hydroxy-3-(trifluoromethyl)propionyl)-4-[(2-methyl-3-pyridyl)cyanomethyl]piperazine**

Following the procedure described in preparation 13, but using the acid obtained in preparation 21 and the product obtained in preparation 6, the title compound of this example was obtained (24% yield).
m.p.: 69.8-77.8 °C;
IR(KBr) $\nu$: 3318, 3056, 2923, 1618, 1439, 1237, 1167, 997, 705 $cm^{-1}$;
$^1H$ NMR (80MHz, $CDCl_3$) $\delta$ (TMS): 8.55 (broad d, J = 4.8Hz, 1H, pyr), 7.81 (broad d, J = 8Hz, 1H, pyr), 7.43 (m, 6H, pyr + Ph), 6.69 (s, 1H, OH), 4.89 (s, 1H, CHCN), 3.52 (m, 4H, pip), 3.15 (s, 1H, $CH_2CO$), 3.07 (s, 1H, $CH_2CO$), 2.63 (s, 3H, $CH_3$), 2.47 (m, 4H, pip).
$^{13}C$ NMR (20.15 MHz, $CDCl_3$) $\delta$ (TMS): 168.95 (C), 162.13 (C), 160.18 (C), 149.81 (CH), 141.97 (C), 136.16 (CH), 128.83 (CH), 128.49 (CH), 126.22 (CH), 121.12 (CH), 117.81 (C), 113.88 (C), 59.74 (CHCN), 45.48 ($CH_2$), 41.27 ($CH_2$), 34.80 ($CH_2$), 21.98 ($CH_3$).
Analysis Calcd. for $C_{22}H_{23}F_3N_4O_2$: C 61.10%; H 5.36%; N 12.96%. Found: C 61.09%; H 5.63%; N 12.60%.

**EXAMPLE 12**

**1-(3-methylbutanoyl)-4-[(2-methyl-3-pyridyl)cyanomethyl]piperazine**

Following the procedure described in preparation 13, but using 3-methylbutyric acid and the product obtained in preparation 6, the title compound of this example was obtained (58% yield).
m.p.: 120.7-120.7 ° C;
IR (film) ν: 3066, 2951, 2809, 2223, 1623, 1443, 1233, 1118, 998, 919 $cm^{-1}$;
$^1H$ NMR (80MHz, $CDCl_3$) δ (TMS): 8.54 (broad d, J = 4.4Hz, 1H, pyr), 7.83 (broad d, J = 8.0Hz, 1H, pyr), 7.22 (dd, $J_a$ = 8.0Hz, $J_b$ = 4.4Hz, 1H, pyr), 4.93 (s, 1H, CHCN), 3.57 (m, 4H, pip), 2.85 (s, 3H, $CH_3$ pyr), 2.55 (m, 4H, pip), 2.18 (s broad, 2H, $CH_2CO$), 2.00 (m, 1H, $\underline{CH}CH_3$), 0.97 (d, J = 6.2Hz, 6H, $\underline{CH_3}CH$).
Analysis Calcd. for $C_{17}H_{24}N_4O$ . 1/4 $Et_2O$: C 67.87%; H 8.47%; N 17.58%. Found: C 68.08%; H 8.33%; N 17.89%.

**EXAMPLE 13**

**1-[3-(N-(*tert*-butoxycarbonyl)amino)-3-phenylpropionyl]-4-[(2-methyl-3-pyridyl)cyanomethyl]-piperazine**

Following the procedure described in preparation 13, but using the acid obtained in preparation 19 and the product obtained in preparation 6, the title compound of this example was obtained (71% yield).
m.p.: 86.4-90.7 ° C;
IR (film) ν: 3365, 2971, 2923, 1703, 1631, 1438, 1244, 1164, 998, 700 $cm^{-1}$;
$^1H$ NMR (80MHz, $CDCl_3$) δ (TMS): 8.53 (dd, $J_a$ = 4.8Hz, $J_b$ = 1.3Hz, 1H, pyr), 7.78 (broad d, J = 7.7Hz, 1H, pyr), 7.30 (s, 5H, Ph), 7.20 (dd, $J_a$ = 7.7Hz, $J_b$ = 4.8Hz, 1H, pyr), 6.09 (broad d, 1H, NH), 5.04 (m, 1H, CHNH), 4.84 (s, 1H, CHCN), 3.52 (m, 2H, pip), 3.23 (m, 2H, pip), 2.86 (dd, 2H, $J_a$ = 5.4Hz, $J_b$ = 9.5Hz, $\underline{CH_2}CO$), 2.60 (s, 3H, $CH_3$ pyr), 2.41 (m, 4H, pip), 1.41 (s, 9H, $CH_3$ (BOC)).
Analysis Calcd. for $C_{26}H_{33}N_5O_3$ . 1/4 $H_2O$: C 66.71%; H 7.21%; N 14.96%. Found: C 66.67%; H 7.26%; N 14.71%

**EXAMPLE 14**

**1-(3-Phenyl-3-aminopropionyl)-4-[(2-methyl-3-pyridyl)cyanomethyl]piperazine**

Following the procedure described in preparation 6, but using the product obtained in example 13 instead of the product obtained in preparation 5, a crude was obtained which was purified by chromatography on silica gel (chloroform : methanol : ammonia, 60: 2: 0.2), to give the title compound of this example (71% yield).
m.p.: 39.6-49.1 ° C;
IR (film) ν: 3600-3100, 3021, 2908, 1631, 1438, 1224, 1126, 998, 701 $cm^{-1}$;
$^1H$ NMR (80MHz, $CDCl_3$) δ (TMS): 8.53 (broad d, J = 4.8Hz, 1H, pyr), 7.80 (broad d, J = 7.8Hz, 1H, pyr), 7.35 (s, 5H, Ph), 7.21 (dd, $J_a$ = 7.8Hz, $J_b$ = 4.8Hz, 1H, pry), 4.88 (s, 1H, CHCN), 4.52 (t, J = 6.3Hz, 1H, $\underline{CH}NH_2$), 3.59 (m, 2H, pip), 3.36 (m, 2H, pip), 2.62 (s, 3H, $CH_3$ pyr), 2.50 (m, 6H, 4H pip, $CH_2CO$), 1.96 (s, $\underline{2H}$, $NH_2$).
Analysis Calcd. for $C_{21}H_{25}N_5O$ . $H_2O$: C 66.12%, H 7.13%, N 18.36%. Found: C 66.36%, H 6.78%, N 18.32%.

**EXAMPLE 15**

**1-[3-(N-Benzylamino)-3-phenylpropionyl]-4-[(2-methyl-3-pyridyl)cyanomethyl]piperazine**

To a solution of 2 g (5.5 mmol) of the product obtained in example 14 in 2 mL of methanol, were added 0.6 mL (5.5 mmol) of benzaldehyde. MeOH / HCl solution was added until pH = 6-7 and finally 0.35 g (5.5 mmol) of sodium cyanoborohydride were added, and the mixture was stirred at room temperature for 18 h. The solvent was removed, 0.1N NaOH was added and the mixture was extracted with chloroform, affording 2.69 g of a crude that was purified by chromatography on silica gel (chloroform : methanol, 3%), to give the title compound of this example (55% yield).
m.p.: 52.0-58.0 ° C;

IR (film) ν: 3312, 3019, 2822, 1631, 1437, 1224, 1126, 998, 752, 700 $cm^{-1}$;
$^1H$ NMR (80MHz, $CDCl_3$) δ (TMS): 8.52 (dd, $J_a$ = 5Hz, $J_b$ = 1.4Hz, 1H, pyr), 7.78 (broad d, J = 8.0Hz, 1H, pyr), 7.32 (m, 11H, pyr + Ph), 4.84 (s, 1H, CHCN), 4.19 (t, J = 6.9Hz, 1H, CHNH), 3.77 (complex signal, 4H, $CH_2NH$, 2H pip), 3.43 (m, 2H, pip), 2.55 (complex signal, 10H, 4H pip, $CH_2CO$, $CH_3$ pyr, NH).
Analysis Calcd. for $C_{28}H_{31}N_5O$ . 1/2 $H_2O$: C 72.70%, H 6.97%, N 15.14%. Found: C 72.67%, H 6.88%, N 15.29%.

## EXAMPLE 16

### 1-[3-(N-Benzoylamino)-3-phenylpropionyl]-4-[(2-methyl-3-pyridyl)cyanomethyl]piperazine

To a solution of 0.5 g (1.4 mmol) of the product obtained in example 14 in chloroform, were added 0.29 mL of triethylamine. Then, 0.18 mL (1.5 mmol) of benzoyl chloride were added and the mixture was stirred 18 h at room temperature. The resulting solution was treated with water and 0.1 N sodium hydroxide until basic pH and then extracted with chloroform. The organic phase was dried over sodium sulfate and evaporated, affording 0.75 g of a crude that was purified by chromatography on silica gel eluting with ethyl acetate, to give 0.34 g of the title compound of this example (52% yield).
m.p.: 112.3-126.3 °C;
IR (film) ν: 3320, 3051, 2912, 1637, 1437, 1301, 998, 699 $cm^{-1}$;
$^1H$ NMR (80MHz, $CDCl_3$) δ (TMS): 8.49 (m, 2H, pyr), 7.82 (m, 3H, Ar), 7.33 (m, 9H, 8H Ar, NH), 5.56 (m, 1H, CHNH), 4.82 (s, 1H, CHCN), 3.23 (m, 4H, pip), 3.07 (d, J = 5Hz, 1H, $CH_2CO$), 2.87 (d, J = 5Hz, 1H, $CH_2CO$), 2.59 (s, 3H, $CH_3$ pyr), 2.40 (m, 2H, pip), 2.01 (m, 2H, pip).
Analysis Calcd. for $C_{28}H_{29}N_5O_2$ . $H_2O$: C 69.26%, H 6.43%, N 14.42%. Found: C 69.26 %, H 6.14%, N 14.16%.

## EXAMPLE 17

### 1-[3-(N-acetylamino)-3-phenylpropionyl]-4-[(2-methyl-3-pyridyl)cyanomethyl]piperazine

To a solution of 0.4 g (1.1 mmol) of the product obtained in example 14 in 2 mL of pyridine, was added 1 mL of acetic anhydride and the mixture was stirred at room temperature for 18 h. After evaporating the solvents, 0.1N NaOH was added and the resulting mixture was extracted with chloroform. The organic phase was dried over sodium sulfate and evaporated, affording 0.63 g of a crude that was purified by chromatography on silica gel, eluting with chloroform-methanol mixtures of increasing polarity. 0.35 g of the title compound of this example were obtained (78% yield).
m.p.: 81.3-100.0 °C;
IR(KBr) ν: 3287, 3054, 2914, 1642, 1438, 1300, 1126, 998, 754, 700 $cm^{-1}$;
$^1H$ NMR (80MHz, $CDCl_3$) δ (TMS): 8.53 (broad d, J = 4.8Hz, 1H, pyr), 7.78 (broad d, J = 8Hz, 1H, pyr), 7.30 (s, 7H, pyr, 5H Ph, NH), 5.36 (q, J = 7.5Hz, 1H, CHNH), 4.84 (s, 1H, CHCN), 3.25 (complex signal, 4H, pip), 2.97 (d, J = 5.3Hz, 1H, $CH_2CO$), 2.78 (d, J = 5.3Hz, 1H, $CH_2CO$), 2.60 (s, 3H, $CH_3$ pyr), 2.41 (m, 4H, pip), 2.03 (s, 3H, $CH_3CO$).
Analysis Calcd. for $C_{23}H_{27}N_5O_2$. $1H_2O$: C 65.23%, H 6.90%, N 16.54%. Found: C 65.04%, H 6.80%, N 16.04%.

## EXAMPLE 18

### 1-(4,4-Diphenylbutanoyl)-4-[(2-methyl-3-pyridyl)cyanomethyl]piperazine

Following the procedure described in preparation 13, but starting from 4,4-diphenylbutyric acid (AA. Cordi et al., Eur. J. Med. Chem., 1990, 25, 557) and the product obtained in preparation 6, the title compound of this example was obtained (58% yield).
m.p.: 59.8-71.1 °C;
IR (film) ν: 3011, 2920, 1637, 1436, 1237, 1125, 998, 703 $cm^{-1}$;
$^1H$ NMR (80MHz, $CDCl_3$) δ (TMS): 8.55 (broad d, J = 5Hz, 1H, pyr), 7.87 (broad d, J = 7Hz, 1H, pyr), 7.24 (m, 11H, pyr + Ph), 4.88 (s, 1H, CHCN), 3.96 (broad t, J = 7Hz, 1H, $Ph_2CH$), 3.62 (complex signal, 2H, pip), 3.26 (complex signal, 2H, pip), 2.62 (s, 3H, $CH_3$), 2.38 (complex signal, 8H, 4H pip, $CH_2CH_2$).
Analysis Calcd. for $C_{28}H_{30}N_4O$: C 76.68%, H 6.89%, N 12.77%. Found: C 76.80%, H 7.08%, N 12.43%.

## EXAMPLE 19

### 1-(2-Amino-2,2-diphenylacetyl)-4-[(2-methyl-3-pyridyl)cyanomethyl]piperazine

Following the procedure described in preparation 13, but using 2,2-diphenylglycine and the product obtained in preparation 6, the title compound of this example was obtained (77% yield).
m.p.: 84.2-85.6 ° C;
IR (film) $\nu$: 3361, 3292, 3050, 2912, 1727, 1632, 1439, 1232, 1141, 998, 701 $cm^{-1}$;
$^1H$ NMR (80MHz, $CDCl_3$) $\delta$ (TMS): 8.48 (dd, $J_a$ = 4.8Hz, $J_b$ = 1.4Hz, 1H, pyr), 7.70 (dd, $J_a$ = 7.8Hz, $J_b$ = 1.4Hz, 1H, pyr), 7.36 (s, 10H, Ph), 7.14 (dd, $J_a$ = 7.8Hz, $J_b$ = 4.8Hz, 1H, pyr), 4.75 (s, 1H, CHCN), 3.43 (m, 4H, pip), 2.53 (m, 5H, $CH_3$, $NH_2$), 2.3-1.9 (complex signal, 4H, pip).
Analysis Calcd. for $C_{26}H_{27}N_5O$ . 1/4 $H_2O$: C 72.61%, H 6.44 %, N 16.28%.
Found: C 72.72%, H 6.60%, N 15.63%.

## EXAMPLE 20

### 1-(2,2-diphenyl-2-hydroxyacetyl)-4-[(2-methyl-3-pyridyl)cyanomethyl]piperazine

Following the procedure described in preparation 13, but using 2,2-diphenyl-2-hydroxyacetic acid and the product obtained in preparation 6, the title compound of this example was obtained (58% yield).
m.p.: 212.5-212.5 ° C;
IR(KBr) $\nu$: 3359, 3054, 3005, 2914, 2822, 2226, 1631, 1439, 1241, 998, 756, 701 $cm^{-1}$;
$^1H$ NMR (80MHz, $CDCl_3$) $\delta$ (TMS): 8.47 (dd, $J_a$ = 4.8Hz, $J_b$ = 1.4Hz, 1H, pyr), 7.71 (dd, $J_a$ = 8Hz, $J_b$ = 1.4Hz, 1H, pyr), 7.37 (m, 11H, pyr + Ph), 5.69 (s, 1H, OH), 4.75 (s, 1H, CHCN), 3.49 (m, 4H, pip), 2.55 (s, 3H, $CH_3$), 2.18 (m, 4H, pip).
Analysis Calcd. for $C_{26}H_{26}N_4O_2$. 1/2 $H_2O$: C 71.73%, H 6.24%, N 12.87%. Found: C 71.78%, H 6.14%, N 12.62%.

## EXAMPLE 21

### 1-[(S)-2-phenyl-2-methoxy-2-(trifluoromethyl)acetyl]-4-[(2-methyl-3-pyridyl)cyanomethyl]piperazine

Following the procedure described in preparation 13, but using S-(-)-2-phenyl-2-methoxy-2-(trifluoromethyl)acetic acid and the product obtained in preparation 6, the title compound of this example was obtained (13% yield).
m.p.: 70.8-89.5 ° C;
$^1H$ NMR (80MHz, $CDCl_3$) $\delta$ (TMS): 8.49 (broad d, J = 3.9Hz, 1H, pyr), 7.73 (m, 1H, pyr), 7.44 (s, 5H, Ph), 7.19 (m, 1H, pyr), 4.80 (s, 1/2H, CHCN), 4.75 (s, 1/2H, CHCN), 3.70 (s, 3H, $OCH_3$), 3.29 (m, 4H, pip), 2.55 (s, 3H, $CH_3$ pyr), 2.05 (m, 4H, pip).
Analysis Calcd. for $C_{22}H_{23}F_3N_5O_2$ . 1/2 $H_2O$: C 59.85%, H 5.47%, N 12.69%. Found: C 59.53%, H 5.45%, N 12.39%.

## EXAMPLE 22

### 1-[N-(Diphenylmethyl)aminoacetyl]-4-[(2-methyl-3-pyridyl)cyanomethyl]piperazine

Following the procedure described in preparation 13, but using N-(diphenylmethyl)aminoacetic acid and the product obtained in preparation 6, the title compound of this example was obtained (38% yield).
m.p.: 63.1-67.8 ° C;
IR(KBr) $\nu$: 3318, 3052, 3019, 2912, 2818, 2224, 1642, 1437, 1235, 998, 746, 705 $cm^{-1}$;
$^1H$ NMR (80MHz, $CDCl_3$) $\delta$ (TMS): 8.54 (dd, $J_a$ = 4.8Hz, $J_b$ = 1.4Hz, 1H, pyr), 7.80 (dd, $J_a$ = 7.2Hz, $J_b$ = 1.4Hz, 1H, pyr), 7.32 (m, 11H, pyr + Ph), 4.89 (s, 1H), 4.87 (s, 1H), 3.7-3.2 (complex signal, 7H, 4H pip, NH, $CH_2CO$), 2.62 (s, 3H, $CH_3$), 2.49 (m, 4H, pip).
Analysis Calcd. for $C_{27}H_{29}N_5O$ . 1/2 $H_2O$: C 72.29%, H 6.74%, N 15.61%. Found: C 72.07%, H 6.75%, N 15.24%.

**EXAMPLE 23**

**1-[N-(Diphenylmethyl)aminoacetyl]-4-[1-(2-methyl-3-pyridyl)-2-propin-1-yl]piperazine**

Following the procedure described in preparation 13, but using N-(diphenylmethyl)aminoacetic acid and the compound obtained in preparation 18, the title compound of this example was obtained (64% yield). $^1H$ NMR (80MHz, $CDCl_3$) δ (TMS): 8.46 (broad d, J = 4.8Hz, 1H, pyr), 7.9 (broad d, J = 7.6Hz, 1H, pyr), 7.27 (m, 11H, pyr + Ph), 4.85 (s, 1H, $CHPh_2$), 4.71 (d, J = 1.9Hz, 1H, CHC≡CH), 3.62 (m, 2H, pip), 3.37 (s, 2H, $CH_2CO$), 3.22 (m, 2H, pip), 2.64 (s, 3H, $CH_3$ pyr), 2.43 (m, 6H, 4H pip, NH, C≡CH).
Analysis Calcd. for $C_{28}H_{30}N_4O$. 1 $H_2O$: C 73.66%, H 7.06%, N 12.27%. Found: C 73.39%, H 7.01%, N 10.59%.

**EXAMPLE 24**

**1-[N-(Diphenylmethyl)aminoacetyl]-4-[(2-ethyl-3-pyridyl)cyanomethyl]piperazine**

Following the procedure described in preparation 13, but using N-(diphenylmethyl)aminoacetic acid and the compound obtained in preparation 12, the title compound of this example was obtained (30% yield).
m.p.: 58.7-64.3 °C;
IR(KBr) ν: 3312, 3018, 2925, 1642, 1433, 1235, 1125, 998, 746, 705 $cm^{-1}$;
$^1H$ NMR (80MHz, $CDCl_3$) δ (TMS): 8.62 (dd, $J_a$ = 4.8Hz, $J_b$ = 1.9Hz, 1H, pyr), 7.80 (dd, $J_a$ = 8.2Hz, $J_b$ = 1.9Hz, 1H, pyr), 7.28 (m, 11H, pyr + Ph), 4.98 (s, 1H), 4.84 (s, 1H), 3.64 (m, 2H, pip), 3.39 (s, 2H, $CH_2CO$), 3.28 (m, 2H, pip), 2.87 (q, J = 7.6Hz, 2H, $CH_2CH_3$), 2.50 (m, 4H, pip), 2.34 (broad s, 1H, NH), 1.33 (t, J = 7.6Hz, 3H, $CH_2CH_3$).
Analysis Calcd. for $C_{28}H_{31}N_5O$: C 74.14%, H 6.89%, N 15.44%. Found: C 74.17%, H 7.01%, N 15.04%.

**EXAMPLE 25**

**1-[N-(bis(4-fluorophenyl)methyl)aminoacetyl]-4-[(2-methyl-3-pyridyl)cyanomethyl]piperazine**

Following the procedure described in preparation 13, but using N-(bis(4-fluorophenyl)methyl)-aminoacetic acid and the product obtained in preparation 6, the title compound of this example was obtained (52% yield).
m.p.: 65.9-76.1 °C;
IR (film) ν: 3314, 3055, 2919, 2820, 1728, 1643, 1499, 1438, 1239, 1043, 998, 825 $cm^{-1}$;
$^1H$ NMR (80MHz, $CDCl_3$) δ (TMS): 8.54 (dd, $J_a$ = 5.7Hz, $J_b$ = 1.6Hz, 1H, pyr), 7.80 (dd, $J_a$ = 8.2 Hz, $J_b$ = 1.6Hz, 1H, pyr), 7.5-6.8 (complex signal, 9H, pyr + Ph), 4.94 (s, 1H), 4.84 (s, 1H), 3.60 (m, 2H, pip), 3.35 (m, 4H, 2H pip, $CH_2CO$), 2.63 (s, 3H, $CH_3$), 2.57 (m, 5H, 4H pip, NH).
Analysis Calcd. for $C_{27}H_{27}F_2N_5O$ . 1/2$H_2O$: C 66.92%, H 5.82%, N 14.45%. Found: C 67.22%, H 5.78%, N 14.30%.

**EXAMPLE 26**

**1-[N-(Fluorenyl)aminoacetyl]-4-[(2-methyl-3-pyridyl)cyanomethyl]piperazine**

Following the procedure described in preparation 13, but using N-(fluorenyl)aminoacetic acid and the product obtained in preparation 6, the title compound of this example was obtained (31% yield).
m.p.: 67.5-76.0 °C;
IR (film) ν: 3311, 3054, 2912, 2818, 1642, 1435, 1234, 1126, 998, 745 $cm^{-1}$;
$^1H$ NMR (80MHz, $CDCl_3$) δ (TMS): 8.50 (broad d, J = 5Hz, 1H, pyr), 7.8-7.1 (complex signal, 10H), 5.06 (s, 1H, CHNH), 4.81 (s, 1H, CHCN), 3.50 (m, 2H, pip), 2.92 (m, 4H, 2H pip, $CH_2CO$), 2.56 (s, 3H, $CH_3$), 2.37 (m, 5H, 4H pip, NH).
Analysis Calcd. for $C_{27}H_{27}N_5O$ . 7/4$H_2O$: C 69.14%, H 6.54%, N 14.93%. Found: C 69.54%, H 5.88%, N 14.63%.

**EXAMPLE 27**

**1-[N-(1,1-diphenylethyl)aminoacetyl]-4-[(2-methyl-3-pyridyl)cyanomethyl]piperazine**

Following the procedure described in preparation 13, but using the acid obtained in preparation 22 and the product obtained in preparation 6, the title compound of this example was obtained (57% yield).
m.p.: 65.9-70.4 ° C;
IR(KBr) $\nu$: 3309, 3051, 2914, 2816, 1643, 1439, 1236, 1143, 998, 768, 701 $cm^{-1}$;
$^1H$ NMR (80MHz, $CDCl_3$) $\delta$ (TMS): 8.53 (broad d, J = 4.8Hz, 1H, pyr), 7.81 (broad d, J = 8Hz, 1H, pyr), 7.30 (m, 11H, pyr + Ph), 4.88 (s, 1H, CHCN), 3.54 (m, 2H, pip), 3.23 (m, 4H, 2H pip, $CH_2CO$), 2.62 (s, 3H, $CH_3$ pyr), 2.49 (m, 5H, 4H pip, NH), 1.80 (s, 3H, $CH_3CPh_2$).
Analysis Calcd. for $C_{28}H_{31}N_5O$ . $1/2H_2O$: C 72.69%, H 6.97%, N 15.13%. Found: C 72.52%, H 6.92%, N 14.82%.

**EXAMPLE 28**

**1-[(S)-2-(N-(Diphenylmethyl)amino)propionyl]-4-[(2-methyl-3-pyridyl)cyanomethyl]piperazine**

Following the procedure described in preparation 13, but using the acid obtained in preparation 29b and the product obtained in preparation 6, the title compound of this example was obtained (41% yield).
m.p.: 62.5-69.4 ° C;
IR (film) $\nu$: 3600-3100, 3019, 2973, 2923, 1729, 1636, 1438, 1232, 998, 706 $cm^{-1}$;
$^1H$ NMR (80MHz, $CDCl_3$) $\delta$ (TMS): 8.53 (dd, $J_a$ = 4.8Hz, $J_b$ = 1.4Hz, 1H, pyr), 7.80 (broad d, J = 7.8Hz, 1H, pyr), 7.26 (m, 11H, pyr, Ph), 4.88 (s, 1H), 4.74 (s, 1H), 3.74 (m, 2H, pip), 3.49 (q, J = 6.7Hz, 1H, $\underline{CH}CH_3$), 3.18 (m, 2H, pip), 2.62 (s, 3H, $CH_3$ pyr), 2.41 (m, 4H, pip), 2.05 (broad s, 1H, NH), 1.23 (d, J = 6.7Hz, 3H, $CH\underline{CH_3}$).
Analysis Calcd. for $C_{28}H_{31}N_5O$ . $1H_2O$: C 71.31%, H 7.05%, N 14.85%. Found: C 71.35%, H 6.77%, N 14.45%.

**EXAMPLE 29**

**1-[(R)-2-(N-(Diphenylmethyl)amino)propionyl]-4-[(2-methyl-3-pyridyl)cyanomethyl]piperazine**

Following the procedure described in preparation 13, but using the acid obtained in preparation 29a and the product obtained in preparation 6, the title compound of this example was obtained (63% yield).
m.p.: 73.9-83.0 ° C;
IR(KBr) $\nu$: 3600-3100, 3019, 2973, 2923, 1730, 1636, 1434, 1235, 998, 706 $cm^{-1}$;
$^1H$ NMR (80MHz, $D_2O$) $\delta$ (TMS): 8.53 (dd, $J_a$ = 4.8Hz, $J_b$ = 1.5Hz, 1H, pyr), 7.80 (broad d, J = 7.8Hz, 1H, pyr), 7.33 (m, 11H, pyr + Ph), 4.89 (s, 1H), 4.75 (s, 1H), 3.54 (m, 3H, 2H pip, $\underline{CH}CH_3$), 3.17 (m, 2H, pip), 2.62 (s, 3H, $CH_3$ pyr), 2.6-2.2 (complex signal, 5H, 4H pip, NH), 1.23 (d, J = 6.9Hz, 3H, $CH\underline{CH_3}$). Analysis Calcd. for $C_{28}H_{31}N_5O$ . $1/2H_2O$: C 72.70%, H 6.97%, N 15.13%. Found: C 72.64%, H 7.15%, N 14.86%.

**EXAMPLE 30**

**1-[(S)-2-(N-(diphenylmethyl)amino)-3-hydroxypropionyl]-4-[(2-methyl-3-pyridyl)cyanomethyl]-piperazine**

Following the procedure described in preparation 13, but using N-(diphenylmethyl)-L-Serine and the product obtained in preparation 6, the title compound of this example was obtained (39% yield).
m.p.: 70.6-80.4 ° C;
IR(KBr) $\nu$: 3600-3100, 3020, 2917, 1631, 1437, 1235, 1126, 997, 747, 705 $cm^{-1}$;
$^1H$ NMR (80MHz, $CDCl_3$) $\delta$ (TMS): 8.54 (broad d, J = 4.8Hz, 1H, pyr), 7.81 (broad d, J = 8Hz, 1H, pyr), 7.31 (m, 11H, pyr + Ph), 4.89 (s, 1H), 4.82 (s, 1H), 3.60 (m, 5H, CHCO, 2H pip, NH, OH), 3.21 (m, 2H, pip), 2.79 (broad s, 2H, $\underline{CH_2}OH$), 2.62 (s, 3H, $CH_3$ pyr), 2.49 (m, 4H, pip).
Analysis Calcd. for $C_{28}H_{31}N_5O_2$ . $1/4H_2O$: C 70.93%, H 6.68%, N 14.77%. Found: C 71.10%, H 7.06%, N 14.38%.

**EXAMPLE 31**

**1-[N-(Diphenylmethyl)-N-(methylsulfonyl)aminoacetyl]-4-[(2-methyl-3-pyridyl)cyanomethyl]-piperazine**

Following the procedure described in preparation 13, but using the acid obtained in preparation 23 and the product obtained in preparation 6, the title compound of this example was obtained (30% yield).
m.p.: 96.3-100.2 °C;
IR(KBr) ν: 3023, 2924, 2846, 1658, 1569, 1443, 1322, 1141, 998, 919 $cm^{-1}$;
$^1H$ NMR (80MHz, $CDCl_3$) δ (TMS): 8.53 (broad d, J = 4.8Hz, 1H, pyr), 7.78 (broad d, J = 6.3Hz, 1H, pyr), 7.33 (m, 11H, pyr + Ph), 6.23 (s, 1H, $CHPh_2$), 4.86 (s, 1H, CHCN), 4.17 (s, 2H, $CH_2CO$), 3.16 (m, 7H, 4H pip, $CH_3SO_2$), 2.58 (s, 3H, $CH_3$ pyr), 2.39 (m, 4H, pip).
Analysis Calcd. for $C_{28}H_{31}N_5O_3S$. 1/3 $Et_2O$ . 5/4 $H_2O$: C 62.37%, H 6.51%, N 12.40%. Found: C 62.19%, H 6.12%, N 12.25%.

**EXAMPLE 32**

**1-[N-(Diphenylmethyl)-N-formylaminoacetyl]-4-[(2-methyl-3-pyridyl)cyanomethyl]piperazine**

Following the procedure described in preparation 13, but using the acid obtained in preparation 30 and the product obtained in preparation 6, the title compound of this example was obtained (65% yield).
m.p.: 81.2-104.1 °C;
IR(KBr) ν: 3022, 2912, 1665, 1438, 1232, 1143, 998, 748, 700 $cm^{-1}$;
$^1H$ NMR (80MHz, $CDCl_3$) δ (TMS): 8.54 (broad d, J = 4.4Hz, 1H, pyr), 8.07 (s, 1H, HCO), 7.83 (broad d, J = 7.2Hz, 1H, pyr), 7.33 (m, 11H, pyr + Ph), 5.99 (s, 1H, $CHPh_2$), 4.90 (s, 1H, CHCN), 4.16 (s, 2H, $CH_2CO$), 3.52 (m, 4H, pip), 2.64 (s, 3H, $CH_3$), 2.57 (m, 4H, pip).
Analysis Calcd. for $C_{28}H_{29}N_5O_2$ . 3/2$H_2O$: C 68.02%, H 6.51%, N 14.16%. Found: C 68.36%, H 6.20%, N 14.13%.

**EXAMPLE 33**

**1-[N-(Diphenylmethyl)-N-acetylaminoacetyl]-4-[(2-methyl-3-pyridyl)cyanomethyl]piperazine**

To a solution of 0.3 g (0.68 mmol) of the product obtained in example 22 in 3 mL of pyridine, 1 mL of acetic anhydride was added. The mixture was stirred at 65 °C for 18 h and then concentrated to dryness. Water was added and the resulting solution was extracted with chloroform. The organic phase was dried over sodium sulfate and evaporated, yielding 0.27 g of a crude that was purified by chromatography on silica gel (chloroform : methanol, 3%) to give the title compound of this example (44% yield).
m.p.: 87.3-106.3 °C;
IR(KBr) n: 2918, 1657, 1436, 1232, 998, 752, 700 $cm^{-1}$;
$^1H$ NMR (80MHz, $CDCl_3$) δ (TMS): 8.52 (broad d, J = 4.6Hz, 1H, pyr), 7.79 (broad d, J = 8Hz, 1H, pyr),7.26 (m, 11H, pyr + Ph), 6.31 (s, 1H, $CHPh_2$), 4.82 (s, 1H, CHCN), 4.11 (s, 2H, $CH_2CO$), 3.24 (m, 4H, pip), 2.59 (s, 3H, $CH_3$ pyr), 2.30 (m, 4H, pip), 2.15 (s, 3H, $CH_3CO$).
Analysis calcd. for $C_{29}H_{31}N_5O$ . $H_2O$: C 69.72%, H 6.66%, N 14.02%. Found: C 69.32%, H 6.43%, N 13.63%.

**EXAMPLE 34**

**1-[3-(N-(Diphenylmethyl)amino)propionyl]-4-[(2-methyl-3-pyridyl)cyanomethyl]piperazine**

Following the procedure described in preparation 13, but using 3-(N-(diphenylmethyl)amino)propionic acid and the product obtained in preparation 6, the title compound of this example was obtained (46% yield).
m.p.: 53.5-57.9 °C;
IR(KBr) n: 3314, 3054, 2912, 2820, 1632, 1438, 1226, 998, 752, 705 $cm^{-1}$;
$^1H$ NMR (80MHz, $CDCl_3$) δ (TMS): 8.54 (broad d, J = 4.8Hz, 1H, pyr), 7.83 (dd, $J_a$ = 8.2Hz, $J_b$ = 1.6Hz, 1H, pyr), 7.29 (m, 11H, pyr + Ph), 4.90 (s, 1H), 4.85 (s, 1H), 3.51 (m, 4H, pip), 2.87 (m, 2H, $CH_2N$), 2.64 (m, 9H, 4H pip, $CH_3$ pyr, $CH_2CO$), 1.87 (broad s, 1H, NH).

Analysis calcd. for $C_{28}H_{31}N_5O$ . $1/4H_2O$: C 73.41%, H 6.93%, N 15.28%. Found: C 73.36%, H 7.02%, N 15.03%.

## EXAMPLE 35

### 1-[(Diphenylmethylen)aminooxyacetyl]-4-[(2-methyl-3-pyridyl)cyanomethyl]piperazine

Following the procedure described in preparation 13, but starting from the acid obtained in preparation 24 and the product obtained in preparation 6, the title compound of this example was obtained (79% yield).
m.p.: 66.6-78.5 °C;
IR(KBr) n: 3050, 2914, 2821, 1649, 1439, 1235, 997, 773, 697 $cm^{-1}$;
$^1H$ NMR (80MHz, $CDCl_3$) $\delta$ (TMS): 8.54 (broad d, J = 4Hz, 1H, pyr), 7.80 (dd, $J_a$ = 7.6Hz, $J_b$ = 1.4Hz, 1H, pyr), 7.41 (m, 11H, pyr + Ph), 4.85 (s, 1H, CHCN), 4.82 (s, 2H, $CH_2CO$), 3.52 (m, 4H, pip), 2.63 (s, 3H, $CH_3$), 2.46 (m, 4H, pip). Analysis Calcd. for $C_{27}H_{27}N_5O_2$: C 71.50%, H 6.00%, N 15.44%. Found: C 71.36%, H 6.19%, N 15.10%.

## EXAMPLE 36

### 1-[(Diphenylmethoxy)acetyl]-4-[(2-methyl-3-pyridyl)cyanomethyl]piperazine

Following the procedure described in preparation 13, but using the acid obtained in preparation 25 and the product obtained in preparation 6, the title compound of this example was obtained (29% yield).
m.p.: 54.5-57.2 °C;
IR(KBr) n: 3054, 2921, 1648, 1438, 1236, 998, 702 $cm^{-1}$;
$^1H$ NMR (80MHz, $CDCl_3$) $\delta$ (TMS): 8.53 (broad d, J = 5Hz, 1H, pyr), 7.81 (broad d, J = 8Hz, 1H, pyr), 7.32 (m, 11H, pyr + Ph), 5.48 (s, 1H, $CHPh_2$), 4.87 (s, 1H, CHCN), 4.15 (s, 2H, $CH_2CO$), 3.52 (m, 4H, pip), 2.63 (s, 3H, $CH_3$), 2.51 (m, 4H, pip).
Analysis Calcd. for $C_{27}H_{28}N_4O_2$: C 73.61%, H 6.41%, N 12.72%. Found: C 73.86%, H 6.80%, N 12.49%.

## EXAMPLE 37

### 1-[(Diphenylmethyl)thioacetyl]-4-[(2-methyl-3-pyridyl)cyanomethyl]piperazine

Following the procedure described in preparation 13, but using the acid obtained in preparation 26 and the product obtained in preparation 6, the title compound of this example was obtained (25% yield).
m.p.: 183.7-183.9 °C;
IR(KBr) n: 3021, 2910, 2835, 1619, 1445, 1272, 1136, 993, 755 $cm^{-1}$;
$^1H$ NMR (80MHz, $CDCl_3$) $\delta$ (TMS): 8.55 (broad d, J = 5.7Hz, 1H, pyr), 7.85 (broad d, J = 8.4Hz, 1H, pyr), 7.28 (m, 11H, pyr + Ph), 5.33 (s, 1H, $CHPh_2$), 4.90 (s, 1H, CHCN), 3.48 (m, 4H, pip), 3.18 (s, 2H, $CH_2CO$), 2.63 (m, 7H, 4H pip, $CH_3$). Analysis Calcd. for $C_{27}H_{28}N_4OS$: C 71.02%, H 6.18%, N 12.27%. Found: C 70.78%, H 6.49%, N 12.52%.

## EXAMPLE 38

### 1-[(Diphenylmethyl)sulfinylacetyl]-4-[(2-methyl-3-pyridyl)cyanomethyl]piperazine

Following the procedure described in preparation 13, but using the acid obtained in preparation 27 and the product obtained preparation 6, the title compound of this example was obtained (50% yield).
m.p.: 79.0-128.8 °C;
IR(KBr) n: 3021, 2913, 1632, 1438, 1275, 1143, 1051, 997, 749, 703 $cm^{-1}$;
$^1H$ NMR (80MHz, $CDCl_3$) $\delta$ ppm(TMS): 8.54 (broad d, J = 4Hz, 1H, pyr), 7.82 (broad d, J = 8Hz, 1H, pyr), 7.43 (m, 11H, pyr + Ph), 5.29 (s, 1H, $CHPh_2$), 4.90 (s, 1H, CHCN), 3.52 (complex signal, 6H, 4H pip, $CH_2CO$), 2.62 (s, 3H, $CH_3$), 2.56 (m, 4H, pip).
Analysis Calcd. for $C_{27}H_{28}N_4O_2S$ . $7/4H_2O$: C 64.32%, H 6.28%, N 11.11%. Found: C 64.32%, H 5.82%, N 10.79%.

**EXAMPLE 39**

**1-[(Diphenylmethyl)sulfonylacetyl]-4-[(2-methyl-3-pyridyl)cyanomethyl]piperazine**

Following the procedure described in preparation 13, but using the acid obtained in preparation 28 and the product obtained in preparation 6, the title compound of this example was obtained (37% yield).
m.p.: 80.1-109.4 °C;
IR(KBr) n: 3054, 2920, 2821, 1725, 1642, 1439, 1316, 1127, 998, 704 $cm^{-1}$;
$^1H$ NMR (80MHz, $CDCl_3$) $\delta$ (TMS): 8.53 (broad d, J = 4.8Hz, 1H, pyr), 7.72 (m, 5H), 7.36 (m, 7H), 5.95 (s, 1H, $CHPh_2$), 4.88 (s, 1H, CHCN), 3.91 (s, 2H, $CH_2CO$), 3.51 (m, 4H, pip), 2.61 (m, 7H, 4H pip, $CH_3$).
Analysis Calcd. for $C_{27}H_{28}N_4O_3S$: C 66.37%, H 5.78%, N 11.47%. Found: C 66.25%, H 6.03%, N 11.39%.

**EXAMPLE 40**

**1-[(S)-2-(N-(Methoxycarbonyl)amino)-3-phenylpropionyl-4-[(2-methyl-3-pyridyl)cyanomethyl]-piperazine**

Following the procedure described in preparation 13, but using the product obtained in preparation 6 and (N-methoxycarbonyl)-L-phenylalanine, the title compound of this example was obtained (52% yield).
m.p.: 74.2-78.3 °C;
IR(KBr) n: 3287, 2943, 1712, 1636, 1439 $cm^{-1}$;
$^1H$ NMR (80MHz, $CDCl_3$) $\delta$ (TMS): 8.52 (broad d, J = 4.8Hz, 1H, pyr), 7.76 (broad d, J = 8.2Hz, 1H, pyr), 7.26 (m, 6H, pyr + Ph), 5.55 (m, 1H, NH), 4.77 (m, 2H, CHCN, CHCO), 3.66 (s, 3H, $OCH_3$), 3.51 (m, 2H, pip), 2.97 (m, 4H, $CH_2Ph$, 2H pip), 2.57 (s, 3H, $CH_3$ pyr), 2.36 (m, 4H, pip).
Analysis Calcd. for $C_{23}H_{27}N_5O_3$ . 1/2$H_2O$: C 64.17%, H 6.55%, N 16.26%. Found: C 64.33%, H 6.70%, N 16.19%.

**EXAMPLE 41**

**1-[3-(N-(Methoxycarbonyl)amino)-3-phenylpropionyl]-4-[(2-methyl-3-pyridyl)cyanomethyl]piperazine**

To a solution of 0.5 g (1.3 mmol) of the product obtained in example 14 and 0.29 mL of triethylamine in 10 mL of methylene chloride, were added 0.12 mL (1.5 mmol) of methyl chloroformate and the mixture was stirred at room temperature for 12 h. The resulting solution was diluted with methylene chloride and treated with 0.1 N sodium hydroxide. The organic phase was dried over sodium sulfate and evaporated, affording 0.49 g of a crude that was purified by chromatography on silica gel eluting with ethyl acetate, to give 0.32 g of the product as a white solid (55% yield).
m.p.: 77.3-86.0 °C;
IR(KBr) $\nu$: 3308, 2945, 1712, 1625, 1438, 1247 $cm^{-1}$;
$^1H$ NMR (80MHz, $CD_3Cl$) $\delta$ (TMS): 8.53 (broad d, J = 4 Hz, 1H, pyr), 7.78 (broad d, J = 8.5Hz, 1H, pyr), 7.30 (m, 6H, pyr + Ph), 6.28 (m, 1H, NH), 5.08 (q, 1H, J = 7.2 Hz, CHN), 4.83 (s, 1H, CHCN), 3.64 (s, 3H, $OCH_3$), 3.60 (m, 2H, pip), 3.24 (m, 2H, pip), 2.86 (dd, $J_a$ = 10Hz, $J_b$ = 5.5 Hz, 2H, $CH_2CO$), 2.59 (s, 3H, $CH_3$ pyr), 2.41 (m, 4H, pip).
Analysis Calcd. for $C_{23}H_{27}N_5O_3$. 1/2 $H_2O$: C 64.19%, H 6.55%, N 16.27%. Found: C 63.99%, H 6.55%, N 15.73%.

The following compounds were obtained using a procedure similar to the one described in Example 41.

**EXAMPLE 42**

**1-[3-(N-(Ethoxycarbonyl)amino)-3-phenylpropionyl]-4-[(2-methyl-3-pyridyl)cyanomethyl]piperazine**

m.p.: 72.7-90.5 °C;
Analysis Calcd. for $C_{24}H_{29}N_5O_3$: C 64.84%, H 6.80%, N 15.75%. Found: C 65.05%, H 7.03%, N 15.40%.

**EXAMPLE 43**

**1-[3-(N-(Isobutyloxycarbonyl)amino)-3-phenylpropionyl]-4-[(2-methyl-3-pyridyl)cyanomethyl]-piperazine**

m.p.: 68.3-76.6 ° C;
Analysis Calcd. for $C_{26}H_{33}N_5O_3$. 1/2 $H_2O$: C 66.08%, H 7.25%, N 14.81%. Found: C 65.70%, H 7.39%, N 14.55%.

**EXAMPLE 44**

**1-[3-(N-(Phenoxycarbonyl)amino)-3-phenylpropionyl]-4-[(2-methyl-3-pyridyl)cyanomethyl]piperazine**

m.p.: 95.0-105.7 ° C;
Analysis Calcd. for $C_{28}H_{29}N_5O_3$. 1/2 $H_2O$: C 68.27%, H 6.13%, N 14.21%. Found: C 68.09%, H 6.14%, N 13.92%.

**EXAMPLE 45**

**1-[3-(N-(Benzyloxycarbonyl)amino)-3-phenylpropionyl]-4-[(2-methyl-3-pyridyl)cyanomethyl]-piperazine**

m.p.: 73-76 ° C;
Analysis Calcd. for $C_{29}H_{31}N_5O_3$.1/4$H_2O$: C 69.37%, H 6.31, N 13.94%. Found: C 69.55%, H6.47%, N 13.72%.

**EXAMPLE 46**

**1-[3-(N-(Methoxycarbonyl)amino)-3-(4-(trifluoromethyl)phenyl)propionyl]-4-[(2-methyl-3-pyridyl)-cyanomethyl]piperazine**

m.p.: 90-94 ° C;
Analysis calcd. for $C_{24}H_{26}F_3N_5O_3$.1/2$H_2O$: C 57.84%, H 5.45%, N 14.05%. Found: C 58.03%, H 5.38%, N 13.99%.

**EXAMPLE 47**

**1-[3-(N-(Benzenesulphonyl)amino)-3-phenylpropionyl]-4-[(2-methyl-3-pyridyl)cyanomethyl]-piperazine**

To a solution of 0.5 g (1.3 mmol) of the product obtained in example 14 and 0.29 mL of triethylamine in 10 mL of methylene chloride, were added 0.2 mL (1.5 mmol) of benzenesulfonyl chloride and the mixture was stirred at room temperature for 18 h. The resulting solution was diluted with methylene chloride and treated with 0.1 N sodium hydroxide. The organic phase was dried over sodium sulfate and evaporated, affording 0.8 g of a crude that was purified by chromatography on silica gel, eluting with ethyl acetate, to give 0.57 g of the product as a white solid (88% yield).
m.p.: 85-86°C;
IR(KBr) $\nu$: 2914, 1625, 1441, 1323, 1158 $cm^{-1}$;
$^1H$ NMR (80MHz, $CD_3Cl$) $\delta$ (TMS): 8.50 (broad d, J = 4 Hz, 1H, pyr), 7.69 (m, 2H, Ar, pyr), 7.15 (m, 10H, pyr + Ph), 6.62 (m, 1H, NH), 4.82 (s, 1H, CHCN), 4.71 (t, J = 5.6Hz, 1H, CHNH), 3.48 (m, 2H, pip), 3.17 (m, 2H, pip), 2.75 (t, J = 6.31Hz, 2H, $CH_2CO$), 2.57 (s, 3H, $CH_3$ pyr), 2.31 (m, 4H, pip).
Analysis Calcd. for $C_{27}H_{29}N_5O_3S$.1/2$H_2O$: C 63.28%, H 5.89%, N 13.67%. Found: C 63.61%, H 6.20%, N 13.44%.

**EXAMPLE 48**

**1-[3-(N-(methylsulphonyl)amino)-3-phenylpropionyl]-4-[(2-methyl-3-pyridyl)cyanomethyl]piperazine**

Following the procedure described in example 47, but using methanosulfonyl chloride instead of benzenesulfonyl chloride, the title compound of this example was obtained (63% yield).
m.p.: 79-83 ° C;
$^1$H NMR (80MHz, $CD_3Cl$) δ (TMS): 8.51 (broad d, J = 4 Hz, 1H, pyr), 7.80 (m, 1H, pyr), 7.37 (m, 6H, pyr + Ph), 6.38 (m, 1H, NH), 4.98 (t, J = 5.6Hz, 1H, CHNH), 4.85 (s, 1H, CHCN), 3.52 (m, 2H, pip), 3.27 (m, 2H, pip), 2.88 (m, 2H, $CH_2CO$), 2.70 (s, 3H, $CH_3$ $SO_2$), 2.60 (s, 3H, $CH_3$ pyr), 2.45 (m, 4H, pip). Analysis Calcd. for $C_{22}H_{27}N_5O_3S.1/2H_2O$: C 58.67%, H 6.26%, N 15.55%. Found: C 58.60%, H 6.33%, N 15.19%.

**EXAMPLE 49**

**1-[3-(N-(1-pyrrolidinylcarbonyl)amino)-3-phenylpropionyl]-4-[(2-methyl-3-pyridyl)cyanomethyl]-piperazine**

A mixture of the product obtained in example 44 (0.2 g, 0.4 mmol) and pyrrolidine (1 mL) in acetonitrile (2 mL) was heated at reflux for 5 h. After removing the solvents, the residue was partitioned between 0.1 N NaOH and chloroform. The organic phase was dried over sodium sulfate and concentrated to dryness. The residue was purified by chromatography on silica gel ($CHCl_3$/MeOH 3%) to yield 0.12 g of the title compound of this example as a white solid (66 % yield).
m.p.: 98-101 ° C;
$^1$H NMR (80MHz, $CD_3Cl$) δ (TMS): 8.50 (broad d, J = 4 Hz, 1H, pyr), 7.79 (broad d, J = 8.5Hz, 1H, pyr), 7.31 (m, 6H, pyr + Ph), 6.23 (m, 1H, NH), 5.26 (q, 1H, J = 6.5 Hz, CHN), 4.82 (s, 1H, CHCN), 3.38 (m, 8H, pyrr, pip), 2.88 (dd, $J_a$ = 14Hz, $J_b$ = 5.1 Hz, 2H, $CH_2CO$), 2.60 (s, 3H, $CH_3$ pyr), 2.40 (m, 2H, pip), 1.90 (m, 7H, pyrr, NH, pip).
Analysis Calcd. for $C_{26}H_{32}N_6O_2.7/4H_2O$: C 63.46%, H 7.25%, N 17.08%. Found: C 63.58%, H 6.86%, N 17.27%.

**EXAMPLE 50**

**1-[3-(N-carbamoylamino)-3-phenylpropionyl]-4-[(2-methyl-3-pyridyl)cyanomethyl]piperazine**

To a solution of 0.7 g (1.9 mmol) of the product obtained in example 14 in 15 mL of THF, was added 0.6 mL (3.8 mmol) of trimethylsilylisocyanate and the mixture was heated at 60°C for 2 h. After removing the solvents, the crude was partitioned between 0.1 N sodium hydroxide and chloroform. The organic phase was dried over sodium sulfate and evaporated. The crude obtained was purified by chromatography on silica gel (chloroform : methanol, 3%) to give 0.50 g of the desired product as a white solid (65% yield).
m.p.: 115-118°C;
Analysis Calcd. for $C_{22}H_{26}N_6O_2.5/4H_2O$: C 61.59%, H 6.68%, N 19.58%. Found: C 61.67%, H 6.60%, N 19.20%.

**EXAMPLE 51**

**1-[3-(N-(3-pyridylmethyl)amino)-3-phenylpropionyl]-4-[(2-methyl-3-pyridyl)cyanomethyl]piperazine**

To a mixture of 0.8 g (2.2 mmol) of the product obtained in example 14, 0.57 mL of triethylamine and 50 mg of DMAP in 15 mL of methylene chloride, were added 0.34 g (2.1 mmol) of 3-(chloromethyl)pyridine hydrochloride and the mixture was stirred at room temperature for 12 h. The resulting solution was diluted with methylene chloride and treated with 0.1 N sodium hydroxide. The organic phase was dried over sodium sulfate and evaporated, affording 0.67 g of a crude that was purified by chromatography on silica gel (chloroform : methanol, 3%) to give 0.2 g of the product as a white solid (21% yield).
m.p.: 58-64°C;
IR(KBr) ν: 2912, 1631, 1437 $cm^{-1}$;
$^1$H NMR (80MHz, $CDCl_3$) δ (TMS): 8.50 (m, 3H, pyr), 7.73 (m, 2H, pyr), 7.37 (m, 7H, pyr + Ph), 4.86 (s, 1H, CHCN), 4.17 (t, J = 5.6Hz, 1H, CHNH), 3.62 (s, 2H, $CH_2$pyr), 3.61 (m, 2H, pip), 3.30 (m, 2H, pip), 2.65 (m, 2H, $CH_2CO$), 2.61 (s, 3H, $CH_3$ pyr), 2.48 (m, 4H, pip), 2.21 (s, 1H, NH).

Analysis Calcd. for $C_{27}H_{30}N_6O.3/2H_2O$: C 67.36%, H 6.91%, N 17.45%. Found: C 67.56%, H 6.66%, N 17.49%.

The following compounds were obtained in the same manner as in example 41.

**EXAMPLE 52**

**1-[3-[N-(2-propenyl)amino]-3-phenylpropionyl]-4-[(2-methyl-3-pyridyl)cyanomethyl]piperazine, trihidrochloride**

m.p.: 155-159°C;
Analysis Calcd. for $C_{24}H_{29}N_5O.3HCl.H_2O$: C 54.29%, H 6.45%, N 13.19%. Found: C 54.35%, H 6.61%, N 12.80%.

**EXAMPLE 53**

**1-[3-[N-(2-propynyl)amino]-3-phenylpropionyl]-4-[(2-methyl-3-pyridyl)cyanomethyl]piperazine**

m.p.: 60-63°C;
Analysis Calcd. for $C_{22}H_{27}N_5O.1/2H_2O$: C 68.71%, H 6.97%, N 16.69%. Found: C 69.10%, H 6.83%, N 16.31%.

**EXAMPLE 54**

**1-[3-(N-methylamino)-3-phenylpropionyl]-4-[(2-methyl-3-pyridyl)cyanomethyl]piperazine**

m.p.: 38-42°C;
Analysis Calcd. for $C_{22}H_{27}N_5O.1/2H_2O$: C 68.37%, H 7.30%, N 18.12%. Found: C 68.44%, H 7.25%, N 17.86%.

**EXAMPLE 55**

**1-[3-phenyl-3-(1-pyrrolidinyl)propionyl]-4-[(2-methyl-3-pyridyl)cyanomethyl]piperazine**

Following the procedure described in example 51, but using 1-bromo-4-chloropropane, the title compound of this example was obtained (38% yield).
m.p.: 58-61°C;
Analysis Calcd. for $C_{25}H_{31}N_5O.1/2H_2O$: C 70.42%, H 7.56%, N 16.42%. Found: C 70.30%, H 7.61%, N 16.33%.

**EXAMPLE 56**

**1-[3-(N,N-dibenzylamino)-3-phenylpropionyl]-4-[(2-methyl-3-pyridyl)cyanomethyl]piperazine**

Following the procedure described in preparation 13, but using 3-(N-(dibenzylamino)propionic acid and the product obtained in preparation 6, the title compound of this example was obtained (71% yield).
m.p.: 79-90 ° C;
Analysis Calcd. for $C_{35}H_{37}N_5O.1/2H_2O$: C 76.06%, H 6.91%, N 12.67%. Found: C 75.81%, H 7.06%, N 12.44%.

**EXAMPLE 57**

**1-[3-(N-benzyl-N-methylamino)-3-phenylpropionyl]-4-[(2-methyl-3-pyridyl)cyanomethyl]piperazine, trihydrochloride**

Following the procedure described in preparation 13, but using 3-(N-benzyl-N-methylamino)propionic acid and the product obtained in preparation 6, the title compound of this example was obtained (46% yield). m.p.: 152-155°C;
Analysis Calcd. for $C_{29}H_{33}N_5O.2HCl.2H_2O$: C 60.41%, H 6.82%, N 12.15%. Found: C 60.46%, H 7.16%, N 11.76%.

**EXAMPLE 58**

**1-[3-[N-benzyl-N-(methoxycarbonyl)amino]-3-phenylpropionyl]-4-[(2-methyl-3-pyridyl)cyanomethyl]-piperazine**

Following the procedure described in preparation 13, but using 3-[N-benzyl-N-(methoxycarbonyl)amino]-propionic acid and the product obtained in preparation 6, the title compound of this example was obtained (30% yield).
m.p.: 88-92°C;
Analysis Calcd. for $C_{30}H_{33}N_5O_3$.3/4$H_2O$: C 68.62%, H 6.61%, N 13.34%. Found: C 69.00%, H 6.82%, N 12.98%.

**EXAMPLE 59**

**1-[3-[N-(methoxycarbonyl)-N-methylamino]-3-phenylpropionyl]-4-[(2-methyl-3-pyridyl)cyanomethyl]-piperazine**

Following the procedure described in preparation 13, but using 3-[N-(methoxycarbonyl)-N-methylamino]propionic acid and the product obtained in preparation 6, the title compound of this example was obtained (49% yield).
m.p.: 68-72°C;
$^1$H NMR (80MHz, $CD_3Cl$) δ (TMS): 8.54 (broad d, J= 4 Hz, 1H, pyr), 7.82 (broad d, J= 8.5Hz, 1H, pyr), 7.31 (m, 6H, pyr+Ph), 5.58 (t, 1H, J= 7.6 Hz, CHN), 4.91 (s, 1H, CHCN), 3.72 (s, 3H, $CH_3O$), 3.57 (m, 4H, pip), 2.86 (broad d, J= 6.9Hz, 2H, $CH_2CO$), 2.74 (s, 3H, $NCH_3$), 2.64 (s, 3H, $CH_3$ pyr), 2.56 (m, 4H, pip).
Analysis Calcd. for $C_{24}H_{29}N_5O_2$.3/2$H_2O$: C 64.58%, H 7.22%, N 15.69%. Found: C 64.66%, H 6.98%, N 15.30%.

**EXAMPLE 60**

**1-[3-(N-(3-pyridylcarbonyl)amino)-3-phenylpropionyl]-4-[(2-methyl-3-pyridyl)cyanomethyl]piperazine**

Following the procedure described in preparation 13, but using nicotinic acid and the product obtained in example 14, the title compound of this example was obtained (62% yield).
m.p.: 89-93°C;
$^1$H NMR (80MHz, $CDCl_3$) δ (TMS): 9.11 (m, 1H, pyr), 8.69 (m, 1H, pyr), 8.52 (m, 1H, pyr), 8.17 (broad d, J = 7.7Hz, 1H, pyr), 7.76 (broad d, J = 7.7Hz, 1H, pyr), 7.34 (m, 9H, pyr, Ar, NH), 5.56 (m, 1H, CHNH), 4.82 (s, 1H, CHCN), 3.43 (m, 4H, pip), 3.05 (d, J= 5Hz, 1H, $CH_2CO$), 2.87 (d, J= 5Hz, 1H, $CH_2CO$), 2.58 (s, 3H, $CH_3$ pyr), 2.42 (m, 2H, pip), 1.95 (m, 2H, pip).
Analysis Calcd. for $C_{27}H_{28}N_6O_2$.2$H_2O$: C 64.27%, H 6.39%, N 16.66%. Found: C 64.12%, H 6.00%, N 16.33%.

The following compounds were obtained using a procedure similar to the one described in Example 60.

**EXAMPLE 61**

**1-[3-(N-(2-pyrazinylcarbonyl)amino)-3-phenylpropionyl]-4-[(2-methyl-3-pyridyl)cyanomethyl]-piperazine**

m.p.: 94-97°C;
Analysis Calcd. for $C_{26}H_{27}N_7O_2$.3/4$H_2O$.1/3EtOAc: C 64.07%, H 6.12%, N 19.13%. Found: C 64.37%, H 5.85%, N 19.08%.

**EXAMPLE 62**

**1-[3-(N-(3-furylcarbonyl)amino)-3-phenylpropionyl]-4-[(2-methyl-3-pyridyl)cyanomethyl]piperazine**

m.p.: 74-76°C;
Analysis Calcd. for $C_{26}H_{27}N_5O_3$.1/2$H_2O$.1/3EtOAc: C 66.20%, H 6.22%, N 14.12%. Found: C 66.42%, H 6.31%, N 13.85%.

**EXAMPLE 63**

**1-[3-[N-((2-hydroxy-3-pyridyl)carbonyl)amino]-3-phenylpropionyl]-4-[(2-methyl-3-pyridyl)-cyanomethyl]piperazine**

m.p.: 129-134°C;
Analysis Calcd. for $C_{27}H_{28}N_6O_3.3/2H_2O.1/3EtOAc$: C 63.94%, H 6.40%, N 15.97%. Found: C 63.55%, H 6.12%, N 15.58%.

**EXAMPLE 64**

**1-[3-[N-((3-hydroxy-2-pyridyl)carbonyl)amino]-3-phenylpropionyl]-4-[(2-methyl-3-pyridyl)-cyanomethyl]piperazine**

m.p.: 89-92°C;
Analysis Calcd. for $C_{27}H_{28}N_6O_3.5/4H_2O.1/3EtOAc$: C 64.49%, H 6.39%, N 16.11%. Found: C 64.77%, H 6.21%, N 16.07%.

**EXAMPLE 65**

**1-[3-Succinimido-3-phenylpropionyl]-4-[(2-methyl-3-pyridyl)cyanomethyl]piperazine**

A mixture of 0.3 g (0.85 mmol) of the product obtained in example 14 and 0.12 g of succinic anhydride in 25 mL of chloroform was heated at 70°C for 18 h. After removing the solvents, 20 mL of acetic anhydride were added and the mixture was heated at 60°C overnight. The solvent was evaporated, affording a crude that was purified by chromatography on silica gel (ethyl acetate-methanol, 4%) to give 0.38 g of the title compound of this example as a white solid (90% yield).
m.p.: 90-95°C;
$^1$H NMR (80MHz, $CDCl_3$) δ (TMS): 8.52 (broad d, J= 4.8Hz, 1H, pyr), 7.79 (broad d, J= 8Hz, 1H, pyr), 7.43 (s, 6H, pyr, Ph), 5.73 (dd, J= 11Hz, J = 5.1Hz, 1H, CHNH), 4.90 (s, 1H, CHCN), 3.87 (m, 1H, $CH_2CO$), 3.52 (m, 4H, pip), 2.90 (dd, J= 15.3Hz, J = 4.6Hz, 1H, $\overline{CH_2}CO$), 2.64 (s, 3H, $CH_3$ pyr), 2.51 (m, 8H, pip, succ).
Analysis Calcd. for $C_{25}H_{27}N_5O_3.H_2O$: C 64.78%, H 6.31%, N 15.11%. Found: C 64.53%, H 6.02%, N 14.73%.

**EXAMPLE 66**

**1-[3-(2-oxo-1-pyrrolidinyl)-3-phenylpropionyl]-4-[(2-methyl-3-pyridyl)cyanomethyl]piperazine**

Following the procedure described in preparation 13, but using 3-(2-oxo-1-pyrrolidinyl)-3-phenylpropionic acid and the product obtained in preparation 6, the title compound of this example was obtained (80% yield).
m.p.: 172-175°C;
Analysis Calcd. for $C_{25}H_{29}N_5O_2.1/2H_2O$: C 68.18%, H 6.86%, N 15.90%. Found: C 68.29%, H 7.01%, N 15.98%.

**EXAMPLE 67**

**1-[3-(1,1-dioxo-2-isothiazolidinyl)-3-phenylpropionyl]-4-[(2-methyl-3-pyridyl)cyanomethyl]piperazine**

Following the procedure described in preparation 13, but using 3-(1,1-dioxo-2-isothiazolidinyl)-3-phenylpropionic acid and the product obtained in preparation 6, the title compound of this example was obtained (62% yield).
m.p.: 85-87°C;
Analysis Calcd. for $C_{24}H_{29}N_5O_3S.3H_2O$: C 55.26%, H 6.76%, N 13.43%. Found: C 55.09%, H 6.37%, N 13.09%.

**EXAMPLE 68**

**1-[3-(2-oxo-3-oxazolidinyl)-3-phenylpropionyl]-4-[(2-methyl-3-pyridyl)cyanomethyl]piperazine**

Following the procedure described in preparation 13, but using the acid obtained in preparation 34 and the product obtained in preparation 6, the title compound of this example was obtained (65% yield).
m.p.: 87-90°C;
$^1$H NMR (80MHz, $CDCl_3$) δ (TMS): 8.52 (broad d, J= 4.8Hz, 1H, pyr), 7.84 (broad d, J= 9Hz, 1H, pyr), 7.36 (s, 6H, pyr, Ph), 5.10 (m, 1H, CHNH), 4.90 (s, 1H, CHCN), 4.27 (t, J = 7.8Hz, 2H, oxa), 3.58 (m, 7H, pip, oxa, $CH_2CO$), 2.87 (dd, J= 15.3Hz, J = 4.3Hz, 1H, $CH_2CO$), 2.64 (s, 3H, $CH_3$ pyr), 2.54 (m, 4H, pip).
Analysis Calcd. for $C_{24}H_{27}N_5O_3$.3/2$H_2O$: C 62.61%, H 6.56%, N 15.21%. Found: C 62.69%, H 6.43%, N 15.02%.

**EXAMPLE 69**

**1-[3-(1-pyrrolyl)-3-phenylpropionyl]-4-[(2-methyl-3-pyridyl)cyanomethyl]piperazine**

Following the procedure described in preparation 13, but using the acid obtained in preparation 33 and the product obtained in preparation 6, the title compound of this example was obtained (62% yield).
m.p.: 71-72°C;
Analysis Calcd. for $C_{25}H_{27}N_5O$.3/4$H_2O$: C 70.32%, H 6.72%, N 16.40%. Found: C 70.53%, H 6.84%, N 16.29%.

**EXAMPLE 70**

**1-(Cis-3-phenyl-3-(trifluoromethyl)propenoyl)-4-[(2-methyl-3-pyridyl)cyanomethyl]piperazine**

Following the procedure described in preparation 13, but using the acid obtained in preparation 31c and the product obtained in preparation 6, the title compound of this example was obtained (50% yield).
m.p.: 212-213 ° C;
Analysis Calcd. for $C_{25}H_{21}F_3N_4O$.1/4$H_2O$: C 63.07%, H 5.17%, N 13.37%. Found: C 63.12%, H 5.23%, N 13.12%.

**EXAMPLE 71**

**1-(3-Phenyl-3-(trifluoromethyl)propionyl)-4-[(2-methyl-3-pyridyl)cyanomethyl]piperazine**

Following the procedure described in preparation 13, but using the acid obtained in preparation 32 and the product obtained in preparation 6, the title compound of this example was obtained (82% yield).
m.p.: 58-62°C;
Analysis Calcd. for $C_{22}H_{23}F_3N_4O$.1/2$H_2O$: C 62.13%, H 5.68%, N 13.17%. Found: C 63.13%, H 5.50%, N 13.03%.

**EXAMPLE 72**

**1-(trans-3-Phenyl-3-(trifluoromethyl)propenoyl)-4-[(2-methyl-3-pyridyl)cyanomethyl]piperazine**

Following the procedure described in preparation 13, but using the acid obtained in preparation 31b and the product obtained in preparation 6, the title compound of this example was obtained (74% yield).
m.p.: 152-155 °C;
Analysis Calcd. for $C_{22}H_{21}F_3N_4O$: C 63.76%, H 5.11%, N 13.52%. Found: C 64.13%, H 5.50%, N 13.24%.

**EXAMPLE 73**

**1-[3-(N-(3,5-di-*tert*-butyl-4-hydroxybenzoyl)amino)-3-phenylpropionyl]-4-[(2-methyl-3-pyridyl)-cyanomethyl]piperazine**

Following the procedure described in preparation 13, but using 3-(N-(3,5-di-*tert*--butyl-4-hydroxybenzoyl)amino)-3-phenylpropionic acid and the product obtained in preparation 6, the title compound of this example was obtained (53% yield)
m.p.: 120-121°C;
Analysis Calcd. for $C_{36}H_{45}N_5O_3.5/4H_2O$: C 69.93%, H 7.73%, N 11.32%. Found: C 69.61%, H 7.39%, N 11.28%.

**EXAMPLE 74**

**1-[3-(N-phenylamino)-3-phenylpropionyl]-4-[(2-methyl-3-pyridyl)cyanomethyl]piperazine**

Following the procedure described in preparation 13, but using the acid obtained in preparation 35b and the product obtained in preparation 6, the title compound of this example was obtained (46% yield).
m.p.: 158-162;
$^1H$ NMR (80MHz, $CDCl_3$) $\delta$ (TMS): 8.50 (broad d, J= 4.8Hz, 1H, pyr), 7.74 (broad d, J= 8.9Hz, 1H, pyr), 7.18 (s, 8H, pyr, Ph), 6.57 (m, 3H, Ph), 4.80 (t, J = 6Hz, 1H, CHNH), 4.80 (s, 1H, CHCN), 3.50 (m, 2H, pip), 3.14 (m, 2H, pip), 2.82 (dd, J= 4.8Hz, J = 1.3Hz, 2H, $CH_2\overline{CO}$), 2.57 (s, 3H, $CH_3$ pyr), 2.38 (m, 2H, pip), 2.16 (m, 2H, pip).
Analysis Calcd. for $C_{27}H_{29}N_5O.1/2H_2O$: C 72.32%, H 6.74%, N 15.62%. Found: C 72.19%, H 7.00%, N 15.33%.

**EXAMPLE 75**

**1-[3-(N-acetyl-N-hydroxyamino)-3-phenylpropionyl]-4-[(2-methyl-3-pyridyl)cyanomethyl]piperazine**

Following the procedure described in preparation 13, but using the acid obtained in preparation 36b and the product obtained in preparation 6, the title compound of this example was obtained (58% yield).
m.p.: 83-87°C;
IR(KBr) $\nu$: 3422, 3156, 2916, 1641, 1437, 1239 $cm^{-1}$;
$^1H$ NMR (80MHz, $CDCl_3$) $\delta$ (TMS): 8.68 (m, 1H, OH), 8.52 (broad d, J= 4.8Hz, 1H, pyr), 7.80 (broad d, J= 8Hz, 1H, pyr), 7.34 (s, 6H, pyr, 5H Ph), 5.78 (m, 1H, CHNH), 4.90 (s, 1H, CHCN), 3.58 (complex signal, 4H, pip), 3.01 (m, 2H, $CH_2CO$), 2.63 (s, 3H, $CH_3$ pyr), 2.58 (m, 4H, pip), 2.16 (s, 3H, $CH_3CO$).
Analysis Calcd. for $C_{23}H_{27}N_5O_3 \cdot H_2O$: C 62.85%, H 6.65%, N 15.93%. Found: C 62.95%, H 6.51%, N 15.54%.

**EXAMPLE 76**

**1-[3-(N-carbamoyl-N-hydroxyamino)-3-phenylpropionyl]-4-[(2-methyl-3-pyridyl)cyanomethyl]-piperazine**

Following the procedure described in preparation 13, but using the acid obtained in preparation 37 and the product obtained in preparation 6, the title compound of this example was obtained (40% yield).
m.p.: 124-125°C;
$^1H$ NMR (80MHz, $CDCl_3$) $\delta$ (TMS): 8.97 (m, 1H, OH), 8.46 (broad d, J= 4.8Hz, 1H, pyr), 7.76 (broad d, J= 8Hz, 1H, pyr), 7.26 (s, 6H, pyr, 5H Ph), 5.69 (m, 1H, CHNH), 5.48 (m, 2H, $NH_2$), 4.88 (s, 1H, CHCN), 3.53 (complex signal, 4H, pip), 3.01 (m, 2H, $CH_2CO$), 2.59 (s, 3H, $CH_3$ pyr), 2.51 (m, 4H, pip).
Analysis Calcd. for $C_{22}H_{26}N_6O_3.1/2H_2O$: C 61.26%, H 6.30%, N 19.48%. Found: C 61.56%, H 6.07%, N 19.47%.

**EXAMPLE 77**

**1-[3-(N-methylcarbamoyl-N-hydroxyamino)-3-phenylpropionyl]-4-[(2-methyl-3-pyridyl)cyanomethyl]-piperazine**

Following the procedure described in preparation 13, but using the acid obtained in preparation 38 and the product obtained in preparation 6, the title compound of this example was obtained (38% yield).
m.p.: 83-89°C;
Analysis Calcd. for $C_{23}H_{28}N_6O_3.H_2O$: C 60.78%, H 6.65%, N 18.49%. Found: C 61.00%, H 6.26%, N 18.11%.

**EXAMPLE 78**

**1-[3-(N-(N'-hydroxy-N'-methylcarbamoyl)amino)-3-phenylpropionyl]-4-[(2-methyl-3-pyridyl)-cyanomethyl]piperazine**

Following the procedure described in preparation 13, but using the acid obtained in preparation 39b and the product obtained in preparation 6, the title compound of this example was obtained (57% yield).
m.p.: 143-147°C;
Analysis Calcd. for $C_{23}H_{28}N_6O_3.H_2O$: C 60.78%, H 6.65%, N 18.49%. Found: C 60.40%, H 6.28%, N 18.10%.

**Claims**

**1.** A compound of formula **I**:

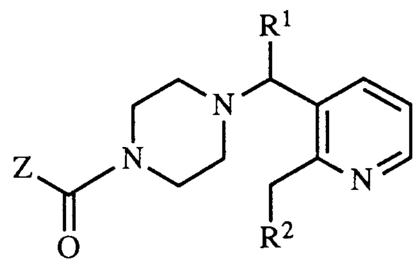

I

wherein:
**R¹** is -CN, $-CO_2R^3$, $-CON(R^3)_2$, $-COR^3$, $-C{\equiv}CR^3$ or $-(R^3)C=N-OR^3$, wherein $R^3$ is hydrogen or a $(C_1-C_6)$ alkyl group;
**$R^2$** is hydrogen or a $(C_1-C_3)$ alkyl group;
**Z** represents a group of formula:

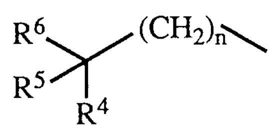

wherein

n is 0, 1 or 2;

$R^4$ represents hydrogen, $(C_1-C_6)$ alkyl, $Ar^1$ or Het; $Ar^1$ is a phenyl group optionally substituted by one or more groups chosen from halogen, trifluoromethyl, $(C_1-C_6)$ alkyl, $(C_1-C_6)$ alkoxy or hydroxy and Het is any radical from a 5- or 6-membered aromatic heterocycle with one ring heteroatom chosen from N, O or S, or with two ring heteroatoms chosen from the pairs N/N, N/O or N/S and which may be optionally substituted by a group chosen from halogen, $(C_1-C_6)$ alkyl, $(C_1-C_6)$ alkoxy or hydroxy;

$R^5$ represents hydrogen, $(C_1-C_6)$ alkyl, $Ar^1$, $Ar^2$, trifluoromethyl or hydroxy-$(C_1-C_6)$ alkyl such that

a) when $R^5$ represents hydrogen, ($C_1$-$C_6$) alkyl or $Ar^1$, $R^6$ represents hydrogen, ($C_1$-$C_6$) alkyl, $OR^7$, $Ar^3S(=O)_m^-$, halogen, $R^3OC(=O)$- or a group $NR^8R^9$, where:

$R^7$ is hydrogen, ($C_1$-$C_6$) alkyl, $Ar^3$ or a group $(Ar^1)_2C=N$-;

m is 0, 1 or 2;

$R^8$ represents hydrogen, ($C_1$-$C_6$) alkyl, $Ar^1$, $Ar^2$, $Ar^3$ or hydroxy; $R^9$ represents hydrogen, ($C_1$-$C_6$) alkyl, ($C_1$-$C_6$) alkenyl, ($C_1$-$C_6$) alkynyl, $Ar^1$, $Ar^2$, $(R^3)_2NC(=O)$-, $HO(R^3)NC(=O)$-, $R^{10}OC(=O)$-, $Ar^1OC(=O)$-, $Ar^2OC(=O)$-, $Ar^1C(=O)$-, Het-($C_1$-$C_6$) alkyl, HetC(=O)-, $R^3C(=O)$-, fluorenyl, $R^{10}SO_2$-, $Ar^1SO_2$- or $Ar^2SO_2$-, or the group $NR^8R^9$ forms a pyrrol, imidazol, or a saturated 5- or 6-membered heterocycle which may contain a second ring heteroatom selected from O, S, N and may be optionally substituted by one or two oxo groups and/or by a group $R^{10}$;

$Ar^2$ is a ($C_1$-$C_6$) alkyl group substituted by one $Ar^1$ group;

$Ar^3$ is a ($C_1$-$C_6$) alkyl group substituted by two $Ar^1$ that can be equal or different;

$R^{10}$ is a ($C_1$-$C_6$) alkyl group;

b) when $R^5$ represents trifluoromethyl, $R^6$ represents hydrogen or $OR^3$, where $R^3$ has the above mentioned meaning;

c) when $R^5$ represents hydroxy-($C_1$-$C_6$) alkyl or $Ar^2$, $R^6$ represents a group $NR^8R^9$, where $R^8$ and $R^9$ have the above mentioned meaning; or **Z** represents a group of formula

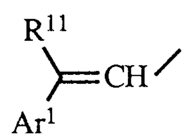

wherein $R^{11}$ represents hydrogen, $Ar^1$ or trifluoromethyl; and the salts thereof.

2. A compound according to Claim 1 in which
   **R¹** is -CN;
   **R²** is hydrogen; and
   Z has the previously defined meaning.

3. A compound according to Claims 1 and 2 in which
   **R¹** is CN;
   **R²** is hydrogen; and
   **Z** represents a group of formula

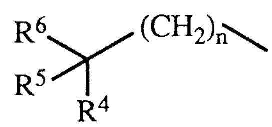

wherein

n is 1;

$R^4$ represents $Ar^1$ or Het, wherein $Ar^1$ and Het have the previously defined meaning;

$R^5$ represents ($C_1$-$C_6$) alkyl or $Ar^1$; and

$R^6$ represents hydrogen, ($C_1$-$C_6$) alkyl, $OR^3$ or $NH_2$, where $R^3$ has the previously defined meaning.

4. A compound according to Claims 1 and 2 in which
   **R¹** is CN;
   **R²** is hydrogen; and
   **Z** represents a group of formula

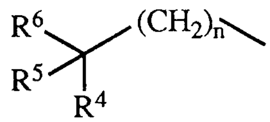

wherein
n is 1;
$R^4$ represents $Ar^1$ or Het, wherein $Ar^1$ and Het have the previously defined meaning;
$R^5$ represents trifluoromethyl; and
$R^6$ represents hydrogen or $OR^3$, where $R^3$ has the previously defined meaning.

**5.** A compound according to Claims 1 and 2 in which
**$R^1$** is CN;
**$R^2$** is hydrogen; and
**Z** represents a group of formula

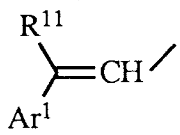

wherein $R^{11}$ represents $Ar^1$ or trifluoromethyl, where $Ar^1$ has the previously defined meaning.

**6.** A compound according to Claims 1 and 2 in which
**$R^1$** is CN;
**$R^2$** is hydrogen;
**Z** represents a group of formula

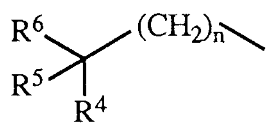

wherein
n is 0 or 1;
$R^4$ and $R^5$ represent each one independently hydrogen or $(C_1\text{-}C_6)$ alkyl; and
$R^6$ represents a group $NR^8R^9$, where:
$R^8$ represents $Ar^3$, being $Ar^3$ as above defined;
$R^9$ represents hydrogen, $(C_1\text{-}C_6)$ alkyl, $(R^3)_2NC(=O)$-,
$R^{10}OC(=O)$-, $R^3C(=O)$- or $R^{10}SO_2$-, being $R^3$ and $R^{10}$ as above defined.

**7.** A compound according to Claims 1 and 2 in which
**$R^1$** is CN;
**$R^2$** is hydrogen;
**Z** represents a group of formula

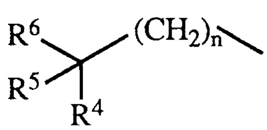

wherein
n is 1;
$R^4$ represents $Ar^1$ or Het, wherein $Ar^1$ and Het have the previously defined meaning;
$R^5$ represents hydrogen or $(C_1\text{-}C_6)$ alkyl; and
$R^6$ represents a group $NR^8R^9$, where $R^8$ represents hydrogen, $(C_1\text{-}C_6)$ alkyl, $Ar^1$ or hydroxy; $R^9$ represents hydrogen, $(C_1\text{-}C_6)$ alkyl, $(C_1\text{-}C_6)$ alkenyl, $(C_1\text{-}C_6)$alkynyl, $Ar^2$, $(R^3)_2NC(=O)$-, $HO(R^3)NC(=O)$-, $R^{10}OC(=O)$-, $Ar^1OC(=O)$-, $Ar^2OC(=O)$-, $Ar^1C(=O)$-, Het-$(C_1\text{-}C_6)$ alkyl, $HetC(=O)$-, $R^3C(=O)$-, $R^{10}SO_2$-, $Ar^1SO_2$- or $Ar^2SO_2$-, or the group $NR^8R^9$ forms an heterocycle of the formula (i), (ii), (iii), (iv),

(v), (vi) or (vii):

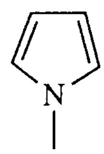

(i)

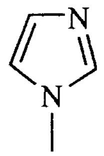

(ii)

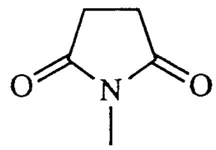

(iii)

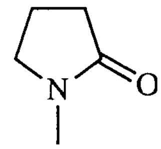

(iv)

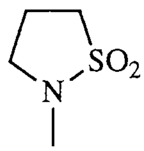

(v)

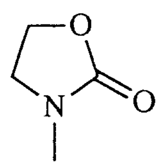

(vi)

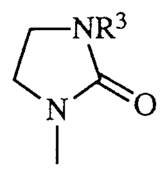

(vii)

wherein $R^3$, $R^{10}$, $Ar^1$, $Ar^2$ and Het have the previously defined meaning.

**8.**

a) 1-(3,3-Diphenyl-3-hydroxypropionyl)-4-[(2-methyl-3-pyridyl)cyanomethyl]piperazine and the salts thereof;

b) 1-(3-Phenyl-3-hydroxy-3-(trifluoromethyl)propionyl)-4-[(2-methyl-3-pyridyl)cyanomethyl]piperazine and the salts thereof;

c) 1-[3-(N-Benzylamino)-3-phenylpropionyl]-4-[(2-methyl-3-pyridyl)cyanomethyl]piperazine and the salts thereof;

d) 1-[3-(N-acetylamino)-3-phenylpropionyl]-4-[(2-methyl-3-pyridyl)cyanomethyl]piperazine and the salts thereof;

e) 1-[N-(Diphenylmethyl)aminoacetyl]-4-[(2-methyl-3-pyridyl)cyanomethyl]piperazine and the salts thereof;

f) 1-[3-(N-(Methoxycarbonyl)amino)-3-phenylpropionyl]-4-[(2-methyl-3-pyridyl)cyanomethyl]piperazine and the salts thereof;

g) 1-[3-(N-(Phenoxycarbonyl)amino)-3-phenylpropionyl]-4-[(2-methyl-3-pyridyl)cyanomethyl]-piperazine and the salts thereof;

h) 1-[3-(2-oxo-3-oxazolidinyl)-3-phenylpropionyl]-4-[(2-methyl-3-pyridyl)cyanomethyl]piperazine and the salts thereof;

i) 1-[3-(N-phenylamino)-3-phenylpropionyl]-4-[(2-methyl-3-pyridyl)cyanomethyl]piperazine and the salts thereof;

j) 1-[3-(N-acetyl-N-hydroxyamino)-3-phenylpropionyl]-4-[(2-methyl-3-pyridyl)cyanomethyl]piperazine and the salts thereof;

k) 1-[3-(N-carbamoyl-N-hydroxyamino)-3-phenylpropionyl]-4-[(2-methyl-3-pyridyl)cyanomethyl]-piperazine and the salts thereof.

**9.** A process for preparing a compound according to Claim 1 which comprises treating a compound of formula **II**

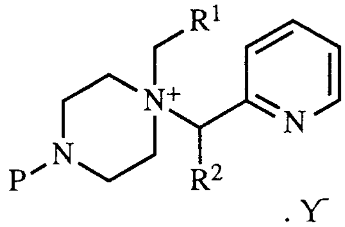

II

(wherein $R^1$ and $R^2$ have the previously defined meaning, P is an amine protecting group or ZCO, being Z as described above, and Y is an anion, such as chloride, bromide, iodide, alkylsulfonate or arylsulfonate) with a base, such as 1,8-diazabicyclo[5.4.0]undec-7-ene in acetonitrile, to give a compound of formula **III**

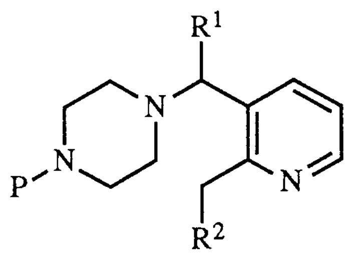

III

wherein $R^1$ and $R^2$ have the previously defined meaning, and P is ZCO (in which case **III** is **I**) or alternatively P is an amine protecting group, in which case it is deprotected to give the corresponding amine of formula **IV**

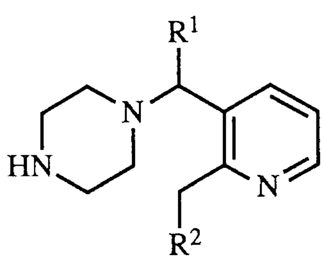

IV

(wherein $R^1$ and $R^2$ have the previously defined meaning), and then reacting **IV** with an acid **ZCOOH** or an equivalent acylating reagent, to give a compound of formula **I**

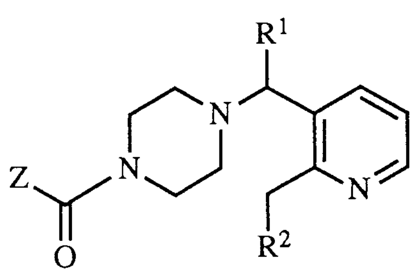

I

(wherein Z, $R^1$ and $R^2$ have the previously defined meaning); and optionally, when in a compound of formula **I**, Z represents a group of formula

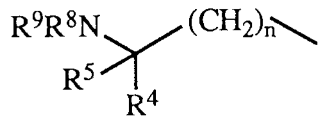

wherein $R^4$, $R^5$, $R^8$, $R^9$ and n have the above mentioned meaning, transforming the groups $R^8$ and/or $R^9$ in a compound of formula **I** into other groups $R^8$ and/or $R^9$.

**10.** A pharmaceutical composition comprising an effective amount of at least one compound of formula **I** or a pharmaceutically acceptable salt thereof in admixture with a pharmaceutically acceptable excipient.

**11.** The use of at least one compound of formula **I** or a pharmaceutically acceptable salt thereof for the manufacture of a medicament for the treatment or prophylaxys of PAF-mediated illnesses.

**Claims for the following Contracting State : GR**

**1.** A process for preparing a compound of formula **I**:

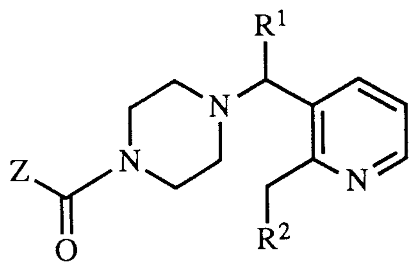

**I**

wherein:
**$R^1$** is -CN, $-CO_2R^3$, $-CON(R^3)_2$, $-COR^3$, $-C{\equiv}CR^3$ or $-(R^3)C=N-OR^3$, wherein $R^3$ is hydrogen or a $(C_1-C_6)$ alkyl group;
**$R^2$** is hydrogen or a $(C_1-C_3)$ alkyl group;
**Z** represents a group of formula:

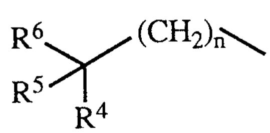

wherein
n is 0, 1 or 2;
$R^4$ represents hydrogen, $(C_1-C_6)$ alkyl, $Ar^1$ or Het; $Ar^1$ is a phenyl group optionally substituted by one or more groups chosen from halogen, trifluoromethyl, $(C_1-C_6)$ alkyl, $(C_1-C_6)$ alkoxy or hydroxy and Het is any radical from a 5- or 6-membered aromatic heterocycle with one ring heteroatom chosen from N, O or S, or with two ring heteroatoms chosen from the pairs N/N, N/O or N/S and which may be optionally substituted by a group chosen from halogen, $(C_1-C_6)$ alkyl, $(C_1-C_6)$ alkoxy or hydroxy;
$R^5$ represents hydrogen, $(C_1-C_6)$ alkyl, $Ar^1$, $Ar^2$, trifluoromethyl or hydroxy-$(C_1-C_6)$ alkyl such that
a) when $R^5$ represents hydrogen, $(C_1-C_6)$ alkyl or $Ar^1$, $R^6$ represents hydrogen, $(C_1-C_6)$ alkyl, $OR^7$, $Ar^3S(=O)_m-$, halogen, $R^3OC(=O)-$ or a group $NR^8R^9$, where:
$R^7$ is hydrogen, $(C_1-C_6)$ alkyl, $Ar^3$ or a group $(Ar^1)_2C=N-$;
m is 0, 1 or 2;
$R^8$ represents hydrogen, $(C_1-C_6)$ alkyl, $Ar^1$, $Ar^2$, $Ar^3$ or hydroxy; $R^9$ represents hydrogen, $(C_1-C_6)$ alkyl, $(C_1-C_6)$ alkenyl, $(C_1-C_6)$ alkynyl, $Ar^1$, $Ar^2$, $(R^3)_2NC(=O)-$, $HO(R^3)NC(=O)-$, $R^{10}OC(=O)-$,

$Ar^1OC(=O)$-, $Ar^2OC(=O)$-, $Ar^1C(=O)$-, Het-($C_1$-$C_6$) alkyl, HetC(=O)-, $R^3C(=O)$-, fluorenyl, $R^{10}SO_2$-, $Ar^1SO_2$- or $Ar^2SO_2$-, or the group $NR^8R^9$ forms a pyrrol, imidazol, or a saturated 5- or 6-membered heterocycle which may contain a second ring heteroatom selected from O, S, N and may be optionally substituted by one or two oxo groups and/or by a group $R^{10}$;

$Ar^2$ is a ($C_1$-$C_6$) alkyl group substituted by one $Ar^1$ group;

$Ar^3$ is a ($C_1$-$C_6$) alkyl group substituted by two $Ar^1$ that can be equal or different;

$R^{10}$ is a ($C_1$-$C_6$) alkyl group;

b) when $R^5$ represents trifluoromethyl, $R^6$ represents hydrogen or $OR^3$, where $R^3$ has the above mentioned meaning;

c) when $R^5$ represents hydroxy-($C_1$-$C_6$) alkyl or $Ar^2$, $R^6$ represents a group $NR^8R^9$, where $R^8$ and $R^9$ have the above mentioned meaning;

or **Z** represents a group of formula

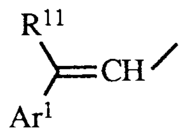

wherein $R^{11}$ represents hydrogen, $Ar^1$ or trifluoromethyl;
and the salts thereof,
**which comprises** treating a compound of formula **II**

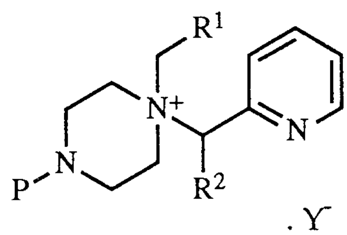

**II**

(wherein $R^1$ and $R^2$ have the previously defined meaning, P is an amine protecting group or ZCO, being Z as described above, and Y is an anion, such as chloride, bromide, iodide, alkylsulfonate or arylsulfonate) with a base, such as 1,8-diazabicyclo[5.4.0]undec-7-ene in acetonitrile, to give a compound of formula **III**

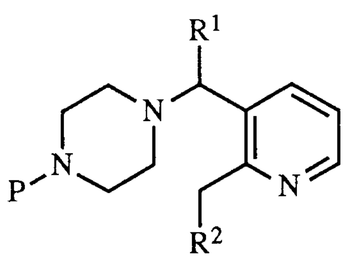

**III**

wherein $R^1$ and $R^2$ have the previously defined meaning, and P is ZCO (in which case **III** is **I**) or alternatively P is an amine protecting group, in which case it is deprotected to give the corresponding amine of formula **IV**

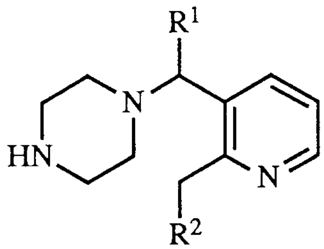

IV

(wherein $R^1$ and $R^2$ have the previously defined meaning), and then reacting **IV** with an acid **ZCOOH** or an equivalent acylating reagent, to give a compound of formula **I**

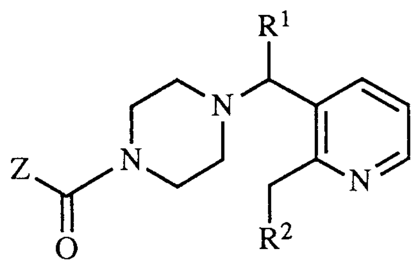

I

(wherein Z, $R^1$ and $R^2$ have the previously defined meaning); and optionally, when in a compound of formula **I**, Z represents a group of formula

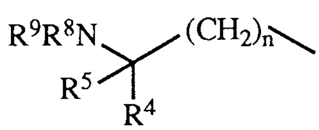

wherein $R^4$, $R^5$, $R^8$, $R^9$ and n have the above mentioned meaning, transforming the groups $R^8$ and/or $R^9$ in a compound of formula **I** into other groups $R^8$ and/or $R^9$.

**2.** A process according to Claim 1 in which the reagents and reaction conditions are so chosen as to prepare a compound of formula **I** wherein:
**$R^1$** is -CN;
**$R^2$** is hydrogen; and
Z has the previously defined meaning.

**3.** A process according to Claim 1 in which the reagents and reaction conditions are so chosen as to prepare a compound of formula **I** wherein:
**$R^1$** is CN;
**$R^2$** is hydrogen; and
**Z** represents a group of formula

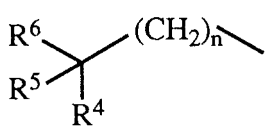

wherein
n is 1;
$R^4$ represents $Ar^1$ or Het, wherein $Ar^1$ and Het have the previously defined meaning;
$R^5$ represents ($C_1$-$C_6$) alkyl or $Ar^1$; and
$R^6$ represents hydrogen, ($C_1$-$C_6$) alkyl, $OR^3$ or $NH_2$, where $R^3$ has the previously defined meaning.

**4.** A process according to Claim 1 in which the reagents and reaction conditions are so chosen as to prepare a compound of formula **I** wherein:
**$R^1$** is CN;
**$R^2$** is hydrogen; and
**Z** represents a group of formula

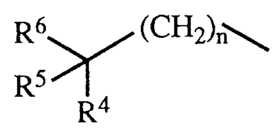

wherein
n is 1;
$R^4$ represents $Ar^1$ or Het, wherein $Ar^1$ and Het have the previously defined meaning;
$R^5$ represents trifluoromethyl; and
$R^6$ represents hydrogen or $OR^3$, where $R^3$ has the previously defined meaning.

**5.** A process according to Claim 1 in which the reagents and reaction conditions are so chosen as to prepare a compound of formula **I** wherein:
**$R^1$** is CN;
**$R^2$** is hydrogen; and
**Z** represents a group of formula

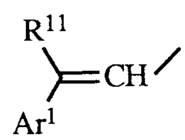

wherein $R^{11}$ represents $Ar^1$ or trifluoromethyl, where $Ar^1$ has the previously defined meaning.

**6.** A process according to Claim 1 in which the reagents and reaction conditions are so chosen as to prepare a compound of formula **I** wherein:
**$R^1$** is CN;
**$R^2$** is hydrogen;
**Z** represents a group of formula

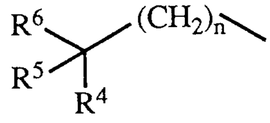

wherein
n is 0 or 1;
$R^4$ and $R^5$ represent each one independently hydrogen or ($C_1$-$C_6$) alkyl; and
$R^6$ represents a group $NR^8R^9$, where:
$R^8$ represents $Ar^3$, being $Ar^3$ as above defined;
$R^9$ represents hydrogen, ($C_1$-$C_6$) alkyl, $(R^3)_2NC(=O)$-,
$R^{10}OC(=O)$-, $R^3C(=O)$- or $R^{10}SO_2$-, being $R^3$ and $R^{10}$ as above defined.

**7.** A process according to Claim 1 in which the reagents and reaction conditions are so chosen as to prepare a compound of formula **I** wherein:

**R¹** is CN;

**R²** is hydrogen;

**Z** represents a group of formula

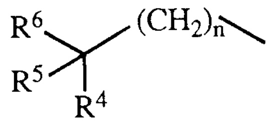

wherein

n is 1;

$R^4$ represents $Ar^1$ or Het, wherein $Ar^1$ and Het have the previously defined meaning;

$R^5$ represents hydrogen or $(C_1\text{-}C_6)$ alkyl; and

$R^6$ represents a group $NR^8R^9$, where $R^8$ represents hydrogen, $(C_1\text{-}C_6)$ alkyl, $Ar^1$ or hydroxy; $R^9$ represents hydrogen, $(C_1\text{-}C_6)$ alkyl, $(C_1\text{-}C_6)$ alkenyl, $(C_1\text{-}C_6)$alkynyl, $Ar^2$, $(R^3)_2NC(=O)$-, $HO(R^3)NC(=O)$-, $R^{10}OC(=O)$-, $Ar^1OC(=O)$-, $Ar^2OC(=O)$-, $Ar^1C(=O)$-, Het-$(C_1\text{-}C_6)$ alkyl, HetC(=O)-, $R^3C(=O)$-, $R^{10}SO_2$-, $Ar^1SO_2$- or $Ar^2SO_2$-, or the group $NR^8R^9$ forms an heterocycle of the formula (i), (ii), (iii), (iv), (v), (vi) or (vii):

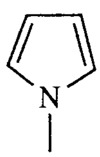

(i)

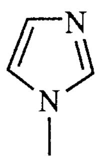

(ii)

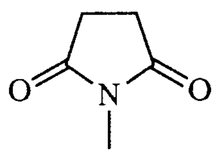

(iii)

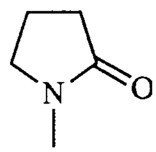

(iv)

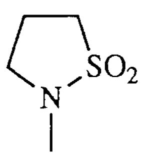

(v)

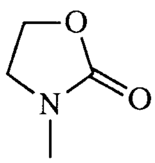

(vi)

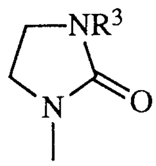

(vii)

wherein $R^3$, $R^{10}$, $Ar^1$, $Ar^2$ and Het have the previously defined meaning.

**8.**

a) A process according to Claim 1 in which the reagents and reaction conditions are so chosen as to prepare 1-(3,3-Diphenyl-3-hydroxypropionyl)-4-[(2-methyl-3-pyridyl)cyanomethyl]piperazine and the salts thereof;

b) A process according to Claim 1 in which the reagents and reaction conditions are so chosen as to prepare 1-(3-Phenyl-3-hydroxy-3-(trifluoromethyl)propionyl)-4-[(2-methyl-3-pyridyl)cyanomethyl]-piperazine and the salts thereof;

c) A process according to Claim 1 in which the reagents and reaction conditions are so chosen as to prepare 1-[3-(N-Benzylamino)-3-phenylpropionyl]-4-[(2-methyl-3-pyridyl)cyanomethyl]piperazine and the salts thereof;

d) A process according to Claim 1 in which the reagents and reaction conditions are so chosen as to prepare 1-[3-(N-acetylamino)-3-phenylpropionyl]-4-[(2-methyl-3-pyridyl)cyanomethyl]piperazine and the salts thereof;

e) A process according to Claim 1 in which the reagents and reaction conditions are so chosen as to prepare 1-[N-(Diphenylmethyl)aminoacetyl]-4-[(2-methyl-3-pyridyl)cyanomethyl]piperazine and the

salts thereof;

f) A process according to Claim 1 in which the reagents and reaction conditions are so chosen as to prepare 1-[3-(N-(Methoxycarbonyl)amino)-3-phenylpropionyl]-4-[(2-methyl-3-pyridyl)cyanomethyl]-piperazine and the salts thereof;

g) A process according to Claim 1 in which the reagents and reaction conditions are so chosen as to prepare 1-[3-(N-(Phenoxycarbonyl)amino)-3-phenylpropionyl]-4-[(2-methyl-3-pyridyl)cyanomethyl]-piperazine and the salts thereof;

h) A process according to Claim 1 in which the reagents and reaction conditions are so chosen as to prepare 1-[3-(2-oxo-3-oxazolidinyl)-3-phenylpropionyl]-4-[(2-methyl-3-pyridyl)cyanomethyl]piperazine and the salts thereof;

i) A process according to Claim 1 in which the reagents and reaction conditions are so chosen as to prepare 1-[3-(N-phenylamino)-3-phenylpropionyl]-4-[(2-methyl-3-pyridyl)cyanomethyl]piperazine and the salts thereof;

j) A process according to Claim 1 in which the reagents and reaction conditions are so chosen as to prepare 1-[3-(N-acetyl-N-hydroxyamino)-3-phenylpropionyl]-4-[(2-methyl-3-pyridyl)cyanomethyl]-piperazine and the salts thereof;

k) A process according to Claim 1 in which the reagents and reaction conditions are so chosen as to prepare 1-[3-(N-carbamoyl-N-hydroxyamino)-3-phenylpropionyl]-4-[(2-methyl-3-pyridyl)cyanomethyl]-piperazine and the salts thereof.

**9.** The use of at least one compound of formula **I** or a pharmaceutically acceptable salt thereof for the manufacture of a medicament for the treatment or prophylaxys of PAF-mediated illnesses.

**Claims for the following Contracting State : ES**

**1.** A process for preparing a compound of formula **I**:

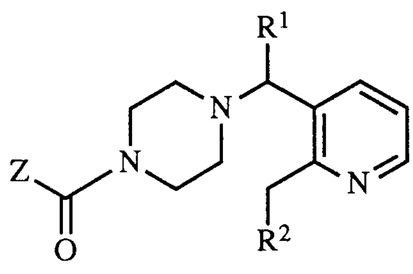

**I**

wherein:

**$R^1$** is -CN, $-CO_2R^3$, $-CON(R^3)_2$, $-COR^3$, $-C\equiv CR^3$ or $-(R^3)C=N-OR^3$, wherein $R^3$ is hydrogen or a $(C_1-C_6)$ alkyl group;

**$R^2$** is hydrogen or a $(C_1-C_3)$ alkyl group;

**Z** represents a group of formula:

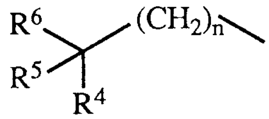

wherein

n is 0, 1 or 2;

$R^4$ represents hydrogen, $(C_1-C_6)$ alkyl, $Ar^1$ or Het; $Ar^1$ is a phenyl group optionally substituted by one or more groups chosen from halogen, trifluoromethyl, $(C_1-C_6)$ alkyl, $(C_1-C_6)$ alkoxy or hydroxy and Het is any radical from a 5- or 6-membered aromatic heterocycle with one ring heteroatom chosen from N, O or S, or with two ring heteroatoms chosen from the pairs N/N, N/O or N/S and which may be optionally substituted by a group chosen from halogen, $(C_1-C_6)$ alkyl, $(C_1-C_6)$ alkoxy or hydroxy;

$R^5$ represents hydrogen, ($C_1$-$C_6$) alkyl, $Ar^1$, $Ar^2$, trifluoromethyl or hydroxy-($C_1$-$C_6$) alkyl such that a) when $R^5$ represents hydrogen, ($C_1$-$C_6$) alkyl or $Ar^1$, $R^6$ represents hydrogen, ($C_1$-$C_6$) alkyl, $OR^7$, $Ar^3S(=O)_m^-$, halogen, $R^3OC(=O)$- or a group $NR^8R^9$, where:

$R^7$ is hydrogen, ($C_1$-$C_6$) alkyl, $Ar^3$ or a group $(Ar^1)_2C=N$-;

m is 0, 1 or 2;

$R^8$ represents hydrogen, ($C_1$-$C_6$) alkyl, $Ar^1$, $Ar^2$, $Ar^3$ or hydroxy; $R^9$ represents hydrogen, ($C_1$-$C_6$) alkyl, ($C_1$-$C_6$) alkenyl, ($C_1$-$C_6$) alkynyl, $Ar^1$, $Ar^2$, $(R^3)_2NC(=O)$-, $HO(R^3)NC(=O)$-, $R^{10}OC(=O)$-, $Ar^1OC(=O)$-, $Ar^2OC(=O)$-, $Ar^1C(=O)$-, Het-($C_1$-$C_6$) alkyl, $HetC(=O)$-, $R^3C(=O)$-, fluorenyl, $R^{10}SO_2$-, $Ar^1SO_2$- or $Ar^2SO_2$-, or the group $NR^8R^9$ forms a pyrrol, imidazol, or a saturated 5- or 6-membered heterocycle which may contain a second ring heteroatom selected from O, S, N and may be optionally substituted by one or two oxo groups and/or by a group $R^{10}$;

$Ar^2$ is a ($C_1$-$C_6$) alkyl group substituted by one $Ar^1$ group;

$Ar^3$ is a ($C_1$-$C_6$) alkyl group substituted by two $Ar^1$ that can be equal or different;

$R^{10}$ is a ($C_1$-$C_6$) alkyl group;

b) when $R^5$ represents trifluoromethyl, $R^6$ represents hydrogen or $OR^3$, where $R^3$ has the above mentioned meaning;

c) when $R^5$ represents hydroxy-($C_1$-$C_6$) alkyl or $Ar^2$, $R^6$ represents a group $NR^8R^9$, where $R^8$ and $R^9$ have the above mentioned meaning; or **Z** represents a group of formula

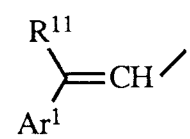

wherein $R^{11}$ represents hydrogen, $Ar^1$ or trifluoromethyl;
and the salts thereof,
**which comprises** treating a compound of formula **II**

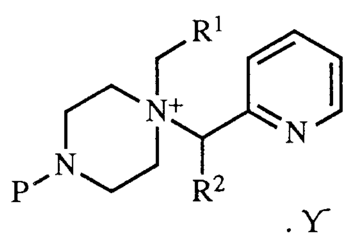

**II**

(wherein $R^1$ and $R^2$ have the previously defined meaning, P is an amine protecting group or ZCO, being Z as described above, and Y is an anion, such as chloride, bromide, iodide, alkylsulfonate or arylsulfonate) with a base, such as 1,8-diazabicyclo[5.4.0]undec-7-ene in acetonitrile, to give a compound of formula **III**

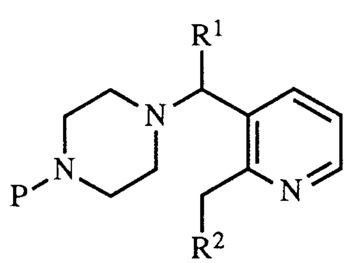

**III**

wherein $R^1$ and $R^2$ have the previously defined meaning, and P is ZCO (in which case **III** is **I**) or alternatively P is an amine protecting group, in which case it is deprotected to give the corresponding amine of formula **IV**

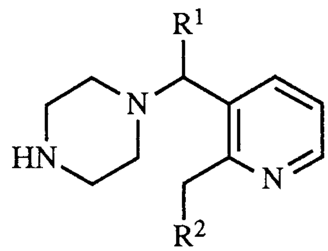

IV

(wherein $R^1$ and $R^2$ have the previously defined meaning), and then reacting **IV** with an acid **ZCOOH** or an equivalent acylating reagent, to give a compound of formula **I**

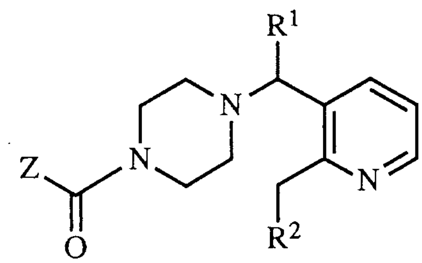

I

(wherein Z, $R^1$ and $R^2$ have the previously defined meaning); and optionally, when in a compound of formula **I**, Z represents a group of formula

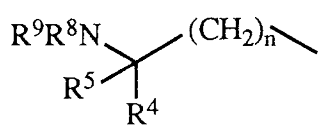

wherein $R^4$, $R^5$, $R^8$, $R^9$ and n have the above mentioned meaning, transforming the groups $R^8$ and/or $R^9$ in a compound of formula **I** into other groups $R^8$ and/or $R^9$.

**2.** A process according to Claim 1 in which the reagents and reaction conditions are so chosen as to prepare a compound of formula **I** wherein:
**$R^1$** is -CN;
**$R^2$** is hydrogen; and
Z has the previously defined meaning.

**3.** A process according to Claim 1 in which the reagents and reaction conditions are so chosen as to prepare a compound of formula **I** wherein:
**$R^1$** is CN;
**$R^2$** is hydrogen; and
**Z** represents a group of formula

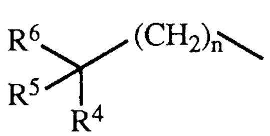

wherein

n is 1;
$R^4$ represents $Ar^1$ or Het, wherein $Ar^1$ and Het have the previously defined meaning;
$R^5$ represents ($C_1$-$C_6$) alkyl or $Ar^1$; and
$R^6$ represents hydrogen, ($C_1$-$C_6$) alkyl, $OR^3$ or $NH_2$, where $R^3$ has the previously defined meaning.

**4.** A process according to Claim 1 in which the reagents and reaction conditions are so chosen as to prepare a compound of formula **I** wherein:
**$R^1$** is CN;
**$R^2$** is hydrogen; and
**Z** represents a group of formula

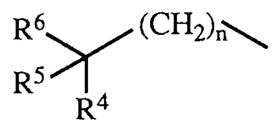

wherein
n is 1;
$R^4$ represents $Ar^1$ or Het, wherein $Ar^1$ and Het have the previously defined meaning;
$R^5$ represents trifluoromethyl; and
$R^6$ represents hydrogen or $OR^3$, where $R^3$ has the previously defined meaning.

**5.** A process according to Claim 1 in which the reagents and reaction conditions are so chosen as to prepare a compound of formula **I** wherein:
**$R^1$** is CN;
**$R^2$** is hydrogen; and
**Z** represents a group of formula

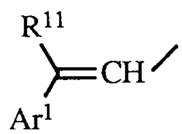

wherein $R^{11}$ represents $Ar^1$ or trifluoromethyl, where $Ar^1$ has the previously defined meaning.

**6.** A process according to Claim 1 in which the reagents and reaction conditions are so chosen as to prepare a compound of formula **I** wherein:
**$R^1$** is CN;
**$R^2$** is hydrogen;
**Z** represents a group of formula

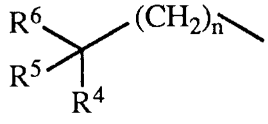

wherein
n is 0 or 1;
$R^4$ and $R^5$ represent each one independently hydrogen or ($C_1$-$C_6$) alkyl; and
$R^6$ represents a group $NR^8R^9$, where:
$R^8$ represents $Ar^3$, being $Ar^3$ as above defined;
$R^9$ represents hydrogen, ($C_1$-$C_6$) alkyl, $(R^3)_2NC(=O)$-,
$R^{10}OC(=O)$-, $R^3C(=O)$- or $R^{10}SO_2$-, being $R^3$ and $R^{10}$ as above defined.

**7.** A process according to Claim 1 in which the reagents and reaction conditions are so chosen as to prepare a compound of formula **I** wherein:

**$R^1$** is CN;

**$R^2$** is hydrogen;

**Z** represents a group of formula

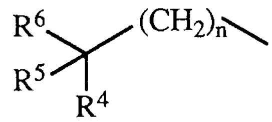

wherein

n is 1;

$R^4$ represents $Ar^1$ or Het, wherein $Ar^1$ and Het have the previously defined meaning;

$R^5$ represents hydrogen or ($C_1$-$C_6$) alkyl; and

$R^6$ represents a group $NR^8R^9$, where $R^8$ represents hydrogen, ($C_1$-$C_6$) alkyl, $Ar^1$ or hydroxy; $R^9$ represents hydrogen, ($C_1$-$C_6$) alkyl, ($C_1$-$C_6$) alkenyl, ($C_1$-$C_6$)alkynyl, $Ar^2$, $(R^3)_2NC(=O)$-, $HO(R^3)NC(=O)$-, $R^{10}OC(=O)$-, $Ar^1OC(=O)$-, $Ar^2OC(=O)$-, $Ar^1C(=O)$-, Het-($C_1$-$C_6$) alkyl, HetC(=O)-, $R^3C(=O)$-, $R^{10}SO_2$-, $Ar^1SO_2$- or $Ar^2SO_2$-, or the group $NR^8R^9$ forms an heterocycle of the formula (i), (ii), (iii), (iv), (v), (vi) or (vii):

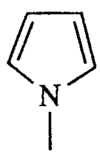

(i)

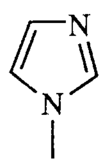

(ii)

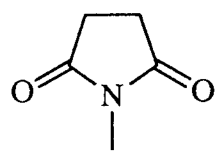

(iii)

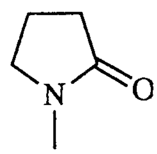

(iv)

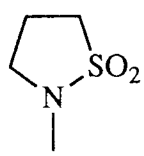

(v)

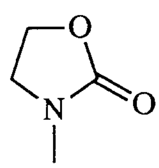

(vi)

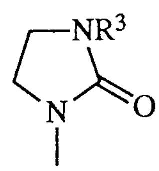

(vii)

wherein $R^3$, $R^{10}$, $Ar^1$, $Ar^2$ and Het have the previously defined meaning.

**8.**

a) A process according to Claim 1 in which the reagents and reaction conditions are so chosen as to prepare 1-(3,3-Diphenyl-3-hydroxypropionyl)-4-[(2-methyl-3-pyridyl)cyanomethyl]piperazine and the salts thereof;

b) A process according to Claim 1 in which the reagents and reaction conditions are so chosen as to prepare 1-(3-Phenyl-3-hydroxy-3-(trifluoromethyl)propionyl)-4-[(2-methyl-3-pyridyl)cyanomethyl]-piperazine and the salts thereof;

c) A process according to Claim 1 in which the reagents and reaction conditions are so chosen as to prepare 1-[3-(N-Benzylamino)-3-phenylpropionyl]-4-[(2-methyl-3-pyridyl)cyanomethyl]piperazine and the salts thereof;

d) A process according to Claim 1 in which the reagents and reaction conditions are so chosen as to prepare 1-[3-(N-acetylamino)-3-phenylpropionyl]-4-[(2-methyl-3-pyridyl)cyanomethyl]piperazine and the salts thereof;

e) A process according to Claim 1 in which the reagents and reaction conditions are so chosen as to prepare 1-[N-(Diphenylmethyl)aminoacetyl]-4-[(2-methyl-3-pyridyl)cyanomethyl]piperazine and the salts thereof;
f) A process according to Claim 1 in which the reagents and reaction conditions are so chosen as to prepare 1-[3-(N-(Methoxycarbonyl)amino)-3-phenylpropionyl]-4-[(2-methyl-3-pyridyl)cyanomethyl]-piperazine and the salts thereof;
g) A process according to Claim 1 in which the reagents and reaction conditions are so chosen as to prepare 1-[3-(N-(Phenoxycarbonyl)amino)-3-phenylpropionyl]-4-[(2-methyl-3-pyridyl)cyanomethyl]-piperazine and the salts thereof;
h) A process according to Claim 1 in which the reagents and reaction conditions are so chosen as to prepare 1-[3-(2-oxo-3-oxazolidinyl)-3-phenylpropionyl]-4-[(2-methyl-3-pyridyl)cyanomethyl]piperazine and the salts thereof;
i) A process according to Claim 1 in which the reagents and reaction conditions are so chosen as to prepare 1-[3-(N-phenylamino)-3-phenylpropionyl]-4-[(2-methyl-3-pyridyl)cyanomethyl]piperazine and the salts thereof;
j) A process according to Claim 1 in which the reagents and reaction conditions are so chosen as to prepare 1-[3-(N-acetyl-N-hydroxyamino)-3-phenylpropionyl]-4-[(2-methyl-3-pyridyl)cyanomethyl]-piperazine and the salts thereof;
k) A process according to Claim 1 in which the reagents and reaction conditions are so chosen as to prepare 1-[3-(N-carbamoyl-N-hydroxyamino)-3-phenylpropionyl]-4-[(2-methyl-3-pyridyl)cyanomethyl]-piperazine and the salts thereof.

European Patent Office

# EUROPEAN SEARCH REPORT

Application Number

EP 92 11 1986

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X,D, P | EP-A-0 441 226 (J. URIACH & CIA. S.A.)<br>* claims 1,25,26-28; examples * | 1-11 | C07D213/57<br>C07D213/55<br>C07D213/53<br>C07D213/38<br>C07D401/12<br>C07D405/12<br>C07D413/12<br>A61K31/44<br>//(C07D401/12, 213:00, 209/00)<br>(C07D405/12, 307:00, 213:00)<br>(C07D413/12, 263:00, 213:00)<br>(C07D401/12, 241:00, 213:00) |
| A,D | EP-A-0 284 359 (TAKEDA CHEMICAL INDUSTRIES, LTD.)<br>* the whole document * | 1-11 | |

TECHNICAL FIELDS SEARCHED (Int. Cl.5)

C07D
A61K

The present search report has been drawn up for all claims

| Place of search | Date of completion of the search | Examiner |
|---|---|---|
| THE HAGUE | 28 OCTOBER 1992 | P. BOSMA |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)